# EUROPÄISCHE PATENTSCHRIFT

(11) **EP 2 257 526 B1**
(45) Veröffentlichungstag und Bekanntmachung des Hinweises auf die Patenterteilung: **14.12.2011**
(21) Anmeldenummer: 09725570.7
(22) Anmeldetag: 25.03.2009
(51) Int. Cl.: C07D 209/14, C07D 471/04, A61K 31/437, A61P 25/04

(54) **SPIRO(5.5)UNDECAN DERIVATE**
SPIRO(5.5)UNDECANE DERIVATIVES
DÉRIVÉS DE SPIRO(5.5)UNDÉCANE

(30) Priorität: 27.03.2008 EP 08005804
(43) Veröffentlichungstag der Anmeldung: 08.12.2010
(73) Patentinhaber: Grünenthal GmbH, 52078 Aachen (DE)
(72) Erfinder: ZEMOLKA, Saskia, 52066 Aachen (DE); SCHUNK, Stefan, 52070 Aachen (DE); LINZ, Klaus, 53343 Wachtberg (DE); SCHRÖBER, Wolfgang, 52074 Aachen (DE); ENGLBERGER, Werner, 52223 Stolberg (DE); THEIL, Fritz, 12247 Berlin (DE); ROLOFF, Birgit, 12587 Berlin (DE)
(74) Vertreter: Bülle, Jan
(86) Internationale Anmeldenummer: PCT/EP2009/002180
(87) Internationale Veröffentlichungsnummer: WO 2009/118169

(56) Entgegenhaltungen:
- WO-A-2005/066183
- WO-A-2008/009416

## Beschreibung

Die vorliegende Erfindung betrifft substituierte spirocyclische Cyclohexan-Derivate, welche eine Affinität an den µ-Opioid-Rezeptor und den ORL1-Rezeptor aufweisen, Verfahren zu deren Herstellung, Arzneimittel enthaltend diese Verbindungen und die Verwendung dieser Verbindungen zur Herstellung von Arzneimitteln.

Spirocyclische Cyclohexan-Derivate, welche eine Affinität an den µ-Opioid-Rezeptor und den ORL1-Rezeptor aufweisen, sind im Stand der Technik bekannt. In diesem Zusammenhang kann beispielsweise vollumfänglich verwiesen werden auf WO2004/043967, WO2005/ 063769, WO2005/066183, WO2006/018184, WO2006/108565 WO2007/12490 und WO2008/009416.

Die bekannten Verbindungen sind jedoch nicht in jeglicher Hinsicht zufrieden stellend und es besteht ein Bedarf an weiteren Verbindungen mit vergleichbaren oder besseren Eigenschaften.

So zeigen die bekannten Verbindungen in geeigneten Bindungsassays mitunter eine gewisse Affinität zum hERG-lonenkanal, zum L-Typ Calcium-lonenkanal (Phenylalkylamin-, Benzothiazepin-, Dihydropyridin-Bindungsstellen) bzw. zum Natrium-Kanal im BTX-Assay (Batrachotoxin), was jeweils als Anzeichen für kardiovaskuläre Nebenwirkungen gedeutet werden kann. Ferner zeigen zahlreiche der bekannten Verbindungen eine nur geringe Löslichkeit in wässrigen Medien, was sich u.a. negativ auf die Bioverfügbarkeit auswirken kann. Darüber hinaus ist die chemische Stabilität der bekannten Verbindungen oft nur unzureichend. So zeigen die Verbindungen mitunter keine ausreichende pH-, UV- bzw. Oxidationsstabilität, was sich u.a. negativ auf die Lagerstabilität und auch auf die orale Bioverfügbarkeit auswirken kann. Ferner weisen die bekannten Verbindungen teilweise ein ungünstiges PK/PD (Pharmakokinetik/Pharmakodynamik)-Profil auf, was sich z.B. in einer zu langen Wirkdauer zeigen kann.

Auch die metabolische Stabilität der bekannten Verbindungen scheint verbesserungsbedürftig. Eine verbesserte metabolische Stabilität kann auf eine erhöhte Bioverfügbarkeit hinweisen. Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner zweigen die bekannten Verbindungen eine mitunter nur geringe Selektivität gegenüber dem kappa-Opioid-Rezeptor, welcher für Nebenwirkungen, wie beispielsweise Dysphorie, Sedation und Diurese verantwortlich ist.

Der Erfindung liegt die Aufgabe zugrunde, Verbindungen zur Verfügung zu stellen, welche sich zu pharmazeutischen Zwecken eignen und Vorteile gegenüber den Verbindungen des Standes der Technik aufweisen.

Diese Aufgabe wird durch den Gegenstand der Patentansprüche gelöst.

Es wurde überraschend gefunden, dass substituierte Verbindungen hergestellt werden können, welche eine Affinität an den µ-Opioid-Rezeptor und den ORL1-Rezeptor aufweisen.

Die Erfindung betrifft Verbindungen der allgemeinen Formel (1), 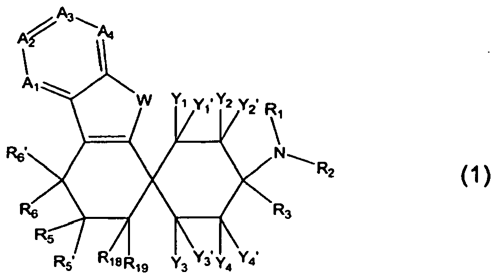 worin
A₁ für -N= oder -CR₇= steht,
A₂ für -N= oder -CR₈= steht,
A₃ für -N= oder -CR₉= steht,
A₄ für -N= oder -CR₁₀= steht;
mit der Maßgabe, dass höchstens zwei der Reste A₁, A₂, A₃ und A₄, vorzugsweise 0, 1 oder 2 der Reste A₁, A₂, A₃ und A₄, für -N= stehen;
Y₁, Y₁', Y₂, Y₂', Y₃, Y₃', Y₄ und Y₄ jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂; vorzugsweise jeweils unabhängig voneinander ausgewählt sind aus der Gruppe bestehend aus -H, -F, -Cl, -CN und -C₁₋₈-Aliphat; oder Y₁ und Y₁', oder Y₂ und Y₂', oder Y₃ und Y₃', oder Y₄ und Y₄ gemeinsam für =O stehen;
W für -NR₄-, -O- oder -S- steht, vorzugsweise für -NR₄- oder -O-;
R₀ jeweils unabhängig für -C₁₋₈-Aliphat, -C₃₋₁₂-Cycloaliphat, -Aryl, -Heteroaryl, -C₁₋₈-Aliphat -C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloatiphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₁₁CH₂CH₂- oder -(CH₂)₃₋₆- stehen;
R₃ für -R₀ steht;
R₄ für -H, -R₀, -COR₁₂ oder -S(=O)₂R₁₂ steht;
R₅, R₅ , R₆, R₆', R₇, R₈, R₉, R₁₀, R₁₈ und R₁₉ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, =O oder -R₀ stehen; oder R₅ und R₆ gemeinsam für -(CH₂)₂₋₆- stehen, wobei einzelne Wasserstoffatome auch durch -F, -Cl, -Br, -I, -NO₂, -CF₃, -OR₁₃, -CN oder -C₁₋₆-Aliphat ersetzt sein können;
R₁₁ jeweils unabhängig für -H, -R₀ oder -C(=O)R₀ steht;
R₁₂ jeweils unabhängig für -H, -R₀, -OR₁₃, oder -NR₁₄R₁₅ steht;
R₁₃ jeweils unabhängig für -H oder -R₀ steht;
R₁₄ und R₁₅ unabhängig voneinander für -H oder -R₀ stehen; oder R₁₄ und R₁₅ zusammen für -CH₂CH₂OCH₂CH₂-, -CH₂CH₂NR₁₆CH₂CH₂- oder -(CH₂)₃₋₆- stehen;
R₁₆ für -H oder -C₁₋₆-Aliphat steht;
wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein ein- oder mehrfach ungesättigter, unsubstituierter oder ein- oder mehrfach substituierter, alicyclischer, mono- oder multicyclischer Kohlenwasserstoffrest ist, dessen Zahl der Ringkohlenstoffatome vorzugsweise im angegebenen Bereich liegt (d.h. "C₃₋₈-"Cycloaliphat weist vorzugsweise 3, 4, 5, 6, 7 oder 8 Ringkohlenstoffatome auf);
wobei in Bezug auf "Aliphat" und "Cycloaliphat" unter "ein- oder mehrfach substituiert" die ein- oder mehrfache Substitution eines oder mehrerer Wasserstoffatome, z.B. die einfache, zweifache, dreifache oder vollständige Substitution, durch Substituenten unabhängig voneinander ausgewählt aus der Gruppe bestehend aus -F, -Cl, -Br, -I, -CN, -NO₂, -CHO, =O -R₀, -C(=O)R₀, -C(=O)-OH, -C(=O)OR₀, -C(=O)NH₂, -C(=O)-NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂. -N⁺(R₀)₃, -N⁺(R₀)₂O⁻, -NHC(=O)R₀, -NHC(=O)OR₀, -NHC(=O)NH₂, -NHC(=O)NHR₀, -NH-C(=O)N(R₀)₂, -Si(R₀)₃, -PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein-oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)OH, -C(=O)ORₒ, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC(=O)N(R₀)₂, -Si(R₀)₃, -PO(OR₀)₂ verstanden wird; wobei ggf. vorhandene N-Ringatome jeweils oxidiert sein können (N-Oxid);
in Form eines einzelnen Stereoisomers oder deren Mischung, der freien Verbindungen und/oder ihrer physiologisch verträglichen Salze.

Bei der Zusammenfassung verschiedener Reste, beispielsweise R₀, R₇, R₈, R₉ und R₁₀ sowie der Zusammenfassung von Resten an deren Substituenten, wie z. B. -OR₁₃, -SR₁₃, -S(=O)₂R₁₃, oder -COOR₁₃, kann ein Substituent, z.B. R₁₃, für zwei oder mehrere Reste, beispielsweise R₀, R₇, R₈, R₉ und R₁₀, innerhalb einer Substanz unterschiedliche Bedeutungen annehmen.

Die erfindungsgemäßen Verbindungen zeigen gute Bindung an den ORL1-Rezeptor und den µ-Opioid-Rezeptor.

In einer bevorzugten Ausführungsform weisen die erfindungsgemäßen Verbindungen ein Verhältnis ORL1/µ-Affinität von wenigstens 0,1 auf. Das ORL1/µ-Verhältnis ist definiert als 1/[K_{i(ORL1)}/K_{i(µ)}]. Besonders bevorzugt beträgt das ORL1/µ-Vehältnis wenigstens 0,2 oder wenigstens 0,5, bevorzugter wenigstens 1,0 oder wenigstens 2,0, noch bevorzugter wenigstens 3,0 oder wenigstens 4,0, am bevorzugtesten wenigstens 5,0 oder wenigstens 7,5 und insbesondere wenigstens 10 oder wenigstens 15. In einer bevorzugten Ausführungsform liegt das ORL1 /µ-Verhältnis im Bereich von 0,1 bis 30, bevorzugter 0,1 bis 25.

In einer anderen bevorzugten Ausführungsform weisen die erfindungsgemäßen Verbindungen ein Verhältnis ORL1/µ-Affinität von mehr als 30, bevorzugter wenigstens 50, noch bevorzugter wenigstens 100, am bevorzugtesten wenigstens 200 und insbesondere wenigstens 300 auf.

Die erfindungsgemäßen Verbindungen weisen bevorzugt einen Kᵢ-Wert am µ-Opioid-Rezeptor von höchstens 500 nM, bevorzugter höchstens 100 nM, noch bevorzugter höchstens 50 nM, am bevorzugtesten höchstens 10 nM und insbesondere höchstens 1,0 nM auf.

Methoden zur Bestimmung des Kᵢ-Werts am µ-Opioid-Rezeptor sind dem Fachmann bekannt. Bevorzugt erfolgt die Bestimmung wie im Zusammenhang mit den Beispielen beschrieben.

Die erfindungsgemäßen Verbindungen weisen bevorzugt einen Kᵢ-Wert am ORL1-Rezeptor von höchstens 500 nM, bevorzugter höchstens 100 nM, noch bevorzugter 50 nM, am bevorzugtesten höchstens 10 nM und insbesondere höchstens 1,0 nM auf.

Methoden zur Bestimmung des Kᵢ-Werts am ORL1-Rezeptor sind dem Fachmann bekannt. Bevorzugt erfolgt die Bestimmung wie im Zusammenhang mit den Beispielen beschrieben.

Überraschenderweise hat sich gezeigt, dass Verbindungen mit Affinität zum ORL1- und µ-Opioid-Rezeptor, bei denen das durch 1/[K_{i(ORL1)}/K_{i(µ)}] definierte Verhältnis von ORL1 zu µ im Bereich von 0,1 bis 30 liegt, vorzugsweise von 0,1 bis 25, ein pharmakologisches Profil aufweisen, das verglichen mit dem anderer Opioidrezeptorliganden deutliche Vorteile aufweist:
1. Die erfindungsgemäßen Verbindungen zeigen eine Wirksamkeit in Akutschmerzmodellen, die mitunter vergleichbar ist mit der gebräuchlicher Stufe-3 Opioide. Gleichzeitig zeichnen sie sich aber durch eine gegenüber klassischen µ-Opioiden deutlich bessere Verträglichkeit aus.
2. Im Gegensatz zu gebräuchlichen Stufe-3 Opioiden zeigen die erfindungsgemäßen Verbindungen eine deutlich höhere Wirksamkeit in Mono- und Polyneuropathieschmerzmodellen, was auf einen Synergismus von ORL1- und µ-Opioid Komponente zurückzuführen ist.
3. Im Gegensatz zu gebräuchlichen Stufe-3 Opioiden zeigen die erfindungsgemäßen Verbindungen in neuropathischen Tieren eine weitgehende, bevorzugt eine vollständige, Trennung von antiallodynischer bzw. antihyperalgetischer Wirkung und antinociceptivem Effekt.
4. Im Gegensatz zu gebräuchlichen Stufe-3 Opioiden zeigen die erfindungsgemäßen Verbindungen in Tiermodellen für chronischen Entzündungsschmerz (u.a. Carrageenan-oder CFA-induzierte Hyperalgesie, viszeraler Entzündungsschmerz) eine deutliche Wirkverstärkung gegenüber dem Akutschmerz.
5. Im Gegensatz zu gebräuchlichen Stufe-3 Opioiden sind µ-opioidtypische Nebenwirkungen (u.a. Atemdepression, opioidinduzierte Hyperalgesie, körperliche Abhängigkeit/Entzug, psychische Abhängigkeit/Sucht) bei den erfindungsgemäßen Verbindungen im therapeutisch wirksamen Dosisbereich deutlich reduziert, bzw. vorzugsweise nicht zu beobachten.

Aufgrund der reduzierten µ-opioiden Nebenwirkungen einerseits und der erhöhten Wirksamkeit bei chronischem, bevorzugt neuropathischem Schmerz andererseits zeichnen sich die gemischten ORL1/µ-Agonisten somit durch deutlich vergrößerte Sicherheitsabstände gegenüber reinen µ-Opioiden aus. Daraus resultiert ein deutlich vergrößertes "therapeutisches Fenster" bei der Behandlung von Schmerzzuständen, bevorzugt chronischen Schmerzen, noch bevorzugter neuropathischen Schmerzen.

In einer bevorzugten Ausführungsform der erfindungsgemäßen Verbindungen sind A₁, A₂, A₃ und A₄ ungleich -N=. In einer anderen bevorzugten Ausführungsform der erfindungsgemäßen Verbindungen sind drei der Reste A₁, A₂, A₃ und A₄ ungleich -N= und der übrige der Reste ist gleich -N=. Vorzugsweise sind A₁, A₂ und A₃ ungleich -N=; oder A₁, A₂ und A₄ sind ungleich -N=; oder A₁, A₃ und A₄ sind ungleich -N=; oder A₂, A₃ und A₄ sind ungleich -N=. In einer anderen bevorzugten Ausführungsform der erfindungsgemäßen Verbindungen sind zwei der Reste A₁, A₂, A₃ und A₄ ungleich -N= und die anderen beiden Reste sind -N=. Vorzugsweise sind A₁ und A₂ -N= und A₃ und A₄ sind ungleich -N=; oder A₂ und A₃ sind -N= und A₁ und A₄ sind ungleich -N=; oder A₃ und A₄ sind -N= und A₁ und A₂ sind ungleich -N=; oder A₁ und A₃ sind -N= und A₂ und A₄ sind ungleich -N=; oder A₁ und A₄ sind -N= und A₂ und A₃ sind ungleich -N=; oder A₂ und A₄ sind -N= und A₁ und A₃ sind ungleich -N=.

Bevorzugt sind Y₁, Y₁', Y₂, Y₂ , Y₃, Y₃', Y₄ und Y₄' jeweils unabhängig voneinander ausgewählt aus der Gruppe bestehend aus -H, -F, -Cl, -Br, -I, -CN, -NH₂, -NH-C₁₋₆-Aliphat, -NH-C_{3 8}-Cycloaliphat, -NH-C₁₋₆-Aliphat-OH, -N(C₁₋₆-Aliphat)₂, -N(C₃₋₈-Cycloaliphat)₂, -N(C₁₋₆-Aliphat-OH)_{2,} -NO₂, -NH-C₁₋₆-Aliphat-C₃₋₈-Cycloaliphat, -NH-C₁₋₆-Aliphat-Aryl, -NH-C₁₋₆-Aliphat-Heteroaryl, -NH-Aryl, -NH-Heteroaryl, -SH, -S-C₁₋₆-Aliphat, -S-C₃₋₈-Cycloaliphat, -S-C₁₋₆-Aliphat-C₃₋₈-Cycloaliphat, -S-C₁₋₆-Aliphat-Aryl, -S-C₁₋₆-Aliphat-Heteroaryl, -S-Aryl, -S-Heteroaryl, -OH, -O-C₁₋₆-Aliphat, -O-C₃₋₈-Cycloaliphat, -O-C₁₋₆-Aliphat-OH, -O-C₁₋₆-Aliphat-C₃₋₈-Cycloaliphat, -O-C₁₋₆-Aliphat-Aryl, -O-C₁₋₆-Aliphat-Heretoaryl, -O-Aryl, -O-Heteroaryl, -O-C(=O)C₁₋₆-Aliphat, -O-C(=O)C₃₋₈-Cycloaliphat, -O-C(=O)C₁₋₆-Aliphat-OH, -O-C(=O)C₁₋₆-Aliphat-C₃₋₈-Cycloaliphat, -O-C(=O)C₁₋₆-Aliphat-Aryl, -O-C(=O)C₁₋₆-Aliphat-Heretoaryl, -O-C(=O)Aryl, -O-C(=O)Heteroaryl, -C₁₋₆-Aliphat, -C₃₋₈-Cycloaliphat, -C₁₋₆-Aliphat-C₃₋₈-Cycloaliphat, -C₁₋₆-Aliphat-Aryl, -C₁₋₆-Aliphat-Heteroaryl, -Aryl, -Heteroaryl, -C(=O)C₁₋₆-Aliphat, -C(=O)C₃₋₈-Cycloaliphat, -C(=O)C₁₋₆-Aliphat-C₃₋₈-Cycloaliphat, -C(=O)C₁₋₆-Aliphat-Aryl, -C(=O)C₁₋₆-Aliphat-Heteroaryl, -C(=O)Aryl, -C(=O)Heteroaryl, -CO₂H, -CO₂-C₁₋₆-Aliphat, -CO₂-C₃₋₈-Cycloaliphat, -CO₂-C₁₋₆-Aliphat-C₃₋₈-Cycloaliphat, -CO₂-C₁₋₆-Aliphat-Aryl, -CO₂-C₁₋₆-Aliphat-Heteroaryl, -CO₂-Aryl, -CO₂-Heteroaryl; oder Y₁ und Y₁', oder Y₂ und Y₂', oder Y₃ und Y₃', oder Y₄ und Y₄' stehen gemeinsam für =O. Bevorzugt sind Y₁, Y₁', Y₂, Y₂', Y₃, Y₃', Y₄ und Y₄' jeweils unabhängig voneinander ausgewählt aus der Gruppe bestehend aus -H, -F, -Cl, -Br, -I, -CN, -NH₂ und -OH.

In einer bevorzugten Ausführungsform ist einer der Reste Y₁, Y₁', Y₂, Y₂', Y₃, Y₃', Y₄ und Y₄' ungleich -H und die übrigen Reste stehen für -H.

Besonders bevorzugt stehen Y₁, Y₁', Y₂, Y₂', Y₃, Y₃', Y₄ und Y₄' jeweils für -H.

Bevorzugte Ausführungsformen der erfindungsgemäßen Verbindungen der allgemeinen Formel (1) haben die allgemeine Formel (2), (3), (4) oder (5):

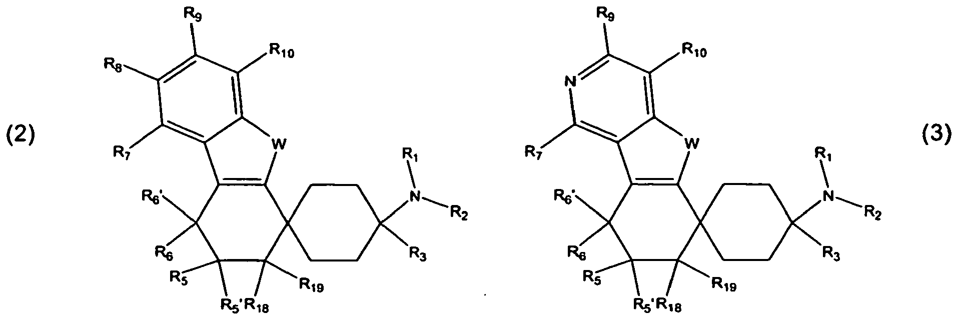

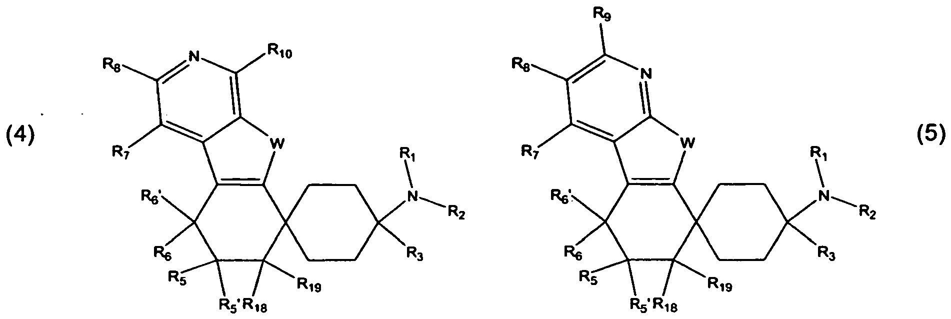

In einer bevorzugten Ausführungsform der erfindungsgemäßen Verbindungen ist W -NR₄-.

R₀ steht jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl. Dabei bedeuten -C₁₋₈-Aliphat-C₃₋₁₂-Cycloaliphat, -C₁₋₈-Aliphat-Aryl oder -C₁₋₈-Aliphat-Heteroaryl, dass die Reste -C₃₋₁₂₋Cycloaliphat, -Aryl oder -Heteroaryl jeweils über eine zweibindige Brücke -C₁₋₈-Aliphat- gebunden sind. Bevorzugte Beispiele für -C₁₋₈-Aliphat-Aryl sind -CH₂-C₆H₅, -CH₂CH₂-C₆H₅, und -CH=CH-C₆H₅. Ferner bedeuten -C₃₋₈-Cycloaliphat-C₁₋₈-Aliphat, -C₁₋₈-Cycloaliphat-Aryl oder -C₃₋₈-Cycloaliphat-Heteroaryl, dass die Reste -C₁₋₈-Aliphat, -Aryl oder -Heteroaryl jeweils über eine zweibindige Brücke -C₃₋₈-Cycloaliphat- gebunden sind. Ein bevorzugtes Beispiel für -C₃₋₈-Cycloaliphat-Aryl ist -Cyclopropyl-Phenyl.

Bevorzugt stehen R₁ und R₂, unabhängig voneinander für -H; -C₁₋₆-Aliphat; -C₃₋₈-Cycloaliphat, -C₁₋₆-Aliphat-Aryl, -C₁₋₆-Aliphat-C₃₋₈-Cycloaliphat oder -C₁₋₆-Aliphat-Heteroaryl; oder die Reste R₁ und R₂ bilden zusammen einen Ring und bedeuten -CH₂CH₂OCH₂CH₂-, -CH₂CH₂NR₁₁CH₂CH₂- oder -(CH₂)₃₋₆-.

Bevorzugter stehen R₁ und R₂ unabhängig voneinander für -H; -C₁₋₅-Aliphat; oder die Reste R₁ und R₂ bilden zusammen einen Ring und bedeuten -CH₂CH₂OCH₂CH₂-, -CH₂CH₂NR₁₁-CH₂CH₂- oder -(CH₂)₃₋₆-, wobei R₁₁ bevorzugt -H oder -C₁₋₅-Aliphat bedeutet.

Besonders bevorzugt sind Verbindungen, worin R₁ und R₂ unabhängig voneinander für -CH₃ oder -H stehen, wobei R₁ und R₂ nicht gleichzeitig -H bedeuten; oder R₁ und R₂ bilden einen Ring und bedeuten -(CH₂)₃₋₄-.

Ganz besonders bevorzugt sind Verbindungen, worin R₁ und R₂ für -CH₃ stehen.

Bevorzugt steht R₃ für -C₁₋₈-Aliphat, -C₁₋₈-Cycloaliphat, -Aryl, -Heteroaryl; oder für jeweils über eine -C₁₋₃-Aliphat-Gruppe gebundenes -Aryl, -Heteroaryl oder -C₃₋₈-Cycloaliphat.

Besonders bevorzugt steht R₃ für -Ethyl, -Propyl, -Butyl, -Pentyl, -Hexyl, -Heptyl, -Cyclopentyl, -Cyclohexyl, -Phenyl, -Benzyl, -Naphthyl, -Anthracenyl, -Thiophenyl, -Benzothiophenyl, -Furyl, -Benzofuranyl, -Benzodioxolanyl, -Indolyl, -Indanyl, -Benzodioxanyl, -Pyrrolyl, -Pyridyl, -Pyrimidyl oder -Pyrazinyl, jeweils unsubstituiert oder ein- oder mehrfach substituiert; über eine gesättigte, unverzweigte -C₁₋₃-Aliphat-Gruppe gebundenes -C₅₋₆-Cycloaliphat, -Phenyl, -Naphthyl, -Anthracenyl, -Thiophenyl, -Benzothiophenyl, -Pyridyl, -Furyl, -Benzofuranyl, -Benzodioxolanyl, -Indolyl, -Indanyl, -Benzodioxanyl, -Pyrrolyl, -Pyrimidyl, -Triazolyl oder -Pyrazinyl, jeweils unsubstituiert oder ein- oder mehrfach substituiert.

Bevorzugter steht R₃ für -Propyl, -Butyl, -Pentyl, -Hexyl, -Phenyl, -Furyl, -Thiophenyl, -Naphthyl, -Benzyl, -Benzofuranyl, -Indolyl, -Indanyl, -Benzodioxanyl, -Benzodioxolanyl, -Pyridyl, -Pyrimidyl, -Pyrazinyl, -Triazolyl oder -Benzothiophenyl, jeweils unsubstituiert oder ein- oder mehrfach substituiert; über eine gesättigte, unverzweigte -C₁₋₃-Aliphat-Gruppe gebundenes -Phenyl, -Furyl oder -Thiophenyl, jeweils unsubstituiert oder ein- oder mehrfach substituiert.

Noch bevorzugter steht R₃ für -Propyl, -Butyl, -Pentyl, -Hexyl, -Phenyl, -Phenethyl, -Thiophenyl, -Pyridyl, -Triazolyl, -Benzothiophenyl oder -Benzyl, jeweils substituiert oder unsubstituiert, besonders bevorzugt für -Propyl, -3-Methoxypropyl, -Butyl, -Pentyl, -Hexyl, -Phenyl, -3-Methylphenyl, -3-Fluorphenyl, -Benzo[1,3]-dioxolyl, -Thienyl, -Benzothiophenyl, -4-Chlorbenzyl, -Benzyl, -3-Chlorbenzyl, -4-Methylbenzyl, -2-Chlorbenzyl, -4-Fluorbenzyl, -3-Methylbenzyl, -2-Methylbenzyl, -3-Fluorbenzyl, -2-Fluorbenzyl, -1-Methyl-1,2,4-triazolyl oder -Phenethyl.

Ganz besonders bevorzugt steht R₃ für -Butyl, -Ethyl, -3-Methoxypropyl, -Benzothiophenyl, -Phenyl, -3-Methylphenyl, -3-Fluorphenyl, -Benzo[1,3]-dioxotyl, -Benzyl, -1-Methyl-1,2,4-triazolyl, -Thienyl oder -Phenethyl.

Am bevorzugtesten steht R₃ für -Phenyl, -Benzyl oder -Phenethyl, jeweils unsubstituiert oder am Ring ein- oder mehrfach substituiert; -C₁₋₅-Aliphat, -C₄₋₆-Cycloaliphat, -Pyridyl, -Thienyl, -Thiazolyl, -Imidazolyl, -1,2,4 Triazolyl oder -Benzimidazolyl, unsubstituiert oder ein- oder mehrfach substituiert.

Insbesondere bevorzugt steht R₃ für -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂, -NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl, jeweils unsubstituiert oder ein- oder mehrfach substituiert mit -OH, -OCH₃ oder -OC₂H₅, wobei vorzugsweise -Thienyl, -Pyridyl, -Thiazolyl, -Imidazolyl, -1,2,4 Triazolyl und -Benzimidazolyl unsubstituiert sind.

Insbesondere bevorzugt steht R₃ für -Phenyl, unsubstituiert oder einfach substituiert mit -F, -C1, -CN, -CH₃; -Thienyl; -Ethyl, -n-Propyl oder -n-Butyl, unsubstituiert oder ein- oder mehrfach substituiert mit -OCH₃, -OH oder -OC₂H₅, insbesondere mit -OCH₃.

Bevorzugt steht R₄ für -H; -C₁₋₆-Aliphat, -Aryl, -Heteroaryl, -C₁₋₆-Aliphat-Aryl, -C₁₋₆-Aliphat-Heteroaryl oder -C₁₋₆-Aliphat-Cycloaliphat, -COR₁₂ oder -SO₂R₁₂**.** Besonders bevorzugt steht R₄für-H.

Bevorzugt stehen R₅ und R₅ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)NR₁₄R₁₅, -CN, -C(=O)OR₁₃, -C(=O)NR₁₃. -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, oder -R₀; oder R₅ und R₅' stehen gemeinsam für =O. Bevorzugter stehen R₅ und R₅' jeweils unabhängig voneinander für -H, -F, -Cl, -Br, -I, -NO₂, -C₁₋₆-Aliphat, -C₃₋₈-Cycloaliphat, -Aryl, -Heteroaryl, -C₁₋₆-Aliphat-Aryl, -C₁₋₆-Aliphat-Heteroaryl, -C₁₋₆-Aliphat-C₃₋₈-Cycloaliphat, -NR₁₄R₁₅, -NHC(=O)R₀, -NHC(=O)NHR₀, -NHC(=O)OR₀, -COOR₁₃, -CONR₁₃ oder -OR₁₃.

In einer bevorzugten Ausführungsform stehen R₅ und R₅' für -H. In einer anderen bevorzugten Ausführungsform steht R₅ für -H und R₅' ist ungleich -H. In einer anderen bevorzugten Ausführungsform sind sowohl R₅ als auch R₅' ungleich -H.

Außerdem bevorzugt steht R₅ für -H, -C₁₋₅-Aliphat oder -COOR₁₃. Besonders bevorzugt steht R₅ für -CH₃, -CH₂OH -COOH oder -COOCH₃. Ganz besonders bevorzugt steht R₅ für -H.

Bevorzugt stehen R₆ und R₆' jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, oder -R₀; oder R₆ und R₆' stehen gemeinsam für =O. Bevorzugt stehen R₆ und R₆' jeweils unabhängig voneinander für -H, -F, -Cl, -NO₂, -CF₃, -OR₁₃, -SR₁₃, -S(=O)₂R₁₃, -S(=O)₂OR₁₃, -CN, -COOR₁₃, -NR₁₄R₁₅, -C₁₋₅-Aliphat, -C₃₋₈-Cycloaliphat, -Aryl- oder Heteroaryl; oder jeweils über C₁₋₃-Aliphat gebundenes -Aryl, -C₃₋₈-Cycloaliphat oder -Heteroaryl; oder R₅ und R₆ bedeuten bevorzugt gemeinsam -(CH₂)ₙ- mit n = 2, 3, 4, 5 oder 6, wobei einzelne Wasserstoffatome auch durch -F, -Cl, -Br, -I, -NO₂, -CF₃, -OR₁₃, -CN oder-C₁₋₅-Aliphat ersetzt sein können.

In einer bevorzugten Ausführungsform stehen R₆ und R₆' für -H. In einer anderen bevorzugten Ausführungsform steht R₆ für -H und R₆' ist ungleich -H. In einer anderen bevorzugten Ausführungsform sind sowohl R₆ als auch R₆' ungleich -H.

Bevorzugt sind auch Verbindungen, bei denen R₆ für -H, -C₁₋₅-Aliphat, -Aryl oder über eine -C₁₋₃-Aliphat-Gruppe (Brücke) verknüpftes -Aryl steht. Besonders bevorzugt steht R₆ für -H, -CH₃, -Phenyl oder -Benzyl. Ganz besonders bevorzugt steht R₆ für -H.

Bevorzugt stehen R₇, R₈, R₉ und R₁₀ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -CN, -COOR₁₃, -NR₁₄R₁₅; -C₁₋₅-Aliphat, -C₃₋₈-Cycloaliphat; -Aryl oder -Heteroaryl; oder jeweils über -C₁₋₃-Aliphat gebundenes -Aryl, -C₃₋₈-Cycloaliphat oder -Heteroaryl.

Bevorzugter stehen R₇, R₈, R₉ und R₁₀ jeweils unabhängig voneinander für -H, -Methyl, -Ethyl, -Propyl, -Butyl, -Pyridyl, -O-Benzyl, -F, -Cl, -Br, -I, -CF₃, -OH, -OCH₃, -NH₂, -COOH, -CO-OCH₃, -NHCH₃, -N(CH₃)₂ oder -NO₂.

Besonders bevorzugt stehen R₇, R₈, R₉ und R₁₀ jeweils unabhängig voneinander für -H, -F, -OH, -CH₃, -Cl, -OCH₃, -Br oder -NO₂.

In einer bevorzugten Ausführungsform stehen R₇, R₈, R₉ und R₁₀ für -H.

In einer anderen bevorzugten Ausführungsform stehen drei der Reste R₇, R₈, R₉ und R₁₀ für -H und der übrige Rest, vorzugsweise R₈ oder R₉, ist ungleich -H, vorzugsweise -F, -Cl, -OH oder -OCH₃.

In einer anderen bevorzugten Ausführungsform stehen zwei der Reste R₇, R₈, R₉ und R₁₀ für -H und die übrigen beiden Reste sind ungleich -H.

Bevorzugt steht R₁₁ für -H, -C₁₋₅-Aliphat, -C₃₋₈-Cycloaliphat, -Aryl, -Heteroaryl, -C₁₋₆-Aliphat-Aryl, -C₁₋₆-Aliphat-C₃₋₈-Cycloaliphat, -C₁₋₆-Aliphat-Heteroaryl, -C(=O)Aryl, -C(=O)Heteroaryl, oder -C(=O)C₁₋₆-Aliphat.

Bevorzugt steht R₁₂ für -H, -C₁₋₅-Aliphat, -C₃₋₈-Cycloaliphat, -Aryl- oder -Heteroaryl, oder jeweils über -C₁₋₃-Aliphat gebundenes -Aryl, -C₃₋₈-Cycloaliphat oder -Heteroaryl, oder für -OR₁₃ oder -NR₁₄R₁₅. Besonders bevorzugt ist R₁₂ über -C₂-Aliphat, vorzugsweise über -CH₂CH₂- oder -CH=CH-, gebundenes -Aryl, vorzugsweise Phenyl.

Bevorzugt steht R₁₃ für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl.

Bevorzugt stehen R₁₄ und R₁₅ unabhängig voneinander für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl; oder R₁₄ und R₁₅ bilden zusammen -CH₂CH₂OCH₂CH₂-, -CH₂CH₂N-R₁₆CH₂CH₂ oder -(CH₂)₃₋₆-.

Bevorzugt steht R₁₆ für -H oder -C₁₋₅-Aliphat.

Bevorzugt stehen R₁₈ und R₁₉ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)-NR₁₃, -C(=O)NR₀OR₀, -NR₁₄R₁₅, -NHC(=O)R₀, -NHC(=O)NHR₀, -NHC(=O)N(R₀)₂, -NHC(=O)-OR₀, -NHS(=O)₁₋₂R₀, oder -R₀; oder R₁₈ und R₁₉ stehen gemeinsam für =O. Bevorzugt stehen R₁₈ und R₁₉ jeweils unabhängig voneinander für -H, -F, -Cl, -NO₂, -CF₃, -OR₁₃, -SR₁₃, -S(=O)₂R₁₃, -S(=O)₂OR₁₃, -CN, -COOR₁₃, -NR₁₄R₁₅, -C₁₋₅-Aliphat, -C₁₋₅-Aliphat-OH, -C₃₋₈-Cycloaliphat, -Aryl- oder Heteroaryl; oder jeweils über C₁₋₃-Aliphat gebundenes -Aryl, -C₃₋₈-Cycloaliphat oder -Heteroaryl. Bevorzugter stehen R₁₈ und R₁₉ jeweils unabhängig voneinander für -H, -C₁₋₅-Aliphat, -C₃₋₈-Cycloaliphat, -Aryl oder -Heteroaryl; oder jeweils über -C₁₋₃-Aliphat gebundenes -Aryl, -C₃₋₈-Cycloaliphat oder -Heteroaryl.

In einer bevorzugten Ausführungsform stehen R₁₈ und R₁₉ für -H. In einer anderen bevorzugten Ausführungsform steht R₁₈ für -H und R₁₉ ist ungleich -H. In einer anderen bevorzugten Ausführungsform sind sowohl R₁₈ als auch R₁₉ ungleich -H.

Zum Zwecke der Beschreibung werden Kohlenwasserstoffreste unterteilt in aliphatische Kohlenwasserstoffreste einerseits und aromatische Kohlenwasserstoffreste andererseits.

Aliphatische Kohlenwasserstoffreste werden ihrerseits unterteilt in nicht-cyclische aliphatische Kohlenwasserstoffreste einerseits (= "Aliphat") und cyclische aliphatische Kohlenwasserstoffreste, d.h. alicyclische Kohlenwasserstoffreste, andererseits (= "Cycloaliphat"). Cycloaliphaten können monocyclisch oder multicyclisch sein. Alicyclische Kohlenwasserstoffreste ("Cycloaliphat") umfassen sowohl reine aliphatische Carbocyclen als auch aliphatische Heterocyclen, d.h. - sofern nicht ausdrücklich spezifiziert - umfasst "Cycloaliphat" reine aliphatische Carbocyclen (z.B. Cyclohexyl), reine aliphatische Heterocyclen (z.B. Piperidyl oder Piperazyl) sowie nicht-aromatische, multicyclische, ggf. gemischte Systeme (z.B. Decalinyl, Decahydrochinolinyl).

Aromatische Kohlenwasserstoffe werden ihrerseits unterteilt in carbocyclische aromatische Kohlenwasserstoffe einerseits (= "Aryl") und heterocyclische aromatische Kohlenwasserstoffe andererseits (= "Heteroaryl").

Die Zuordnung von multicyclischen, wenigstens teilaromatischen Systemen richtet sich vorzugsweise danach, ob wenigstens ein aromatischer Ring des multicyclischen Systems wenigstens ein Heteroatom (üblicherweise N, O oder S) im Ring aufweist. Ist wenigstens ein solches Heteroatom in diesem Ring vorhanden, handelt es sich bevorzugt um ein "Heteroaryl" (selbst wenn ggf. als zusätzlich vorhandener Cyclus des multicyclischen Systems ein weiterer carbocyclischer aromatischer oder nicht-aromatischer Ring mit oder ohne Heteroatom vorhanden ist); ist in keinem der ggf. mehreren aromatischen Ringe des multicyclischen Systems ein solches Heteroatom vorhanden, handelt es sich bevorzugt um "Aryl" (selbst wenn in einem ggf. zusätzlich vorhandenen, nicht-aromatischen Cyclus des multicyclischen Systems ein Ringheteroatom vorhanden ist). -

Innerhalb der cyclischen Substituenten gilt demnach bevorzugt folgende Priorität bei der Zuordnung: Heteroaryl > Aryl > Cycloaliphat.

Zum Zwecke der Beschreibung werden einbindige und mehrbindige, z.B. zweibindige Kohlenwasserstoffreste nicht begrifflich unterschieden, d.h. "C₁₋₃-Aliphat" umfasst, je nach Sinnzusammenhang, z.B. sowohl -C₁₋₃-Alkyl, -C₁₋₃-Alkenyl und -C₁₋₃-Alkinyl, als auch z.B. -C₁₋₃-Alkylen-, -C₁₋₃-Alkenylen- und C₁₋₃-Alkinylen.

Bevorzugt ist Aliphat jeweils ein verzweigter oder unverzweigter, gesättigter oder ein ein-oder mehrfach ungesättigter, unsubstituierter oder ein- oder mehrfach substituierter, aliphatischer Kohlenwasserstoffrest. Soweit Aliphat ein- oder mehrfach substituiert ist, sind die Substituenten unabhängig voneinander ausgewählt aus der Gruppe bestehend ausgewählt aus der Gruppe bestehend aus -F, -Cl, -Br, -I, -CN, -NO₂, -CHO, =O, -R₀, -C(=O)R₀, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -Si(R₀)₃, -PO(OR₀)₂. So umfasst "Aliphat" acyclische gesättigte oder ungesättigte Kohlenwasserstoffreste, die verzweigt oder geradkettig sein können, d.h. Alkanyle, Alkenyle und Alkinyle. Dabei weisen Alkenyle mindestens eine C=C-Doppelbindung und Alkinyle mindestens eine C≡C-Dreifachbindung auf. Bevorzugte unsubstituierte einbindige Aliphaten umfassen -CH₃, -CH₂CH₃, -CH₂CH₂CH₃, -CH(CH₃)₂, -CH₂CH₂-CH₂CH₃, -CH(CH₃)CH₂CH₃, -CH₂CH(CH₃)₂, -C(CH₃)₃, -CH₂CH₂CH₂CH₂CH₃ und -CH₂CH₂-CH₂CH₂-CH₂CH₃; aber auch -CH=CH₂, -C≡CH, -CH₂CH=CH₂, -CH=CHCH₃, -CH₂C≡CH, -C=CCH₃ und -CH=CHCH=CH₂. Bevorzugte unsubstituierte zweibindige Aliphaten umfassen -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- und -CH₂CH₂-CH₂CH₂-; aber auch -CH=CH-, -C≡C-, -CH₂CH=CH-, -CH=CHCH₂-, -CH₂C≡C- und -C≡CCH₂-. Bevorzugte substituierte einbindige Aliphaten umfassen -CH₂F, -CHF₂, -CF₃, -CH₂CF₃, -CF₂CF₃, -CH₂OH, -CH₂CH₂-OH, -CH₂CHOHCH₃, -CH₂OCH₃ und CH₂CH₂OCH₃. Bevorzugte substituierte zweibindige Aliphaten umfassen -CF₂-, -CF₂CF₂-, -CH₂CHOH-, -CHOHCH₂- und -CH₂CHOHCH₂-. Besonders bevorzugt sind Methyl, Ethyl, n-Propyl und n-Butyl.

Bevorzugt ist Cycloaliphat jeweils ein gesättigter oder ein ein- oder mehrfach ungesättigter, unsubstituierter oder ein- oder mehrfach substituierter, aliphatischer (d.h. nicht aromatischer), mono- oder multicyclischer Kohlenwasserstoffrest. Die Zahl der Ringkohlenstoffatome liegt vorzugsweise im angegebenen Bereich (d.h. ein "C₃₋₈-"Cycloaliphat weist vorzugsweise 3, 4, 5, 6, 7 oder 8 Ringkohlenstoffatome auf). Zum Zwecke der Beschreibung ist "C₃₋₈-Cycloaliphat" bevorzugt ein cyclischer Kohlenwasserstoff mit 3, 4, 5, 6, 7 oder 8 Ringkohlenstoffatomen, gesättigt oder ungesättigt, aber nicht aromatisch, wobei ggf. ein oder zwei Kohlenstoffatome unabhängig voneinander durch ein Heteroatom S, N oder O ersetzt sind. Soweit Cycloalkyl ein- oder mehrfach substituiert ist, sind die Substituenten unabhängig voneinander ausgewählt aus der Gruppe bestehend aus -F, -Cl, -Br, -I*,* -CN, -NO₂, -CHO, =O, -R_{0,} -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)-N(R₀)₂, -SH, -SR₀, -SO₃H, -S(=O)₁₋₂-R₀, -S(=)₁₂NH₂, -NH₂, -NHR₀, -N(R₀)₂, -N⁺(R₀)₃, -N+(R₀)₂O⁻, -NHC(=O)R₀, -NHC(=O)OR₀, -NHC(=O)NH₂, -NHC(=O)NHR₀, -NHC(=O)N(R₀)_{2,} -Si(R₀)₃, -PO(OR₀)₂. Vorteilhaft ist C₃₋₈-Cycloaliphat aus der Gruppe ausgewählt bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl.

Bevorzugt wird im Zusammenhang mit "Aliphat" bzw. "Cycloaliphat" unter "ein- oder mehrfach substituiert" die ein- oder mehrfache Substitution, z.B. einfache, zweifache, dreifache oder vierfache Substitution, eines oder mehrerer Wasserstoffatome durch -F, -Cl, -Br, -I, -OH, -OC₁₋₆-Alkyl, -OC(=O)C₁₋₆-Alkyl, -SH, -NH₂, -NHC₁₋₆-Alkyl, -N(C₁₋₆-Alkyl)₂, -C(=O)OC₁₋₆ Alkyl oder -C(=O)OH verstanden. Besonders bevorzugte Substituenten sind -F, -CI, -OH, -SH, -NH₂ und -C(=O)OH.

Unter mehrfach substituierten Resten sind solche Reste zu verstehen, die entweder an verschiedenen oder an gleichen Atomen mehrfach, z. B. zwei- oder dreifach, substituiert sind, beispielsweise dreifach am gleichen C-Atom, wie im Falle von -CF₃ oder -CH₂CF₃, oder an verschiedenen Stellen, wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfasst -OAliphat u.a. auch -OCH₂CH₂O-CH₂CH₂OH. Bevorzugt ist es, wenn Aliphat oder Cycloaliphat substituiert sind mit -F, -Cl, -Br, -I, -CN, -CH₃, -C₂H₅, -NH₂, -NO₂, -SH, -CF₃, -OH, -OCH₃, -OC₂H₅ oder -N(CH₃)₂. Ganz besonders bevorzugt ist es, wenn Aliphat oder Cycloaliphat substituiert sind mit -OH, -OCH₃ oder -OC₂H₅.

Bevorzugt steht Aryl jeweils unabhängig für ein carbocyclisches Ringsystem mit mindestens einem aromatischen Ring, aber ohne Heteroatome in diesem Ring, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können. Bevorzugte Aryle sind Phenyl, Naphthyl, Anthracenyl, Phenanthrenyl, Fluoranthenyl, Fluorenyl, Indanyl und Tetralinyl. Besonders bevorzugt sind Phenyl und Naphthyl. Soweit Aryl ein- oder mehrfach substituiert ist, können die Aryl-Substituenten gleich oder verschieden und in jeder beliebigen und möglichen Position des Aryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)N(R₀)₂, -Si(R₀)₃, -PO(OR₀)₂. Bevorzugte substituierte Aryle sind 2-Fluor-Phenyl, 3-Fluor-Phenyl, 4-Fluor-Phenyl, 2,3-Difluor-Phenyl, 2,4-Difluor-Phenyl, 3,4-Difluor-Phenyl, 2-Chlor-Phenyl, 3-Chlor-Phenyl, 4-Chlor-Phenyl, 2,3-Dichlor-Phenyl, 2,4-Dichlor-Phenyl, 3,4-Dichlor-Phenyl, 2-Methoxy-Phenyl, 3-Methoxy-Phenyl, 4-Methoxy-Phenyl, 2,3-Dimethoxy-Phenyl, 2,4-Dimethoxy-Phenyl, 3,4-Dimethoxy-Phenyl, 2-Methyl-Phenyl, 3-Methyl-Phenyl, 4-Methyl-Phenyl, 2,3-Dimethyl-Phenyl, 2,4-Dimethyl-Phenyl und 3,4-Dimethyl-Phenyl.

Bevorzugt steht Heteroaryl für einen 5-, 6- oder 7-gliedrigen cyclischen aromatischen Rest, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein- oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann. Bevorzugt ist "Heteroaryl" ausgewählt aus der Gruppe bestehend aus Pyrrolyl, Indolyl, Furyl (Furanyl), Benzofuranyl, Thienyl (Thiophenyl), Benzothienyl, Benzothiadiazolyl, Benzooxadiazolyl, Benzothiazolyl, Benzooxazolyl, Benzotriazolyl, Benzodioxolanyl, Benzodioxanyl, Phtalazinyl, Pyrazolyl, Imidazolyl, Thiazolyl, Oxazolyl, lsoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, wobei die Bindung über jedes beliebige und mögliche Ringglied des Heteroaryl-Restes erfolgen kann. Soweit Heteroaryl ein- oder mehrfach substituiert ist, können die Heteroaryl-Substituenten gleich oder verschieden und in jeder beliebigen und möglichen Position des Heteroaryls sein, und sind unabhängig voneinander ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)-NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NH-C(=O)R₀, -NHC(=O)OR₀, -NHC(=O)NH₂, -NHC(=O)NHR₀, -NHC(=O)N(R₀)₂, -Si(R₀)₃, -PO(OR₀)₂.

In Bezug auf "Aryl" oder "Heteroaryl" versteht man unter "ein- oder mehrfach substituiert" die ein- oder mehrfache, z.B. zwei-, drei- vier- oder fünffache, Substitution eines oder mehrerer Wasserstoffatome des Ringsystems.

Besonders bevorzugt sind die (Hetero-)Aryl-Substituenten unabhängig voneinander ausgewählt aus -F, -Cl, -Br, -I, -CN, -CHO, -CO₂H, -NH₂, -NO₂, -NHR₀, -N(R₀)₂, -N⁺(R₀)₃, -N⁺(R₀)₂O⁻, -SH, -SR₀, -OH, -OR₀, -C(=O)R₀, -CO₂R₀, -C(=O)NH₂, -C(=O)NHR₀, -C(=O)N(R₀)₂, -S(=O)₁-₂R₀, -S(=O)₂NH₂, -SO₃H, =O oder -Ro. Bevorzugte Substituenten sind -F, -Cl, -Br, -I, -OH, -OC₁₋₆-Alkyl, -O-C(=O)-C₁₋₆-Alkyl, -SH, -NH₂, -NHC₁₋₆-Alkyl, -N(C₁₋₆-Alkyl)₂, -C(=O)OC₁₋₆-Alkyl oder -C(=O)OH. Besonders bevorzugte Substituenten sind -F, -Cl, -OH, -SH, -NH₂ und -C(=O)OH.

Die erfindungsgemäßen Verbindungen können in Form eines einzelnen Stereoisomers oder deren Mischung, der freien Verbindungen und/oder ihrer physiologisch verträglichen Salze und/oder Solvate vorliegen.

Die erfindungsgemäßen Verbindungen kommen je nach Substitutionsmuster am Cyclohexanring in Form von Stereisomeren vor (cis-trans-, Z/E- bzw. syn-anti-Isomere).

In einer bevorzugten Ausführungsform beträgt der Diastereomerenüberschuss des cis-Isomers wenigstens 50%de, bevorzugter wenigstens 75%de, noch bevorzugter wenigstens 90%de, am bevorzugtesten wenigstens 95%de und insbesondere wenigstens 99%de. In einer anderen bevorzugten Ausführungsform beträgt der Diastereomerenüberschuss des trans-Isomers wenigstens 50%de, bevorzugter wenigstens 75%de, noch bevorzugter wenigstens 90%de, am bevorzugtesten wenigstens 95%de und insbesondere wenigstens 99%de. Beide Diastereomere unterscheiden sich in ihrer Polarität, so dass nachfolgend das unpolare Diastereomer vom polaren Diastereomer unterschieden wird. Beide Diastereomere liegen (im Falle zweier Stereozentren) als Enantiomerenpaare vor (RR+SS bzw. RS+SR).

Geeignete Methoden zur Trennung der Isomere (Diastereomere) sind dem Fachmann bekannt. Als Beispiele können Säulenchromatographie, präparative HPLC und Kristallisationsverfahren genannt werden. Die Polarität ist beispielsweise für die Reihenfolge verantwortlich, in der beide Diastereomere bei der Dünnschichtchromatographie eluiert werden (keine *reversed-phase* Bedingungen).

Die erfindungsgemäßen Verbindungen können je nach Substitutionsmuster chiral oder achiral sein.

Sind die erfindungsgemäßen Verbindungen chiral, so liegen sie vorzugsweise als Racemat oder in angereicherter Form eines Enantiomers vor. In einer bevorzugten Ausführungsform beträgt der Enantiomerenüberschuss (ee) des S-Enantiomers wenigstens 50%ee, bevorzugter wenigstens 75%ee, noch bevorzugter wenigstens 90%ee, am bevorzugtesten wenigstens 95%ee und insbesondere wenigstens 99%ee. In einer anderen bevorzugten Ausführungsform beträgt der Enantiomerenüberschuss (ee) des R-Enantiomers wenigstens 50%ee, bevorzugter wenigstens 75%ee, noch bevorzugter wenigstens 90%ee, am bevorzugtesten wenigstens 95%ee und insbesondere wenigstens 99%ee.

Geeignete Methoden zur Trennung der Enantiomere sind dem Fachmann bekannt. Als Beispiele können präparative HPLC an chiralen stationären Phasen und Überführung in diastereomere Intermediate genannt werden. Die Überführung in diastereomere Intermediate kann beispielsweise als Salzbildung mit Hilfe chiraler, enantiomerenreiner Säuren erfolgen. Nach der Trennung der so gebildeten Diastereomere kann das Salz dann wieder in die freie Base oder ein anderes Salz überführt werden.

Sofern nicht ausdrücklich spezifiziert umfasst jeder Verweis auf die erfindungsgemäßen Verbindungen alle Isomere (z.B. Stereoisomere, Diastereomere, Enantiomere) in beliebigem Mischungsverhältnis.

Sofern nicht ausdrücklich spezifiziert umfasst jeder Verweis auf die erfindungsgemäßen Verbindungen die freien Verbindungen (d.h. die Formen, welche nicht als Salz vorliegen) und alle physiologisch verträglichen Salze.

Zum Zwecke der Beschreibung liegen physiologisch verträgliche Salze der erfindungsgemäßen Verbindungen vor als Salze mit Anionen oder Säuren der jeweiligen Verbindung mit anorganischen bzw. organischen Säuren, die physiologisch - insbesondere bei Anwendung im Menschen und/oder Säugetier - verträglich sind.

Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure. Besonders bevorzugt sind das Hydrochlorid, das Citrat und das Hemicitrat.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Die erfindungsgemäßen Verbindungen sind durch Substituenten definiert, beispielsweise durch R₁, R₂ und R₃ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise Y₁ = -R₀ wobei R₀ = -C₁₋₈-Aliphat (Substituent der 1. Generation), so kann -C₁₋₈-Aliphat seinerseits substituiert sein, z.B. mit -OR₀ wobei R₀ = -Aryl (Substituent der 2. Generation). Es ergibt sich daraus die funktionelle Gruppe -C₁₋₈-Aliphat-OAryl. -Aryl kann dann seinerseits erneut substituiert sein, z.B. mit -Cl (Substituent der 3. Generation). Es ergibt sich daraus dann insgesamt die funktionelle Gruppe -C₁₋₈-Aliphat-OAryl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für R₀ bis R₁₉ jeweils ggf. substituiert sein, die jeweiligen Substituenten können dann ihrerseits jedoch nicht erneut substituiert sein.

In einer anderen bevorzugten Ausführungsform können bereits die Substituenten der 1. Generation nicht erneut substituiert sein, d.h. es gibt dann weder Substituenten der 2. noch Substituenten der 3. Generation. Mit anderen Worten können in dieser Ausführungsform die funktionellen Gruppen für R₀ bis R₁₉ jeweils nicht substituiert sein.

Bevorzugt sind Verbindungen, wobei "Aliphat substituiert" oder "Cycloaliphat, substituiert" Aliphat oder Cycloaliphat substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet; und "Aryl substituiert" oder "Heteroaryl substituiert" Aryl oder Heteroaryl substituiert mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ bedeutet, in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Für eine bevorzugte Ausführungsform der erfindungsgemäßen Verbindungen gilt, dass R₁ und R₂ gemeinsam einen Ring bilden und für -CH₂CH₂CH₂- oder -CH₂CH₂CH₂CH₂- stehen. Besonders bevorzugt sind Verbindungen, worin R₁ und R₂ einen Ring bilden und gemeinsam -CH₂CH₂CH₂- bedeuten.

Weiterhin bevorzugt sind auch Verbindungen, worin R₃ für Phenyl, Benzyl oder Phenethyl, jeweils unsubstituiert oder am Ring ein- oder mehrfach substituiert; -C₁₋₅-Alkyl, unsubstituiert oder ein- oder mehrfach substituiert; -C₄₋₆-Cycloalkyl, unsubstituiert oder ein- oder mehrfach substituiert; -Pyridyl, -Thienyl, -Thiazolyl, -Imidazolyl, -1,2,4 Triazolyl oder -Benzimidazolyl, unsubstituiert oder ein- oder mehrfach substituiert, steht.

Besonders bevorzugt sind Verbindungen, worin R₃ -Phenyl, -Benzyl, -Phenethyl, -Thienyl, -Pyridyl, -Thiazolyl, -Imidazolyl, -1,2,4 Triazolyl, -Benzimidazolyl oder -Benzyl, unsubstituiert oder ein- oder mehrfach substituiert mit -F, -Cl, -Br, -CN, -CH₃, -C₂H₅, -NH₂,-NO₂, -SH, -CF₃, -OH, -OCH₃, -OC₂H₅ oder -N(CH₃)₂; -Ethyl, -n-Propyl, -2-Propyl, -Allyl, -n-Butyl, -iso-Butyl, -sec.-Butyl, -tert.-Butyl, -n-Pentyl, -iso-Pentyl, -neo-Pentyl, -n-Hexyl, -Cyclopentyl oder -Cyclohexyl steht, jeweils unsubstituiert oder ein- oder mehrfach substituiert mit -OH, -OCH₃ oder -OC₂H₅, wobei -Thienyl, -Pyridyl, -Thiazolyl, -imidazolyl, -1,2,4 Triazolyl und -Benzimidazolyl vorzugsweise unsubstituiert sind; insbesondere -Phenyl, unsubstituiert oder einfach substituiert mit -F, -Cl, -CN, -CH₃; -Thienyl; -Ethyl, -n-Propyl oder -n-Butyl, unsubstituiert oder ein- oder mehrfach substituiert mit -OCH₃, -OH oder -OC₂H₅, insbesondere mit -OCH₃.

Für eine bevorzugte Ausführungsform der erfindungsgemäßen Verbindungen gilt, dass R₅ für -H, -CH₃, -COOH, -COOCH₃, -CH₂O-Phenyl, wobei der Phenylrest mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ substituiert sein kann, oder -CH₂OH steht. Besonders bevorzugt sind Verbindungen, worin R₅ für H steht.

Bevorzugt sind auch Verbindungen, worin R₆ -H; -Methyl, -Ethyl, -CF₃, -Benzyl oder -Phenyl bedeuten kann, wobei der Benzyl- oder Phenylrest mit -F, -Cl, -Br, -I, -CN, -CH₃, -C₂H₅, -NH₂, -NO₂, -SH, -CF₃, -OH, -OCH₃, -OC₂H₅ oder -N(CH₃)₂ substituiert sein kann. Besonders bevorzugt sind spirocyclische Cyclohexanderivate, worin R₆ H bedeutet.

Bevorzugt sind weiterhin Verbindungen, worin R₇, R₈, R₉ und R₁₀ unabhängig voneinander -H; -C₁₋₅-Alkyl, verzweigt oder unverzweigt, unsubstituiert oder ein- oder mehrfach substituiert; -F, -Cl, -Br, -I, -CF₃, -OH, -OCH₃, -NH₂, -COOH, -COOCH₃, -NHCH₃, -Thienyl, -Pyrimidinyl, -Pyridyl, -N(CH₃)₂ oder -NO₂ bedeuten; vorzugsweise einer der Reste R₇, R₈, R₉ und R₁₀ für -H; -C₁₋₅-Alkyl, verzweigt oder unverzweigt, unsubstituiert oder ein- oder mehrfach substituiert; -F, -Cl, -Br, -I, -OH, -OCH₃, -COOH, -COOCH₃, -NH₂, -NHCH₃ oder -N(CH₃)₂ oder -NO₂ steht, während die übrigen Reste -H sind; oder zwei der Reste R₇, R₈, R₉ und R₁₀ unabhängig voneinander für -H; -C₁₋₅-Alkyl, verzweigt oder unverzweigt, unsubstituiert oder ein- oder mehrfach substituiert; -F, -Cl, -Br, -I, -OH, -OCH₃. -COOH, -COOCH₃, -NH₂, -NHCH₃ oder -N(CH₃)₂ oder -NO₂ stehen, während die übrigen Reste -H sind. Besonders bevorzugt sind spirocyclische Cyclohexanderivate, worin R₇, R₈. R₉ und R₁₀ unabhängig voneinander für -H, -F, -OH, -Cl oder -OCH₃ stehen.

Bevorzugte Ausführungsformen der erfindungsgemäßen Verbindungen der allgemeinen Formeln (2) haben die allgemeine Formel (2.1) oder (2.2) bzw. (2.3) oder (2.4):

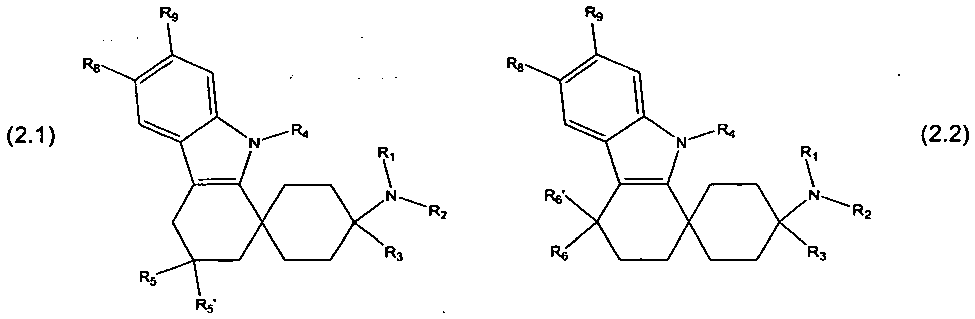

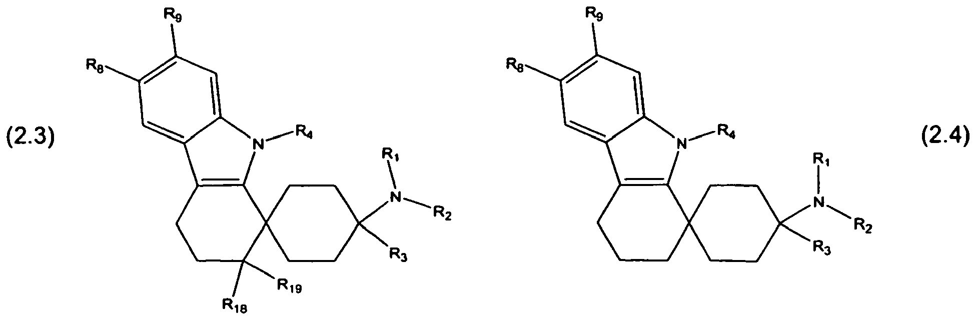

Besonders bevorzugte Vertreter der Verbindungen der allgemeinen Formel (2.1), (2.2), (2.3) bzw. (2.4) sind nachfolgend aufgeführt:

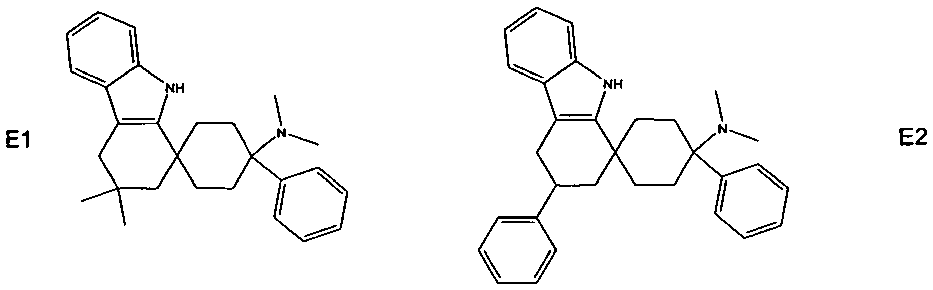

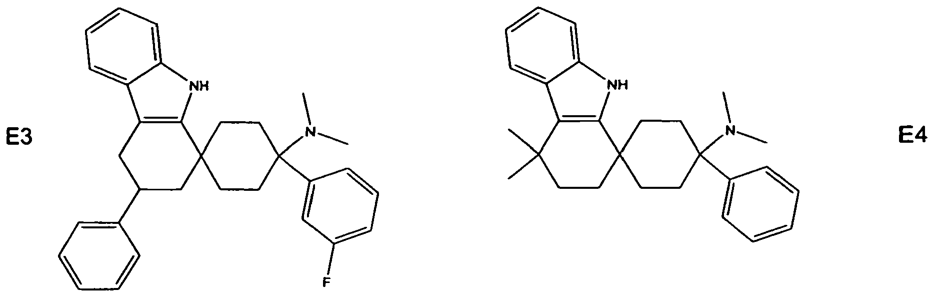

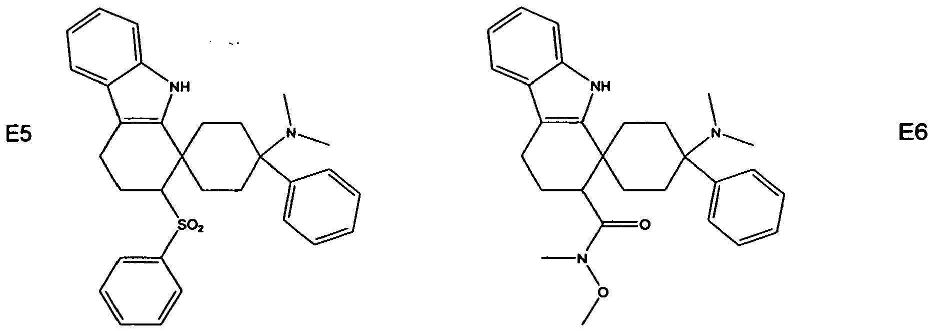

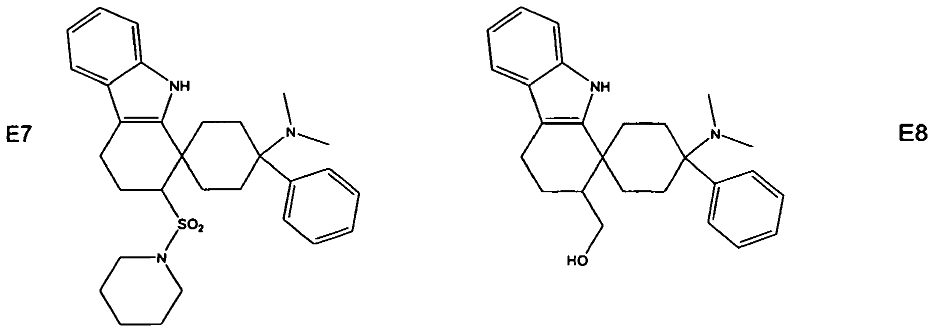

Bevorzugte Ausführungsformen der erfindungsgemäßen Verbindungen der allgemeinen Formeln (2.1) haben die allgemeine Formel (2.1.1): 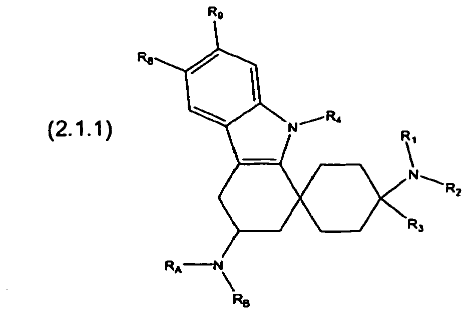 worin R_{A} und R_{B} unabhängig voneinander ausgewählt sind aus der Gruppe bestehend aus -H, -C₁₋₆-Aliphat, -C(=O)-C₁₋₆-Aliphat, -C(=O)-C₃₋₈-Cycloaliphat, -C(=O)-C₁₋₆-Aliphat-C₃₋₈-Cycloaliphat, -C(=O)-C₁₋₆-Aliphat-Aryl, -C(=O)-C₁₋₆-Aliphat-Heteroaryl, -C(=O)-Aryl, -C(=O)-Heteroaryl, -C(=O)-NHC₁₋₆-Aliphat, -C(=ONHC₃₋₈-Cycloaliphat, -C(=O)-NHC₁₋₆-Aliphat-C₃₋₈-Cycloaliphat, -C(=O)-NHC₁₋₆-Aliphat-Aryl, -C(=O)-NHC₁₋₆-Aliphat-Heteroaryl, -C(=O)-NHAryl, -C(=O)-NHHeteroaryl, -C(=O)-N(C₁₋₆-Aliphat)₂, -C(=O)-N(C₃₋₈-Cycloaliphath, -C(=O)-N(C₁₋₆-Aliphat-C₃₋₈-Cycloaliphat)₂, -C(=O)-N(C₁₋₆-Aliphat-Aryl)₂, -C(=O)-N(C₁₋₆-Aliphat-Heteroaryl)₂, -C(=O)-N(Aryl)₂, -C(=O)-N(Heteroaryl)₂, -C(=O)-OC₁₋₆-Aliphat, -C(=O)-OC₃₋₈-Cycloaliphat, -C(=O)-OC₁₋₆-Aliphat-C₃₋₈-Cycloaliphat, -C(=O)-OC₁₋₆-Aliphat-Aryl, -C(=O)-OC₁₋₆-Aliphat-Heteroaryl, -C(=O)-OAryl und -C(=O)-OHeteroaryl. Vorzugsweise ist R_{A} -H und R_{B} ungleich -H.

Besonders bevorzugte Vertreter der Verbindungen der allgemeinen Formel (2.1.1) sind nachfolgend aufgeführt:

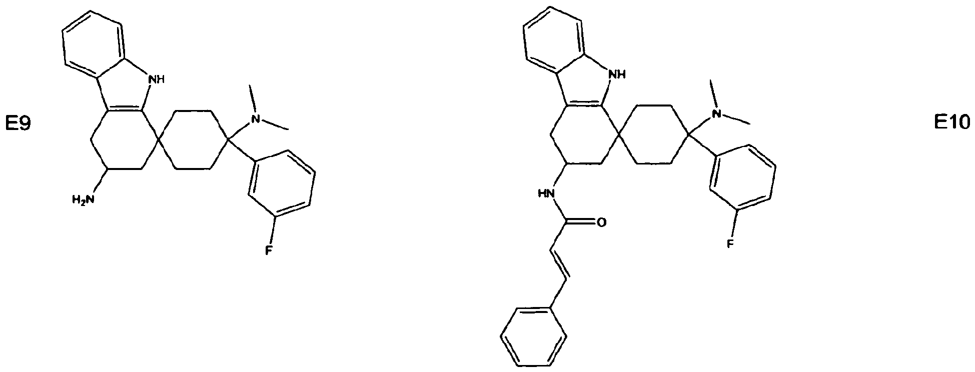

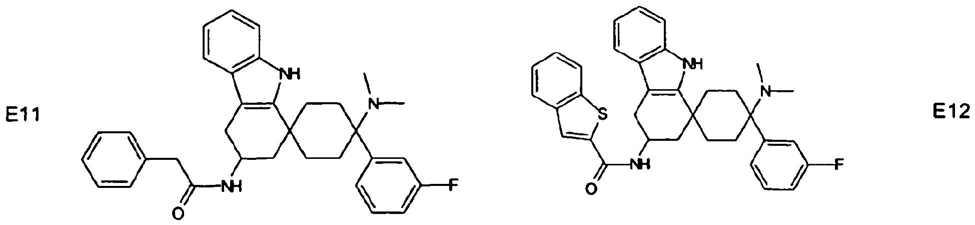

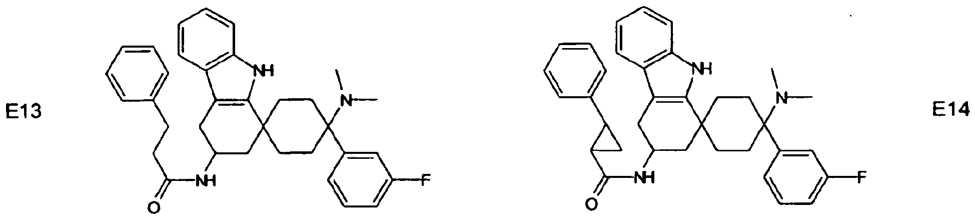

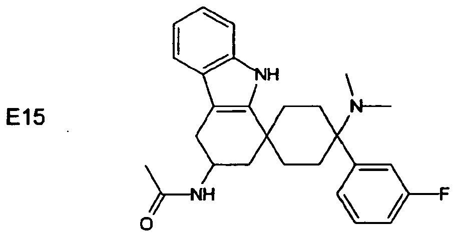

Andere bevorzugte Ausführungsformen der erfindungsgemäßen Verbindungen der allgemeinen Formeln (2) haben die allgemeine Formel (2.5), (2.6), (2.7) oder (2.8):

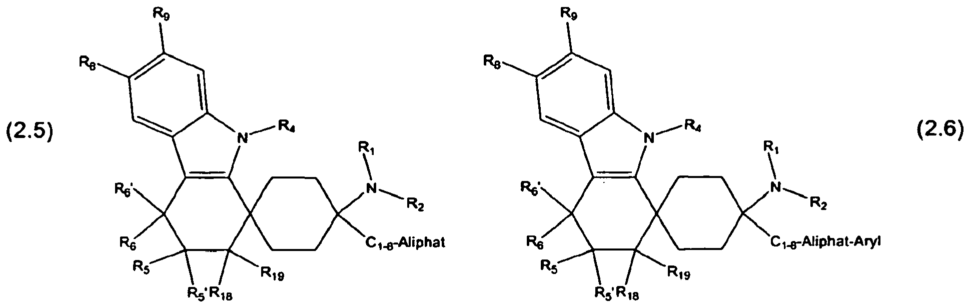

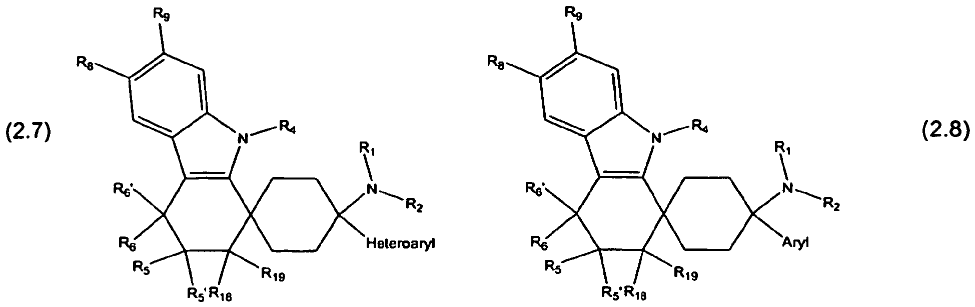

Bevorzugte Vertreter der Verbindungen der allgemeinen Formeln (2.5), (2.6), (2.7) oder (2.8) sind nachfolgend abgebildet:

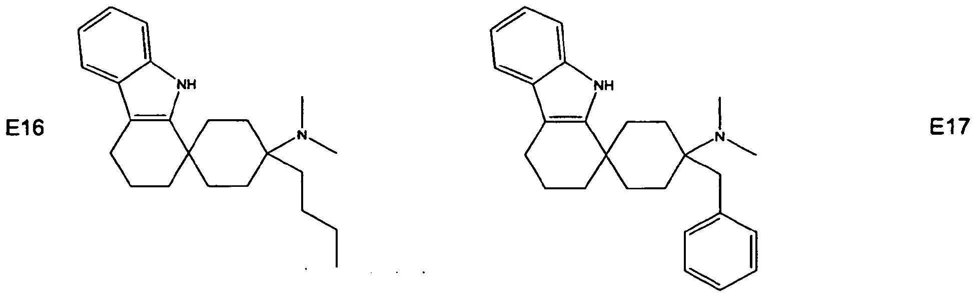

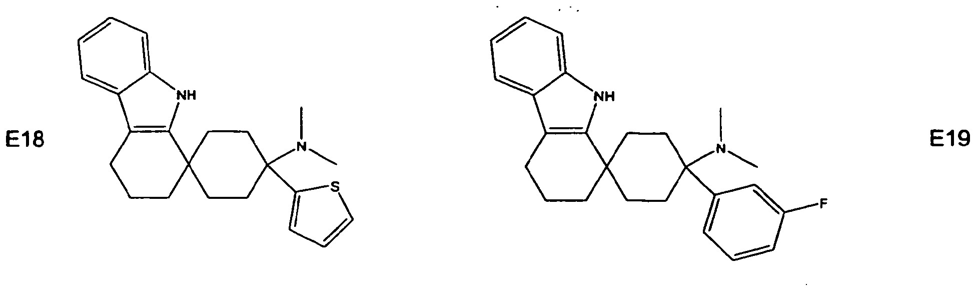

Andere bevorzugte Ausführungsformen der erfindungsgemäßen Verbindungen der allgemeinen Formeln (2) haben die allgemeine Formel (2.9) oder (2.10):

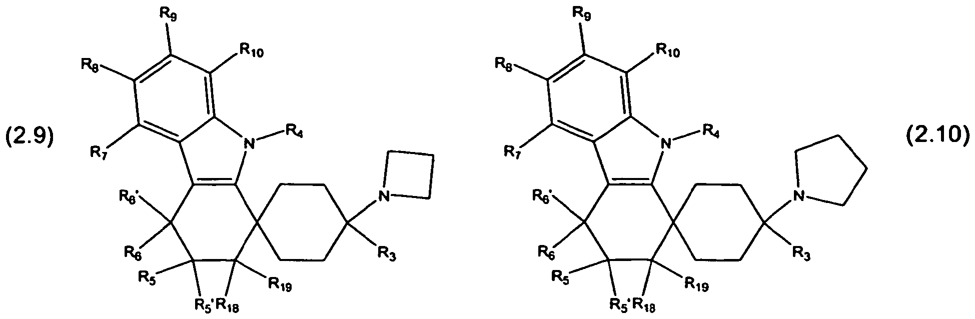

Bevorzugte Vertreter der Verbindungen der allgemeinen Formeln (2.9) bzw. (2.10) sind nachfolgend abgebildet:

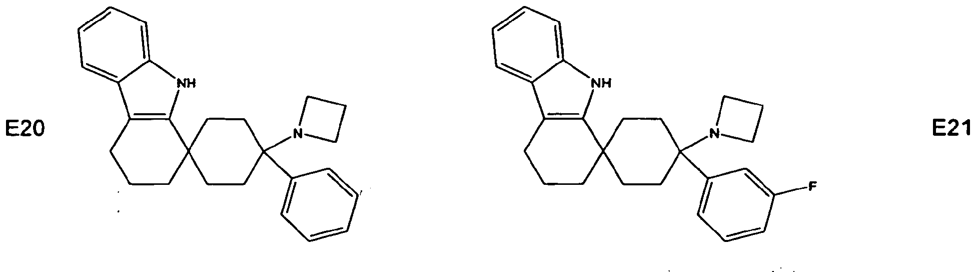

Erfindungsgemäß besonders bevorzugt sind Verbindungen der allgemeinen Formel (6) 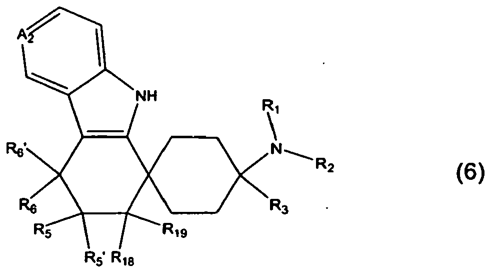 worin
A₂ für -N= oder -CR₈= steht,
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ für -CH₃ steht;
R₂ für -H oder -CH₃ steht;
oder R₁ und R₂ zusammen für -(CH₂)₃₋₄- stehen;
R₃ für -C₁₋₆-Aliphat, -Aryl, -Heteroaryl, -C₁₋₆-Aliphat-Aryl oder -C₁₋₆-Aliphat-Heteroaryl;
R₅, R₅ , R₆, R₆', R₈, R₁₈ und R₁₉ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)-NR₁₃, -C(=O)NR₀OR₀, -NR₁₄R₁₅, -NHC(=O)R₀, -NHC(=O)NHR₀, -NHC(=O)N(R₀)₂, -NHC(=O)-OR₀, -NHS(=O)₁₋₂R₀, =O oder -R₀ stehen;
R₁₃ jeweils unabhängig für -H oder -R₀ steht;
R₁₄ und R₁₅ unabhängig voneinander für -H oder -R₀ stehen.

Ganz besonders bevorzugt sind Verbindungen aus der Gruppe bestehend aus N,N,3,3-Tetramethyl-4'-phenyl-2,3,4,9-tetrahydrospiro[carbazole-1,1'-cyclohexan]-4'-amin; N,N-Dimethyl-3,4'-diphenyl-2,3,4,9-tetrahydrospiro[carbazol-1,1'-cyclohexan]-4'-amin; 4'-(3-Fluorphenyl)-N,N-dimethyl-3-phenyl-2,3,4,9-tetrahydrospiro[carbazol-1,1'-cyclohexan]-4'-amin; N,N,4,4-Tetramethyl-4'-phenyl-2,3,4,9-tetrahydrospiro[carbazol-1,1'-cyclohexan]-4'-amin; N,N-Dimethyl-4'-phenyl-2-(phenylsulfonyl)-2,3,4,9-tetrahydrospiro[carbazol-1,1'-cyclohexan]-4'-amin; 4'-(Dimethylamino)-N-methoxy-N-methyl-4'-phenyl-2,3,4,9-tetrahydrospiro-[carbazol-1,1'-cyclohexan]-2-carboxamid; N,N-Dimethyl-4'-phenyl-2-(piperidin-1-ylsulfonyl)-2,3,4,9-tetrahydrospiro[carbazol-1,1'-cyclohexan]-4'-amin; (4'-(Dimethylamino)-4'-phenyl-2,3,4,9-tetrahydrospiro[carbazole-1,1'-cyclohexane]-2-yl)methanol; N4',N4'-dimethyl-4'-(3-fluorphenyl)-2,3,4,9-tetrahydrospiro[carbazol-1,1'-cyclohexan]-3,4'-diamin; N-(4'-(Dimethylamino)-4'-(3-fluorophenyl)-2,3,4,9-tetrahydrospiro[carbazol-1,1'-cydohexan]-3-yl)cinnamamid; N-(4'-(Dimethylamino)-4'-(3-fluorophenyl)-2,3,4,9-tetrahydrospiro[carbazole-1,1'-cyclohexan]-3-yl)-2-phenylacetamid; N-(4'-(Dimethy)amino)-4'-(3-ftuoropheny))-2,3,4,9-tetrahydrospiro[carbazol-1,1'-cyclohexan]-3-yl)benzo[b]thiophen-2-carboxamid; N-(4'-(Dimethylamino)-4'-(3-fluorphenyl)-2,3,4,9-tetrahydrospiro[carbazole-1,1'-cyclohexan]-3-yl)-3-phenylpropanamid; N-(4'-(Dimethylamino)-4'-(3-fluorophenyl)-2,3,4,9-tetrahydrospiro[carbazol-1,1'-cyclohexan]-3-yl)-2-phenylcyclopropanecarboxamid; N-(4'-(Dimethylamino)-4'-(3-fluorpheny)-2,3,4,9-tetrahydrospiro[carbazol-1,1'-cyclohexan]-3-yl)acetamid; 4'-Butyl-N,N-dimethyl-2,3,4,9-tetrahydrospiro[carbazol-1,1'-cyclohexan]-4'-amin; 4'-Benzyl-N,N-dimethyl-2,3,4,9-tetrahydrospiro[carbazol-1,1'-cyclohexan]-4'-amin; N,N-Dimethyl-4'-(thiophen-2-yl)-2,3,4,9-tetrahydrospiro[carbazol-1,1'-cyclohexan]-4'-amin; 4'-(3-Fluorphenyl)-N,N-dimethyl-2,3,4,9-tetrahydrospiro[carbazol-1,1'-cyclohexan]-4'-amin; 4'-(Azetidin-1-yl)-4'-phenyl-2,3,4,9-tetrahydrospiro[carbazol-1,1'-cyclohexan]; 4'-(Azetidin-1-yl)-4'-(3-fluorphenyl)-2,3,4,9-tetrahydrospiro[carbazol-1,1'-cyclohexan], 4'-(Acetidin-1-yl)-4'-phenyl-2,3,4,9-tetrahydrospiro[carbazol-1,1'-cyclohexan] (GRT15126H), N,N-Dimethyl-N-(4-butyl-2',3',4',9'-tetrahydro-1H-spiro[cyclohexan-1,1'-carbazol]-4-yl)amin , 4'-Benzyt-N,N-dimethyl-2,3,4,9-tetrahydrospiro[carbazol-1,1'-cyclohexan]-4'-amin , 4'-(3-Fluorphenyl)-N,N-dimethyl-2;3,4,9-tetrahydrospiro[carbazol-1,1'-cyclohexan]-4'-amin , N,N-Dimethyl-4'-(thiophen-2-yl)-2,3,4,9-tetrahydrospiro[carbazol-1,1'-cyclohexan]-4'-amin, (S)-N,N,3-Trimethyl-4'-phenyl-2,3,4,9-tetrahydrospiro[carbazol-1,1'-cyclohexan]-4'-amin oder deren physiologisch verträgliche Salze und/oder Solvate.

Die erfindungsgemäßen Verbindungen wirken beispielsweise auf den im Zusammenhang mit verschiedenen Erkrankungen relevanten ORL1-Rezeptor, sodass sie sich als pharmazeutischer Wirkstoff in einem Arzneimittel eignen.

Ein weiterer Gegenstand der Erfindung betrifft daher Arzneimittel, welche wenigstens eine erfindungsgemäße Verbindung enthalten, sowie ggf. geeignete Zusatz- und/oder Hilfsstoffe und/oder ggf. weitere Wirkstoffe.

Die erfindungsgemäßen Verbindungen weisen eine vergleichbare Affinität zum µ-Opioid- bzw. zum ORL1-Rezeptor auf wie die Verbindungen, die als Beispielverbindungen in WO 2004/043967 offenbart sind. Im Vergleich zu diesen Verbindungen zweigen sie jedoch eine größere Selektivität gegenüber dem kappa-Opioid-Rezeptor, welcher für Nebenwirkungen, wie beispielsweise Dysphorie, Sedation und Diurese verantwortlich ist. Daher eignen sie sich besonders für die Arzneimittelentwicklung.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfinungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,001 bis 0,5 mg/kg wenigstens einer erfindungsgemäßen Verbindung appliziert.

Für alle vorstehenden Formen der erfindungsgemäßen Arzneimittel ist es besonders bevorzugt, wenn das Arzneimittel neben wenigstens einer erfindungsgemäßen Verbindung noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittels liegt eine enthaltene erfindungsgemäße Verbindung als reines Diastereomer und/oder Enantiomer vor.

Der ORL1-Rezeptor wurde insbesondere im Schmerzgeschehen identifiziert. Entsprechend können erfindungsgemäße Verbindungen zur Herstellung eines Arzneimittels zur Behandlung von Schmerz, insbesondere von akutem, neuropathischem oder chronischem Schmerz, verwendet werden.

Ein weiterer Gegenstand der Erfindung betrifft daher die Verwendung einer erfindungsgemäßen Verbindung zur Herstellung eines Arzneimittels zur Behandlung von Schmerz, insbesondere von akutem, viszeralem, neuropathischem oder chronischem Schmerz.

Ein weiterer Gegenstand der Erfindung betrifft die Verwendung einer erfindungsgemäßen Verbindung zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Haminkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn eine verwendete Verbindung als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung betrifft ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis einer erfindungsgemäßen Verbindung, oder eines erfindungsgemäßen Arzneimittels.

Ein weiterer Gegenstand der Erfindung betrifft ein Verfahren zur Herstellung der erfindungsgemäßen Verbindungen wie in der folgenden Beschreibung und Beispielen ausgeführt.

In einer bevorzugten Ausführungsform folgt die Synthese der erfindungsgemäßen Verbindungen dem folgenden allgemeinen Syntheseschema:

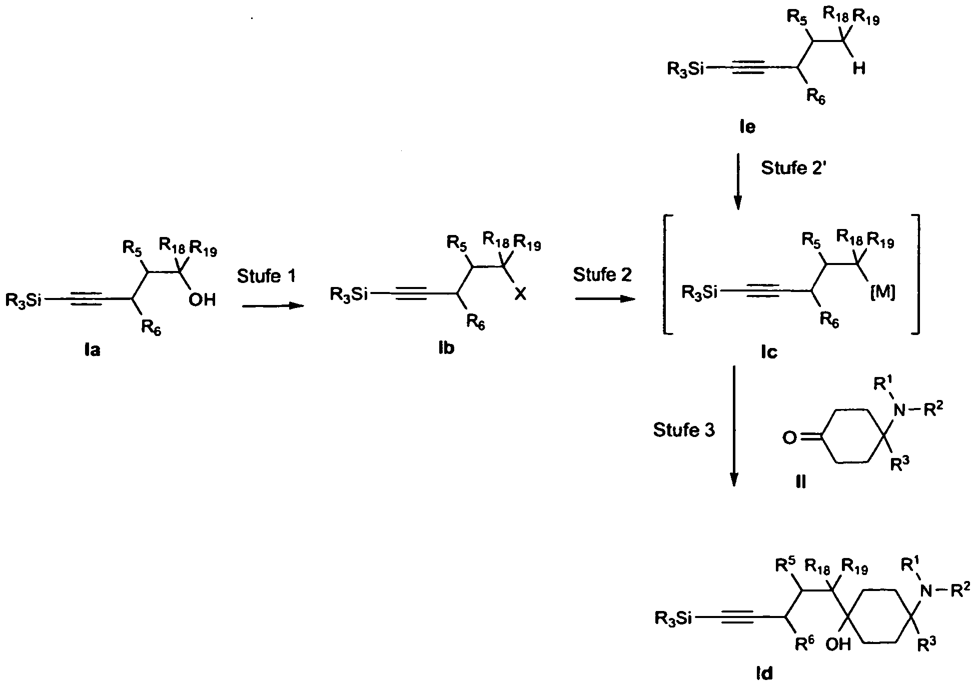

In Stufe 1 werden Alkohole der allgemeinen Formel Ia entweder nach Überführung in eine Abgangsgruppe (z. B. -OSO₂-Me -OSO₂-p-Toluol, -OTf) oder direkt (im Sinne einer Mukaiyama Redoxkondensation) in Halogenide der allgemeinen Formel Ib (X= Cl, Br, I) überführt. Diese werden durch Halogen-Metallaustausch entweder zu den entsprechenden Lithiumorganylen ([M]=Li]) oder Grignardreagenzien ([M]=MgX]) vom Typ Ic umgesetzt (Stufe 2). Alternativ dazu werden in Stufe 2' ausgehend von Alkinen der allgemeinen Formel Ie (mit R₁₈ oder R₁₉ = z.B. SO₂Ph, SOPh, -CN, -C(=O)N(CH₃)OCH₃) durch Deprotonierung mit Lithiumamiden (z. B. LDA) Lithiumorganyle vom Typ Ic hergestellt. In Stufe 3 werden die metalierten Organyle der allgemeinen Formel lc im Sinne einer 1,2-Addition an die Carbonylgruppe von Cyclohexanonen der allgemeinen Formel II zu den entsprechenden Alkinbausteinen vom Typ Id umgesezt. Die Synthesen der Cycloxexanonderivate mit der allgemeinen Formel II sind in der Literatur bekannt (vgl. z.B. W005066183, WO040043967, W00290317, US 4065573, Lednicer et al., J. Med. Chem., 23, 1980, 424-430).

In einer anderen bevorzugten Ausführungsform folgt die Synthese der erfindungsgemäßen Verbindungen dem folgenden allgemeinen Syntheseschema (Larockreaktion und Spirozyklisierung):

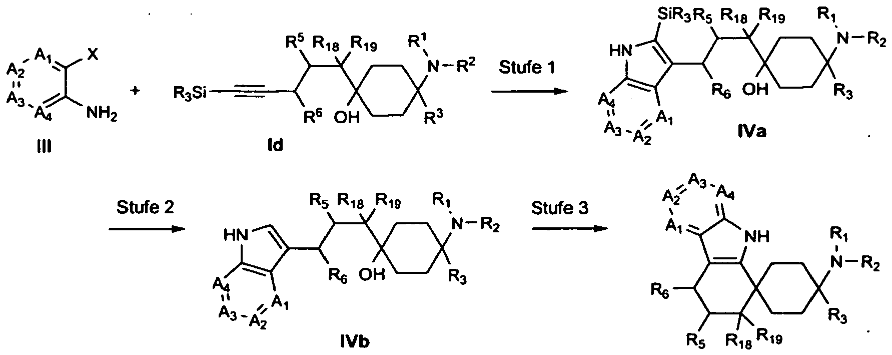

In Stufe 1 werden Verbindungen der allgemeinen Formel III, worin X für einen Halogenrest oder einen Sulfonsäureester steht, im Sinne einer Indolsynthese nach Larock unter Zusatz eines Palladiumkatalysators mit Alkinen der allgemeinen Formel Id zu Indolen der allgemeinen Formel IVa umgesetzt. Verbindungen der allgemeinen Formel III sind kommerziell erhältlich (exemplarische Synthesen siehe auch W02008009416). In Stufe 2 werden Verbindungen der allgememeinen Formel IVa in Gegenwart von Fluorid oder in Gegenwart einer oganischen oder anorganischen Säure desilyliert und zu Verbindungen der allgemeinen Formel IVb umgesetzt. Zur Herstellung der spirocyclischen Verbindungen der allgemeinen Formel 1 werden die Alkohole der allgemeinen Formel IVb unter Zusatz einer organischen Säure oder deren Trimethylsilylester oder einer anorganischen Säure oder unter Zuatz eines Übergangsmetallsalzes umgesetzt.

Bezüglich weiterer Einzelheiten zur Synthese der erfindungsgemäßen Verbindungen, insbesondere im Hinblick auf die Synthese geeigneter Eduktbausteine, wird ferner vollumfänglich verwiesen auf WO2004/043967, WO2005/063769, WO2005/066183, WO2006/018184, WO2006/108565, WO2007/124903 und WO2008/009416. Ein Fachmann erkennt, dass geeignete Eduktbausteine für die Synthese der erfindungsgemäßen Verbindungen in Analogie zu den in diesen Druckschriften offenbarten Syntheseschemata und Ausführungsbeispielen hergestellt werden können.

### Beispiele:

Die folgenden Beispiele dienen zur näheren Erläuterung der Erfindung, sind jedoch nicht einschränkend auszulegen.

Die Ausbeuten der hergestellten Verbindungen sind nicht optimiert. Alle Temperaturen sind unkorrigiert. Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei),"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt. Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Laufmitteln für chromatographische Untersuchungen sind stets in Volumen/Volumen angegeben.

### Synthesevorschriften

### a) Bausteinsynthesen- Alkine

*Triphenylphosphindiiodid*Eine Suspension von lod (13.0 g, 51 mmol) und Toluol (100 mL) wurde 20 min unter Rückfluss erhitzt. Die entstandene Lösung wurde auf Raumtemperatur abgekühlt und zu einer Lösung von Triphenylphosphin (14.0 g, 53 mmol) in wasserfreiem Diethylether (100 mL) getropft. Der ausgefallene gelbe Niederschlag wurde abfiltriert und mit Diethylether gewaschen.
*Ausbeute:* 26.4 g (96 %), gelber Feststoff
*Schmelzpunkt:* 140-145 °C

*Triethyl-(5-iodpent-1-inyl)silanZu* einer Lösung von Triphenylphosphindiiodid (2.80 g, 5.4 mmol) und Imidazol (1.04 g, 15.4 mmol) in absolutem Acetonitril (30 mL) wurde unter Argon eine Lösung von 5-(Triethylsilyl)-4-pentin-1-ol (416 mg, 2.1 mmol) in absolutem Acetonitril (20 mL) getropft und über Nacht bei Raumtemperatur gerührt. Das Rohprodukt wurde mittels Flashchromatographie (38 g, 20 x 2.5 cm) mit Cyclohexan / Ethylacetat (9:1) gereinigt.
*Ausbeute:* 450 mg (70 %), farbloses Öl
*¹H-NMR (DMSO-d₆):* 0.51-0.57 (m, 6H); 0.92-0.97 (m, 9H); 1.90 (quint, 2H, J = 6.8 Hz); 2.35 (t, 2H, J = 6.8 Hz); 3.34 (t, 2H, J = 6.8 Hz).

### 4-Dimethylamino-4-phenyl-1-(5-triethylsilanyl-pent-4-inyl)cyclohexanol

Zu einer Lösung von Triethyl-(5-iodpent-1-inyl)silan (4.81 g, 15.6 mmol) in wasserfreiem Diethylether (150 mL) wurde bei -75°C unter Argon eine 1.7 *M* Lösung von *tert-* Butyllithium (18.4 mL, 31.2 mmol) in Pentan mittels Kanüle direkt in die Lösung eingeführt, wobei die Innentemperatur zwischen -70°C und -75 °C gehalten wurde. Nach 2 h Rühren bei -75 °C wurde eine Lösung von 4-(Dimethylamino)-4-phenylcyclohexanon (3.39 g, 15.6 mmol, Synthese vgl. WO2008009415 Ketonbaustein Ket-10). In wasserfreiem Diethylether (150 mL) langsam zugetropft, noch 30 min bei -75°C gerührt und die Reaktionslösung dann auf Raumtemperatur erwärmt. Anschließend wurde das Reaktionsgemisch mit gesättigter Ammoniumchloridlösung (100 mL) versetzt, die Phasen wurden getrennt, die wässrige Phase mit Diethylether (3 × 50 mL) extrahiert, die vereinigten organischen Phasen mit Natriumchloridlösung (50 mL) gewaschen, mit Natriumsulfat getrocknet und i. Vak. eingeengt. Das Rohprodukt wurde mittels Flashchromatographie (400 g, 20 × 7.5 cm) mit Ethylacetat / Methanol (9:1) gereinigt.
*Ausbeute:* 1.70 g (28 %), gelbes Öl, ein Diastereomer.

### Triphenyl-(5-trimethylsilylpent-4-inyl)phosphoniumiodid

Eine Lösung von Triethyl-(5-iodpent-1-inyl)silan (5.02 g, 16.3 mmol) und Triphenylphosphin (3.94 g, 15 mmol) in absolutem Ethanol (100 mL) wurde 4 h unter Rückfluss und über Nacht bei 50 °C gerührt sowie im Anschluss nochmals für 7 h unter Rückfluss erhitzt. Die Reaktionslösung wurde anschließend i. Vak. eingeengt, die gebildeten gelblichen Kristalle mit Diethylether (50 mL) versetzt und gerührt. Der Diethylether wurde wiederum i. Vak. eingeengt und die Kristalle mit Ethylacetat (50 mL) versetzt. Nach 24 h Stehen im Kühlschrank wurden die Kristalle abgesaugt, mit kaltem Ethylacetat (50 mL) gewaschen und im Exsikkator über Phosphorpentoxid getrocknet.
Ausbeute: 5.51 g (70 %), weißer Feststoff
¹H-NMR (DMSO-d₆): 0.51 (q, J = 7.8, 6H); 0.88 (t, J = 7.9, 9H); 1.71 (t, J = 7.4, 2H); 2.48-2.55 (m, 2H); 3.52-3.62 (m, 2H); 7.68 (m, 15H).

### b) Beispiele

### Beispiel Nr. 1 und Beispiel Nr. 2

### Stufe 1:

### 4-Dimethylamino-4-phenyl-1-[3-(2-triethylsitanyl-1H-indol-3-yl)propyl]cyclohexanol

Eine Mischung von 4-Dimethylamino-4-phenyl-1-(5-triethylsilanyl-pent-4-inyl)cyclohexanol (504 mg, 1.26 mmol), lodanilin (331 mg, 1.51 mmol), [1,3-Bis-(2,6-diisopropylphenyl)-imidazol-2-yliden]-(3-chlorpyridyl)palladium(II)-chlorid (PEPPSI, 171 mg, 0.25 mmol) und Natriumcarbonat (668 mg, 6.3 mmol) wurde 30 min evakuiert (Ölpumpe). Anschließend wurde mit Argon gespült und über einen Schlenk-Aufsatz absolutes N,N Dimethylformamid (5 mL, vorher 1 h mit Argon gespült) per Spritze zugegeben. Das Gemisch wurde 18 h bei 100°C gerührt, wobei es sich dunkelbraun färbte. Danach wurde das Reaktionsgemisch i. Vak. eingeengt, der Rückstand mehrfach in Toluol aufgenommen (3×10 mL) und jeweils wieder eingeengt, zwischen Wasser und Ethylacetat (je 20 mL) verteilt und die Phasen wurden getrennt. Die wässrige Phase wurde mit Ethylacetat (2 × 30 mL) extrahiert, die vereinigten organischen Phasen wurden mit 1 *M* Natriumthiosulfat-Lösung (30 mL) und gesättigter Natriumchlorid-Lösung (50 mL) gewaschen, mit Natriumsulfat getrocknet und i. Vak. eingeengt. Das Rohprodukt (750 mg) wurde mittels Flashchromatographie (38 g, 20 × 2.5 cm) mit Chloroform / Methanol (97:3) gereinigt. Die erhaltenen Mischfraktionen (489 mg) wurden nochmals mittels MPLC [LiChroprep Si60 (15-25 µm), 130 g, 46 x 2.6 cm] ebenfalls mit Chloroform / Methanol (97:3) gereinigt.
*Ausbeute:* 406 mg (66 %)
*Schmelzpunkt:* 79-83 °C
*¹H-NMR (DMSO-d₆):* 0.87-0.92 (m, 6H); 0.93-0.98 (m, 9H); 1.25-1.32 (m, 2H); 1.45-1.52 (m, 2H); 1.57-1.70 (m, 4H); 1.76-1.84 (m, 2H); 1.89 (s, 6H); 2.12-2.23 (m, 2H); 2.73 (t, 2H, J = 7.7 Hz); 3.80 (s, 1 H); 6.94 (t, 1 H; J = 7.0 Hz); 7.05 (t, 1 H; J = 7.0 Hz); 7.19-7.24 (m, 1 H); 7.30-7.34 (m, 4H); 7.36 (d, 1H; J = 8.2 Hz); 7.50 (d, 1 H, J = 8.0 Hz); 10.35 (s, 1H).
*¹³C-NMR (DMSO-d₆):* 3.3 (3C); 7.4 (3C); 25.8; 26.4; 28.4 (2C); 32.5 (2C); 37.7 (2C); 42.9; 58.8; 68.7; 109.3; 111.2; 117.8; 118.4; 121.1; 125.3; 126.0; 126.7 (2C); 127.2 (2C); 128.2; 129.7; 138.9.

### Stufe 2:

### 4-Dimethylamino-1-[3-(1H-indol-3-yl)propyl]-4-phenylcyclohexanol

Eine Lösung von 4-Dimethylamino-4-phenyl-1-[3-(2-triethylsilanyl-1H indol-3-yl)propyl]cyclohexanol (852 mg, 1.8 mmol) in absolutem Tetrahydrofuran (50 mL) wurde mit Tetra-n-butytammoniumfluorid-Trihydrat (1.66 g, 5.3 mmol) versetzt, 6 h unter Rückfluss, anschließend über das Wochenende bei 50 °C gerührt und danach i. Vak. eingeengt. Der Rückstand wurde mittels Flashchromatographie (38 g, 20 × 2.5 cm) mit Ethylacetat / Methanol (9:1) gereinigt. Es wurden 80 mg der Titelverbindung erhalten, die über-Nacht mit Cyclohexan (20 mL) gerührt wurden. Weiterhin wurden 406 mg der Zielverbindung erhalten, welche noch größere Mengen an Verunreingung enthielt. Diese Mischung wurde nochmals mittels Flashchromatographie (38 g, 20 × 2.5 cm) gereinigt, wobei zunächst Cyclohexan / Ethylacetat (4:1) als Laufmittel eingesetzt wurde. Sämtliche Fraktionen, die die Zielverbindung enthielten, wurden wie oben mit Cyclohexan behandelt.Ausbeute: 241 mg (36 %), gelblicher Feststoff
*¹H-NMR (CDCl₃):* 1.36-1.48 (m, 2H); 1.63-1.71 (m, 2H); 1.76-1.90 (m, 5H); 1.95-2.01 (m, 2H); 2.03 (s, 6H); 2.13-2.26 (m, 2H); 2.80 (t, 2H, J = 7.23 Hz); 7.01 (s, 1 H); 7.31 (t, 1 H, J = 7.3 Hz); 7.19 (t, 1 H, J = 7.4 Hz); 7.26 (s, 2H); 7.31-7.39 (m, 4H); 7.62 (d, 1 H; J=7.8 Hz); 7.95 (br s, 1 H).

### Stufe 3:

### N, N-Dimethyl-4'-phenyl-2,3,4,9-tetrahydrospiro(carbazol-1,1'-cyclohexan)-4'-amin (Beispiel Nr. 1, polares Diastereomer und Beispiel Nr. 2, unpolares Diastereomer)

Zu einer Aufschlämmung von 4-Dimethylamino-1-[3-(1*H*-indol-3-yl)propyl]-4-phenylcyclohexanol (320 mg, 0.85 mmol) in absolutem Dichlormethan (20 mL) wurde Trifluormethansulfonsäuretrimethylsilylester (755 mg, 617 µL, 3.4 mmol) gegeben, wobei sich eine klare braune Lösung bildete, welche 14 d bei Raumtemperatur gerührt wurde. Die Reaktionslösung wurde anschließend mit Dichlormethan (10 mL) verdünnt, mit 1 *N* Natriumcarbonat-Lösung (2×10 mL), Wasser und gesättigter Natriumchloridlösung (je 10 mL) gewaschen, mit Natriumsulfat getrocknet und i. Vak. eingeengt. Der Rückstand (270 mg) wurde mit Methanol (30 mL) versetzt und über Nacht bei Raumtemperatur gerührt, wobei sich ein heller Feststoff bildete, welcher abfiltriert und mit wenig Methanol (2 mL) gewaschen wurde.
*Ausbeute:* 102 mg (33 %), heller Feststoff
Das erhaltene Diastereomerengemisch wurde mittels präparativer HPLC getrennt [Säule: Gemini 5µ C18, 250 x 4.6 mm; Eluent: CH₃CN: H₂O: DEA= 750: 250: 1; 1 ml/min].
Beispiel Nr. 1 (polares Diastereomer): Retentionszeit: 10.65 min, MH⁺: 359,3.
*Ausbeute:* 37 mg
Beispiel Nr. 2 (unpolares Diastereomer): Retentionszeit: 18.05 min, MH⁺: 359,3.
*Ausbeute:* 56 mg

### Beispiel Nr. 3 und Beispiel Nr. 4

### Stufe 1:

### 4-Dimethylamino-1-[3-(5-fluor-2-triethylsilanyl-1H-indol-3-yl)propyl]-4-phenylcyclohexanol

Eine Mischung von 4-Dimethylamino-4-phenyl-1-(5-triethylsilanyl-pent-4-inyl)cyclohexanol (525 mg, 1.31 mmol), 4-Fluor-2-iodanilin (374 mg, 1.58 mmol), [1,3-Bis-(2,6-diisopropylphenyl)imidazol-2-yliden]-(3-chlorpyridyl)palladium(II)-chlorid (PEPPSI, 178 mg, 0.26 mmol) und Natriumcarbonat (694 mg, 6.6 mmol) wurde 30 min evakuiert (Ölpumpe). Anschließend wurde mit Argon gespült und über einen Schlenk-Aufsatz absolutes N,N-Dimethylformamid (5 mL, vorher 1 h mit Argon gespült) per Spritze zugegeben. Das Gemisch wurde 18 h bei 100°C gerührt, wobei es sich dunkelbraun färbte. Danach wurde das Reaktionsgemisch i. Vak. eingeengt, der Rückstand mehrfach in Toluol aufgenommen (3×10 mL) und jeweils wieder eingeengt, zwischen Wasser und Ethylacetat (je 20 mL) verteilt und die Phasen wurden getrennt. Die wässrige Phase wurde mit Ethylacetat (2 x 30 mL) extrahiert, die vereinigten organischen Phasen wurden mit 1 M Natriumthiosulfat-Lösung (30 mL) und gesättigter Natriumchlorid-Lösung (50 mL) gewaschen, mit Natriumsulfat getrocknet und i. Vak. eingeengt. Das Rohprodukt (1.10 g) wurde mittels Flashchromatographie (50 g, 20 × 3.0 cm) mit Chloroform / Methanol (97:3) gereinigt.
*Ausbeute:* 379 mg (57 %)
*Schmelzpunkt* 48-50 °C
*¹H-NMR (DMSO-d₆):* 0.83-0.99 (m, 15H); 1.23-1.34 (m, 2H); 1.43-1.52 (m, 2H); 1.55-1.64 (m, 6H); 1.72-1.85 (m, 2H); 1.89 (s, 6H); 2.69 (t, 2H, J = 7.5 Hz); 3.84 (s, 1 H); 6.89 (dt, 1H, J = 2.5, 9.3 Hz); 7.22 (dd, 2H, J = 2.5, 10.1 Hz); 7.29-7.36 (m, 5H); 10.48 (s, 1 H).
*¹³C-NMR (DMSO-d₆):* 3.2 (3C); 7.3 (3C); 25.6; 26.3; 28.4 (2C); 32.5 (2C); 37.7 (2C); 42.7; 58.8; 68.7; 102.7 (d, J = 22 Hz); 109.2 (d, J = 27 Hz), 112.0; 125.3; 126.0; 126.7 (2C); 127.2 (2C); 128.2 (d, J = 9. Hz); 132.5; 135.6; 139.1; 156.4 (d, J = 230 Hz).

### Stufe 2:

### 4-Dimethylamino-1-[3-(5-fluor-1H-indol-3-yl)propyl]-4-phenylcyclohexanol

Eine Lösung von 4-Dimethylamino-1-[3-(5-fluor-2-triethylsilanyl-1H-indol-3-yl)propyl]-4-phenylcyclohexanol (350 mg, 0.69 mmol) in absolutem Tetrahydrofuran (25 mL) wurde mit Tetra-n-butylammoniumfluorid-Trihydrat (868 mg, 2.8 mmol) versetzt und 6 h unter Rückfluss und anschließend über Nacht bei Raumtemperatur gerührt. Danach wurde das Reaktionsgemisch i. Vak. eingeengt und der Rückstand mittels Flashchromatographie (18 g, 20 x 2.0 cm) mit Ethylacetat / Methanol (9:1) gereinigt.
*Ausbeute:* 217 mg (80 %), hellbeigefarbener Feststoff
*Schmelzpunkt:* 199-200 °C
*¹H-NMR (DMSO-d₆):* 1.31 (d, 2H, J = 13.2 Hz); 1.44-1.48 (m, 2H); 1.61 (t, 2H, J = 10.9 Hz); 1.67-1.74 (m, 2H); 1.80 (d, 2H; J = 12.7 Hz); 1.90 (s, 6H); 2.18 (d, 2H, J=13.3 Hz); 2.64 (t, 2H, J = 7.4 Hz); 3.81 (s, 1 H); 6.89 (dt, 1H; J = 2.5, 9.2 Hz); 7.18 (d, 1 H, J=2.2); 7.23 (dd, 2H, J = 2.5, 10.1 Hz); 7.30 (d, 1 H, J = 4.6 Hz); 7.32-7.36 (m, 4H); 10.82 (s, 1 H).
*¹³C-NMR (DMSO-d₆):* 23.6 25.2; 28.5 (2C); 32.6 (2C); 37.7 (2C); 42.4; 58.8; 68.8; 102.9 (d, J = 23 Hz); 108.7 (d, J = 26 Hz), 112.1 (d, J = 9 Hz); 115.2; 124.2; 126.0; 126.7 (2C); 127.2 (2C); 127.4 (d, J = 9 Hz); 132.9; 139.1; 156.5 (d, J = 229 Hz).

### Stufe 3:

### 6-Fluor-N,N-dimethyl-4'-phenyl-2,3,4,9-tetrahydrospiro[carbazol-1,1'-cyclohexan]-4'-amin (Beispiel Nr. 3, polares Diastereomer und Beispiel Nr. 4, unpolares Diastereomer)

Zu einer Aufschlämmung von 4-Dimethylamino-1-[3-(5-fluor-1*H*-indol-3-yl)propyl]-4-phenylcyclohexanol (365 mg, 0.92 mmol) in absolutem Dichlormethan (50 mL) wurde Trifluormethansulfonsäuretrimethylsilylester (822 mg, 671 µL, 3.70 mmol) gegeben, und 10 d bei Raumtemperatur gerührt. Die Reaktionslösung wurde anschließend mit Dichlormethan (10 mL) verdünnt, mit 1 N Natriumcarbonat-Lösung (2 × 10 mL), Wasser und gesättigter Natriumchloridlösung (je 10 mL) gewaschen, die organische Phase mit Natriumsulfat getrocknet und i. Vak. eingeengt. Der Rückstand (249 mg) wurde mit Methanol (50 mL) versetzt und über das Wochenende bei Raumtemperatur gerührt, wobei sich ein heller Feststoff bildete, welcher abfiltriert und mit wenig Methanol (2 mL) gewaschen wurde.
*Ausbeute:* 74 mg (21 %), heller Feststoff
Das erhaltene Diastereomerengemisch wurde mittels präparativer HPLC getrennt [Säule: Gemini 5µ C18, 250 x 4.6mm; Eluent: CH₃CN: H₂O = 750: 250; 1 ml/min].
Beispiel Nr. 3 (polares Diastereomer): Retentionszeit: 10.91 min, MH⁺: 377,0.
*Ausbeute:* 8 mg
Beispiel Nr. 4 (unpolares Diastereomer): Retentionszeit: 17.51 min, MH⁺: 377,3.
*Ausbeute:* 50 mg

### Beispiel Nr. 5

### Stufe 1:

### 4-Dimethylamino-4-phenyl-1-[3-(2-triethylsilanyl-1H-pyrrolo[3,2-c]pyridin-3-yl)propyl]-cyclohexanol

Eine Suspension von 4-Dimethylamino-4-phenyl-1-(5-triethylsilanyl-pent-4-inyl)cyclohexanol (741 mg, 1.85 mmol), 4-Amino-3-iodpyridin (488 mg, 2.22 mmol), [1,3-Bis-(2,6-diisopropylphenyl)imidazo)-2-yliden]-(3-chlorpyridyl)palladium(II)-chlorid (PEPPSI, 251 mg, 0.37 mmol) und Natriumcarbonat (980 mg, 9.25 mmol) in sauerstofffreiem *N,N-*Dimethylformamid (10 mL) wurde 20 h bei 100°C gerührt. Anschließend wurde das Reaktionsgemisch i. Vak. eingeengt, der Rückstand wiederholt in Toluol (10 mL) aufgenommen und jeweils wieder i. Vak. eingeengt, dieser Rückstand in Ethylacetat (30 mL) und Wasser (30 mL) aufgenommen und die organische Phase mit Wasser (2×15 mL) und Natriumthiosulfat-Lösung (2 × 25 mL) gewaschen, mit Natriumsulfat getrocknet und i. Vak. eingeengt. Das Rohprodukt wurde mittels Flashchromatographie (38 g, 20 x 2.5 cm) mit Ethylacetat / Methanol (4:1) gereinigt.
*Ausbeute:* 450 mg (44 %), gelber Schaum
*Schmelzpunkt:* 89-102 °C
*¹H-NMR (DMSO-d₆):* 0.86-0.99 (m, 15H); 1.23-1.34 (m, 2H); 1.45-1.87 (m, 7H); 1.92 (s, 6H); 2.08-2.25 (m, 2H); 2.79 (t, 2H, J = 7.4 Hz); 3.87 (s, 1 H); 7.18-7.28 (m, 1H); 7.34 (d, 6H, J = 4.0 Hz); 8.11 (d, 1 H, J = 5.8 Hz); 8.84 (s, 1 H); 10.86 (s, 1 H).

### Stufe 2:

### 4-Dimethylamino-4-phenyl-1-[3-(1H-pyrrolo[3,2-c]pyridin-3-yl)propyl]cyclohexanol

Eine Lösung von 4-Dimethylamino-4-phenyl-1-[3-(2-triethylsilanyl-1*H*-pyrrolo[3,2-c]pyridin-3-yl)propyl]cyclohexanol (410 mg, 0.83 mmol) in wasserfreiem Tetrahydrofuran (25 mL) wurde mit Tetra-n-butylammoniumfluorid-Trihydrat (1.05 g, 3.32 mmol) versetzt und 6 h unter Rückfluss gekocht. Das Reaktionsgemisch wurde anschließend i. Vak. eingeengt und das Rohprodukt mittels Flashchromatographie (38 g, 20 × 2.5 cm) mit Ethylacetat / Methanol (2:1) gereinigt. Das Produkt wurde in Dichlormethan (40 mL) aufgenommen, die Lösung mit Natriumsulfat getrocknet und i. Vak. eingeengt.
*Ausbeute:* 198 mg (63 %), gelber Schaum
*¹H-NMR (DMSO-d₆):* 1.21-1.36 (m, 2H); 1.42-1.50 (m, 2H); 1.54-1.68 (m, 2H); 1.71-1.80 (m, 2H); 1.94 (s, 6H); 2.10-2.24 (m, 2H); 2.74 (t, 2H, J = 7.3 Hz); 3.87 (s, 1 H); 7.20-7.30 (m, 2H); 7.32-7.40 (m, 6H); 8.14 (d, 1 H, J = 5.8 Hz); 8.85 (s, 1 H); 11.25 (s, 1H).

### Stufe 3:

### N,N-Dimethyl-4-phenyl-5', 7',8', 9'-tetrahydrospiro[cyclohexan-1, 6'-pyrido[4,3-b]indol]-4-amin (Beispiel Nr. 5, ein Diastereomer)

Zu einer Lösung von 4-Dimethylamino-4-phenyl-1-[3-(1H-pyrrolo[3,2-c]pyridin-3-yl)propyl]-cyclohexanol (172 mg, 0.39 mmol) in wasserfreiem Dichlormethan (30 mL) wurde Trifluormethansulfonsäuretrimethylsilylester (409 mg, 334 µL, 1.84 mmol) gegeben und 10 d bei Raumtemperatur gerührt. Anschließend wurde das Reaktionsgemisch mit Dichlormethan (10 mL) verdünnt, danach zunächst mit 1 *N* Natriumcarbonat-Lösung (2×10 mL), dann mit Wasser und gesättigter Natriumchloridlösung (je 10 mL) gewaschen, mit Natriumsulfat getrocknet und i. Vak. eingeengt. Der Rückstand (131 mg) wurde in Methanol (15 mL) aufgenommen, über Nacht bei Raumtemperatur gerührt und danach i. Vak. eingeengt.
*Ausbeute:* 125 mg, braunes Öl
Das erhaltene Rohprodukt wurde mittels präparativer HPLC getrennt [Säule: Gemini 5µ C18 110A, 250 x 21.2 mm; Eluent: CH₃CN: H₂O: DEA = 40: 60: 0.1; 15 ml/min].
Beispiel Nr. 5 (ein Diastereomer): Retentionszeit: 9.96 min, MH⁺: 360,2.
*Ausbeute:* 17 mg.

### Beispiel Nr. 6

### Stufe 1:

### 4-Azetidin-1-yl-4-phenyl-1-(5-triethylsilylpent-4-inyl) cyclohexanol

In einer ausgeheizten Apparatur wurde unter Argon in eine Lösung von Triethyl-(5-iodpent-1-inyl)silan (5.26 g, 17.1 mmol) in absolutem Diethylether (150 mL) bei einer Innentemperatur von -70°C bis -75 °C langsam eine 1.7 *M* Lösung von tert-Butyllithium in Pentan (20.1 mL, 34.1 mmol) getropft. Nach 120 min wurde bei dieser Temperatur eine Lösung von 4-Azetidin-1-yl-4-phenylcyclohexan (3.92 g, 17.1 mmol) in absolutem Diethylether (100 mL) langsam zugetropft und 30 min nachgerührt. Die Reaktionslösung wurde auf Raumtemperatur erwärmt und mit gesättigter Ammoniumchlorid-Lösung (100 mL) versetzt. Die Phasen wurden getrennt und die wässrige Phase mit Diethylether (3 × 50 mL) extrahiert. Die vereinigten organischen Phasen wurden mit gesättigter Natriumchlorid-Lösung (50 mL) gewaschen, mit Natriumsulfat getrocknet und i. Vak. eingeengt. Der Rückstand (6.86 g) wurde mittels Flashchromatographie (400 g, 20 × 7.5 cm) mit Cyclohexan / Ethylacetat (2:1) gereinigt.
Ausbeute: 3.50 g (50 %), farbloses Öl
¹H-NMR (DMSO-d₆): 0.55 (q, 6H; J = 7.8 Hz); 0.96 (t, 9H, J = 7.9 Hz); 1.24-1.92 (m, 14H); 2.19-2.26 (m, 2H); 2.83 (t, 4H, J = 6.8 Hz); 3.85 (s, 1 H); 7.24-7.42 (m, 5H).
¹³C-NMR (DMSO-d₆): 4.0; 7.3; 19.7; 22.3; 29.9; 32.7; 41.2; 46.0; 57.4; 68.6; 80.9; 109.5; 126.2; 126.8; 127.5; 139.7.
LC-MS: m/z: [M+1]⁺ = 412.4, Rₜ 3.3 min.

### Stufe 2:

### 4-(Azetidin-1-yl)-4-phenyl-1-[3-(2-(triethylsilyl)-1H-indol-3-yl)propyl]cyclohexanol

Eine Mischung von 4-Azetidin-1-yl-4-phenyl-1-(5-triethylsilylpent-4-inyl)cyclohexanol (1.20 g, 2.91 mmol), 2-lodanilin (766 mg, 3.50 mmol), [1,3-Bis(2,6-diisopropylphenyl)imidazol-2-yliden]-3-chlol-pyridyl-palladium(II)-chlorid (PEPPSI, 198 mg, 0.29 mmol) und Natriumcarbonat (1.54 g, 14.6 mmol) wurde 30 min evakuiert (Ölpumpe). Anschließend wurde mit Argon gespült und über einen Schlenk-Aufsatz absolutes *N,N* Dimethylformamid (5 mL, vorher 1 h mit Argon gespült) per Spritze zugegeben. Der Ansatz wurde 18 h bei 100°C gerührt, wobei sich die Lösung dunkelbraun färbte. Anschließend wurde das Reaktionsgemisch i. Vak. eingeengt, der Rückstand mehrfach mit Toluol versetzt (3 × 30 mL) und jeweils wieder eingeengt. Der Rückstand wurde zwischen Wasser und Ethylacetat (je 100 mL) verteilt, die Phasen wurden getrennt und die wässrige Phase mit Ethylacetat (2 x 50 mL) extrahiert. Die vereinigten organischen Phasen wurden mit 1 *M* Natriumthiosutfat-Lösung und gesättigter Natriumchlorid-Lösung (je 50 mL) gewaschen, mit Natriumsulfat getrocknet und i. Vak. eingeengt. Der Rückstand (1.59 g) wurde mittels Flashchromatographie (100 g, 20 x 4.0 cm) mit Dichlormethan / Methanol (95:5) gereinigt.
Ausbeute: 1.46 g (54 %), beigefarbener fester Schaum
Schmelzpunkt: 50-52 °C
¹H-NMR (DMSO-d₆): 0-88-0.92 (m, 6H); 0.94-0.99 (m, 9H); 1.25-1.30 (m, 2H); 1.47-1.52 (m, 2H); 1.57-1.71 (m, 10H); 1.80-1.90 (m, 2H); 2.71-2.76 (m, 2H); 2.78-2.85 (m, 2H); 3.76 (s, 1H); 6.95 (ddd, 1 H, J = 1.0, 7.0, 7.9 Hz); 7.05 (ddd, 1 H, J = 1.1; 6.9, 8.1 Hz); 7.24-7.28 (m, 2H); 7.29-7.34 (m, 2H); 7.36-7.39 (m, 2H); 7.51 (d, 1H, J = 7.7 Hz); 10.36 (s, 1 H). ¹³C-NMR (DMSO-d₆): 3.2; 7.4; 15.5; 25.8; 26.4; 26.8; 32.5; 46.0; 57.3; 68.7; 111.2; 117.8; 118.4; 121.1; 125.3; 126.2; 126.8; 127.4; 128.2; 129.8; 138.9.
LC-MS: m/z: [M+1]⁺ = 504.4, Rₜ 3.2 min.

### Stufe 3:

### 4-Azetidin-1-yl-1-[3-(1H-indol-3-yl)propyl]-4-phenylcyclohexanol

Eine Lösung von 4-(Azetidin-1-yl)-4-phenyl-1-[3-(2-(triethylsilyl)-1*H*-indol-3-yl)propyl]cyclohexanol (740 mg, 1.5 mmol) in absolutem Tetrahydrofuran (50 mL) wurde mit Tetra-n-butylammoniumfluorid-Trihydrat (1.86 g, 5.9 mmol) versetzt und zunächst 6 h unter Rückfluss, dann über Nacht bei Raumtemperatur gerührt. Das Reaktionsgemisch wurde i. Vak. eingeengt und der Rückstand mittels Flashchromatographie (100 g, 20 × 4.0 cm) mit Ethylacetat / Methanol (9:1) gereinigt.
Ausbeute: 389 mg (66 %), hellgelber Feststoff
Schmelzpunkt: 176-180 °C
¹H-NMR (DMSO-d₆): 1.26-1.34 (m, 2H); 1.46-1.50 (m, 2H); 1.57-1.77 (m, 8H); 1.81-1.89 (m, 2H); 2.68 (t, 2H, J = 7.4 Hz); 2.80-2.88 (m, 4H); 3.76 (s, 1 H); 6.97 (t, 1H; J= 7.4 Hz); 7.05 (t, 1 H, J = 7.2 Hz); 7.10 (d, 1H, J = 2.1 Hz); 7.26 (t, 1H, J = 7.0 Hz); 7.33 (d, 3H, J = 8.0 Hz); 7.39 (t, 2H, J = 7.5 Hz); 7.51 (d, 1 H, J = 7.8 Hz); 10.71 (s, 1 H). ¹³C-NMR (DMSO-d₆): 15.6; 23.7; 25.4; 26.9; 32.7; 42.5; 46.0; 57.3; 68.7; 111.2; 114.9; 117.9; 118.3; 120.6; 122.0; 126.2; 126.8; 127.2; 127.5; 136.2; 139.9.
LC-MS: m/z: [M+1]⁺ = 289.3, Rₜ 2.0 min.

### Stufe 4:

### 4'-(Acetidin-1-yl)-4'-phenyl-2,3,4,9-tetrahydrospiro(carbazol-1,1'cyclohexan] 2-Hydroxypropan-1,2,3-tricarboxylat (Beispiel Nr. 6, ein Diastereomer)

Zu einer Lösung von 4-Azetidin-1-yl-1-[3-(1*H*-indol-3-yl)propyl]-4-phenylcyclohexanol (337 mg, 0.87 mmol) in absolutem Dichlormethan (50 mL) wurde Trifluormethansulfonsäuretrimethylsilylester (771 mg, 629 µL, 3.47 mmol) gegeben und 48 h bei Raumtemperatur gerührt. Anschließend wurde die Reaktionslösung nacheinander mit 1 *N* Natriumcarbonatlösung (2 × 30 mL), Wasser und gesättigter Natriumchlorid-Lösung (je 30 mL) gewaschen mit Natriumsulfat getrocknet und i. Vak. eingeengt. Das Rohprodukt (304 mg) wurde mit Methanol (50 mL) versetzt und über Nacht bei Raumtemperatur gerührt, wobei sich ein heller Feststoff bildete. Das Lösungsmittel wurde abdekantiert, das Filtrat i. Vak. eingeengt und der Rückstand wiederholt mit Methanol (3 × 20 mL) versetzt und jeweils dekantiert. Die Methanol-unlöslichen Chargen wurden i. Vak. getrocknet. Zur Reinigung wurde das Produkt mit Citronensäure als Citrat gefällt.
Beispiel Nr. 6:
Ausbeute: 100 mg, beigefarbener Schaum
LC-MS : m/z: [M+H]⁺ = 371.3, Rₜ = 4.1 min.

### Beisniel Nr. 7 und Beispiel Nr. 8 -

### Stufe 1:

### 1-Butyl-N, N-dimethyl-4-(5-triethylsilyl)pent-4-inyliden)cyclohexanamin

Zu einer Aufschlämmung von Triphenyl-(5-trimethylsilylpent-4-inyl)phosphoniumiodid (4.40 g, 7.7 mmol) in absolutem Tetrahydrofuran (200 mL) in einer ausgeheizten Apparatur wurde bei -78 °C langsam und unter Argon eine 2.5 M Lösung von n-Butyllithium in Hexan (3.1 mL, 7.7 mmol) getropft. Nach 30 min Rühren bei -40 °C wurde wieder auf -78 °C gekühlt, bei dieser Temperatur eine Lösung von 4-Butyl-4-(dimethylamino)cyclohexanon (2.20 g, 10.1 mmol) in absolutem Tetrahydrofuran (20 mL) langsam zugetropft und 30 min nachgerührt. Die Reaktionslösung wurde auf Raumtemperatur erwärmt und mit gesättigter Ammoniumchlorid-Lösung (100 mL) versetzt. Anschließend wurde der pH (7) mit 4 N Natriumhydroxid-Lösung auf 9-10 eingestellt. Die Phasen wurden getrennt, die wässrige Phase mit Diethylether (3 x 50 mL) extrahiert und die vereinigten organischen Phasen wurden mit gesättigter Natriumchlorid-Lösung (50 mL) gewaschen, mit Natriumsulfat getrocknet und i. Vak. eingeengt. Der Rückstand (5.40 g) wurde mittels Flashchromatographie (200 g, 20 x 5.7 cm) mit Cyclohexan / Ethylacetat (1:1) gereinigt. Ausbeute: 1.83 g (66 %), hellgelbes Öl
¹H NMR (DMSO-d₆): 0.52 (q, J = 7.8 Hz, 6H); 0.87 (t, J = 7.0 Hz, 3H); 0.93 (t, J = 7.8 Hz, 9H); 1.04-1.40 (m, 12H); 1.60-1.70 (m, 2H); 2.07-2.14 (m, 2H); 2.16 (s, 6H); 2.19-2.29 (m, 2H); 5.07 (t, J = 6.9 Hz, 1 H).
¹³C-NMR (DMSO-d₆): 4.0; 7.3; 14.0; 22.9; 23.0; 23.2; 25.7; 25.9; 30.2; 31.2; 32.3; 32.8; 37.0; 56.1; 81.5; 109.5; 119.2; 139.7.
LC-MS: m/z: [M+1]⁺ = 362.4, Rₜ 3.8 min.

### Stufe 2:

### 1-Butyl-N,N-dimethyl-4-[3-(2-(triethylsilyl)-1H-indol-3-yl)propyliden]cyclohexanamin

Eine Mischung von 1-Butyl-N,N-dimethyl-4-(5-triethylsilyl)pent-4-inyliden)cyclohexanamin (1.80 g, 5.0 mmol), 2-lodanilin (1.31 g, 6.0 mmol), [1,3-Bis-(2,6-diisopropylphenyl)imidazol-2-yliden]-(3-chlorpyridyl)palladium(II)-chlorid (PEPPSI, 340 mg, 0.5 mmol) und Natriumcarbonat (2.65 g, 25 mmol) wurde 30 min evakuiert (Ölpumpe). Anschließend wurde mit Argon gespült und über einen Schlenk-Aufsatz absolutes N,N-Dimethylformamid (5 mL, vorher 1 h mit Argon gespült) per Spritze zugegeben. Das Reaktionsgemisch wurde 18 h bei 100 °C gerührt, wobei es sich dunkelbraun färbte. Die Reaktionslösung wurde anschließend i. Vak. eingeengt, der Rückstand mehrfach mit Toluol versetzt (3 × 50 mL) und jeweils wieder eingeengt. Der Rückstand wurde zwischen Wasser und Ethylacetat (je 100 mL) verteilt, die Phasen wurden getrennt und die wässrige Phase mit Ethylacetat (2 x 50 mL) extrahiert. Die vereinigten organischen Phasen wurden mit 1 M Natriumthiosulfat-Lösung und gesättigter Natriumchlorid-Lösung (je 50 mL) gewaschen, mit Natriumsulfat getrocknet und i. Vak. eingeengt. Das Rohprodukt (2.33 g) wurde mittels Flashchromatographie (100 g, 20 x 4.0 cm) mit Dichlormethan / Methanol (95:5) gereinigt.
Ausbeute: 1.27 g (56 %), zähes braunes Öl
¹H-NMR (DMSO-d₆): 0.85-0.95 (m, 18H); 1.03-1.17 (m, 4H); 1.19-1.35 (m, 4H); 1.44-1.65 (m, 2H); 1.77-1.95 (m, 2H); 1.98-2.09 (m, 2H); 2.16 (m, 6H); 2.22-2.30 (m, 2H); 2.73-2.78 (m, 2H); 5.18 (br s, 1 H); 6.95 (ddd, J = 7.9, 7.0, 1.0 Hz, 1 H); 7.04 (ddd, J = 8.1, 6.9; 1.1 Hz, 1 H); 7.35 (d, J = 8.1 Hz, 1 H); 7.49 (d, J = 7.8 Hz, 1 H); 10.38 (s, 1 H).
¹³C-NMR (DMSO-d₆): 3.3; 7.3; 13,9; 22.9; 23.1; 25.7; 26.4; 29.5; 30.1; 31.2; 32.1; 37.0; 54.8; 111.2; 117.8; 118.4, 121.1; 124.3; 128.1; 130.0; 139.0.
LC-MS: m/z: [M+1]⁺ = 453.4, Rₜ 4.0 min.

### Stufe 3:

### 4-[3-(1H-Indol-3-yl)propyliden]-1-butyl-N,N-dimethylcyclohexanamin

Eine Lösung von 1-Butyl-N,N-dimethyl-4-[3-(2-(triethylsilyl)-1 H-indol-3-yl)propyliden]cyclohexanamin (1.20 g, 2.7 mmol) in absolutem Tetrahydrofuran (100 mL) wurde mit Tetra-n-butylammoniumfluorid-Trihydrat (3.34 g, 10.6 mmol) versetzt, 7 h unter Rückfluss und dann 18 h bei Raumtemperatur gerührt. Das Reaktionsgemisch wurde i. Vak. eingeengt und der Rückstand mittels Flashchromatographie (100 g, 20 × 4.0 cm) mit Dichlormethan / Methanol (9:1) gereinigt. Es wurden 517 mg der Zielverbindung erhalten, die aber als Salz (vermutlich Hydrochlorid) vorlagen. Dieses wurde in gesättigter Kaliumcarbonat-Lösung (50 mL) aufgenommen und die Lösung mit Dichlormethan (3 × 30 mL) extrahiert. Die vereinigten organischen Phasen wurden mit Natriumsulfat getrocknet und i. Vak. eingeengt, wodurch 457 mg reine Zielverbindung erhalten wurden.
Außerdem wurden 379 mg Zielverbindung erhalten, die noch mit einem Tetrabutylammoniumsalz verunreinigt waren. Erneute Reinigung mittels Flashchromatographie (18 g, 20 × 2.0 cm) mit Dichlormethan / Methanol (9: 1) ergab jedoch lediglich weitere 32 mg reine Zielverbindung.
Ausbeute: 489 mg (54 %), zähes braunes Öl
¹H-NMR (DMSO-d₆): 0.87 (t, J = 7.2 Hz, 3H); 1.00-1.15 (m, 2H); 1.18-1.31 (m, 6H); 1.48-1.64 (m, 2H); 1.82-1.89 (m, 1H); 1.99-2.10 (m, 2H); 2.13 (s, 6H); 2.18-2.27 (m, 1 H); 2.29-2.37 (m, 2H); 2.69 (t, J = 7.4 Hz, 2H); 5.14 (t, J = 6.8 Hz, 1 H); 6.95 (t, J = 7.4 Hz, 1 H); 7.04 (t, J=7.5Hz, 1 H); 7.08 (s, 1 H); 7.31 (d, J = 7.8 Hz, 1 H); 7.49 (d, J = 7.7 Hz, 1 H); 10.70 (s, 1 H). ¹³C-NMR (DMSO-d₆): 14.0; 22.9; 23.2; 25.3; 25.8; 27.7; 30.2; 31.2; 32.0; 32.8; 37.0; 56.1; 111.2; 114.3; 117.9; 118.2; 120.6; 120.8; 122.1; 127.2; 136.2; 138.5.
LC-MS: m/z: [M+1]⁺ = 339.3, Rₜ 3.1 min.

### Stufe 4:

### N,N-Dimethyl-N-(4-butyl-2',3',4',9'-tetrahydro-1H-spiro[cyclohexan-1,1'-carbazol]-4-yl)amin (Beispiel Nr. 7, polares Diastereomer und Beispiel Nr. 8, unpolares Diastereomer)

Zu einer Lösung von 4-[3-(1H-Indol-3-yl)propyliden]-1-butyl-N,N-dimethylcyclohexanamin (289 mg, 0.85 mmol) in absolutem Toluol (10 mL) wurde p-Toluolsulfonsäure (323 mg, 1.7 mmol) gegeben und 18 h bei 100 °C gerührt. Die Reaktionslösung wurde anschließend mit Dichlormethan (30 mL) verdünnt und nacheinander mit 1 N Natriumcarbonatlösung (2 × 30 mL), Wasser und gesättigter Natriumchlorid-Lösung (je 30 mL) gewaschen, mit Natriumsulfat getrocknet und i. Vak. eingeengt. Das Rohprodukt (272 mg) wurde mit Methanol (100 mL) versetzt und über Nacht bei Raumtemperatur gerührt, wobei sich sehr wenig Niederschlag bildete (heller Feststoff). Das ¹H-NMR-Spektrum und die LC-MS-Daten zeigten, dass es sich um zwei Isomere handelt, wobei aus dem ¹H-NMR-Spektrum hervorgeht, dass diese im Verhältnis von ca. 1:1 vorliegen und olefinische Nebenprodukte höchstens in sehr geringer Menge vorhanden sind. Die Reaktionslösung wurde i. Vak. Eingeengt (259 mg, 90%). Das Diastereomerengemisch wurde durch präparative HPLC getrennt.
Beispiel Nr. 7: polares Diastereomer (61 mg, 21%)
LC-MS: m/z: [M+1]⁺ = 339,3, Rₜ 4,4 min.
Beispiel Nr. 8: unpolares Diastereomer (86 mg, 30%)
LC-MS: m/z: [M+1]⁺ = 339,3, Rₜ 4,7 min.

### Beispiel Nr. 9 und Beispiel Nr. 10

### Stufe 1:

### 4-Benzyl-4-dimethylamino-1-(5-(triethylsilyl)pent-4-inyl)cyclohexanol

Zu einer Lösung von Triethyl-(5-iodpent-1-inyl)silan (6.10 g, 19.8 mmol) in wasserfreiem Diethylether (150 mL) in einer ausgeheizten Apparatur wurde bei -75 °C unter Argon eine 1.7 M Lösung von tert-Butyllithium (23.3 mL, 39.6 mmol) in Pentan mittels Kanüle direkt in die Lösung eingespritzt, so dass die Innentemperatur zwischen -70 °C und -75 °C gehalten wurde. Nach 120 min Rühren wurde eine Lösung von 4-Benzyl-4-dimethylaminocyclohexanon (4.58 g, 19.8 mmol) in wasserfreiem Diethylether (150 mL) langsam zugetropft, 30 min nachgerührt und die Reaktionslösung dann auf Raumtemperatur erwärmt. Anschließend wurde das Reaktionsgemisch mit gesättigter Ammoniumchloridlösung (100 mL) versetzt, die Phasen wurden getrennt und die wässrige Phase mit Diethylether (3 × 50 mL) extrahiert. Die vereinigten organischen Phasen wurden mit Natriumchloridlösung (50 mL) gewaschen, mit Natriumsulfat getrocknet und i. Vak. eingeengt. Das Rohprodukt wurde mittels Flashchromatographie (400 g, 20 × 7.5 cm) mit Cyclohexan / Ethylacetat (4:1) gereinigt.
Ausbeute: 3.90 g (48 %), gelbes Öl (enthält ca. 25% 4-Benzyl-1-tert-butyl-4-dimethylaminocyclohexanol)
¹H-NMR (DMSO-d₆): 0.47-0.55 (m, 6H); 0.93 (t, 9H, J= 7.9 Hz); 1.10-1.14 (m, 2H); 1.28-1.33 (m, 2H); 1.37-1.52 (m, 8H); 2.15 (t, 2H, J= 6.8 Hz); 2.22 (s, 6H); 2.54-2.56 (m, 2H); 3.45 (s, 1 H); 7.11-7.18 (m, 3H); 7.22-7.27 (m, 2H).
Es handelt sich um ein einheitliches Diastereoisomer.
LC-MS: m/z: [M+H]⁺ = 414.4, Rₜ= 3.3 min.

### Stufe 2:

### 4-Benzyl-4-dimethylamino-1-[3-(2-(triethylsilyl)-1H-indol-3-yl)propyl]cyclohexanol

Ein Gemisch von 4-Benzyl-4-dimethylamino-1-(5-triethylsilyl)pent-4-inyl)cyclohexanol (900 mg, 1.64 mmol), 2-Iodanilin (431 mg, 1.97 mmol), [1,3-Bis-(2,6-diisopropylphenyl)imidazol-2-yliden]-(3-chlorpyridyl)palladium(II)-chlorid (PEPPSI, 109 mg, 0.16 mmol) und Natriumcarbonat (869 mg, 8.20 mmol) wurde entgast, unter Argon mit sauerstofffreiem *N,N-*Dimethylformamid (10 mL) versetzt und 18 h bei 100 °C gerührt. Anschließend wurde das Reaktionsgemisch i. Vak. eingeengt, der Rückstand wiederholt in Toluol (10 mL) aufgenommen und jeweils wieder i. Vak. eingeengt. Dieser Rückstand wurde zwischen Ethylacetat (30 mL) und Wasser (30 mL) verteilt, die organische Phase mit Wasser (2 × 15 mL) und Natriumthiosulfat-Lösung (2 × 15 mL) gewaschen, mit Natriumsulfat getrocknet und i. Vak. eingeengt. Das Rohprodukt wurde mittels Flashchromatographie (100 g, 20 x 4.0 cm) mit Dichlormethan / Methanol (95:5) gereinigt.
Ausbeute: 707 mg (85 %), braunes Öl
¹H-NMR (DMSO-d₆):0.83-0.88 (m, 6H); 0.90-0.95 (m, 9H); 1.07-1.11 (m, 2H); 1.30-1.49 (m, 8H); 1.50-1.60 (m, 2H); 2.19 (s, 6H); 2.54 (s, 2H); 2.66 (t, 2H, J = 7.9 Hz); 3.38 (s, 1H); 6.92 (ddd, 1H, J= 1.0, 7.0, 7.9 Hz); 7.03 (ddd, 1H, J= 1.1, 6.9, 8.1 Hz); 7.10-7.16 (m, 3H); 7.23 (t, 2H, J = 7.3 Hz); 7.34 (d, 1H, J = 8.1 Hz); 7.45 (d, 1H, J = 8.0 Hz); 10.33 (s, 1 H).
¹³C-NMR (DMSO-d₆): 3.8; 7.8; 26.4; 26.9; 27.7; 31.7; 36.7; 37.2; 44.9; 57.9; 69.3; 111.9; 118.5; 118.8; 121.7; 125.8; 126.0; 128.2; 128.7; 130.4; 131.1; 139.4; 139.7
LC-MS: m/z: [M+H]⁺ = 505.5, Rₜ = 3.4 min.

### Stufe 3:

### 1-(3-(1H-Indol-3-yl)propyl)-4-benzyl-4-(dimethylamino)cyclohexanol

Eine Lösung von 4-Benzyl-4-dimethylamino-1-[3-(2-(triethylsilyl)-1H-indol-3-yl)propyl]cyclohexanol (2.50 g, 4.95 mmol) in wasserfreiem Tetrahydrofuran (25 mL) wurde mit Tetra-n-butylammoniumfluorid-Trihydrat (6.25 g, 19.8 mmol) versetzt, 7 h unter Rückfluss gekocht und dann über Nacht bei Raumtemperatur gerührt. Da die Umsetzung noch nicht vollständig war, wurde das Reaktionsgemisch weitere 7 h unter Rückfluss gekocht und dann über das Wochenende bei Raumtemperatur gerührt. Das Reaktionsgemisch wurde i. Vak. eingeengt und der Rückstand mittels Flashchromatographie (400 g, 20 x 7.5 cm) mit Dichlormethan / Methanol (95:5 + 1 % konz. wässriges Ammoniak) gereinigt.
Ausbeute: 1.32 g (68 %), weißer Feststoff
Schmelzpunkt: 202-215 °C
¹H-NMR (DMSO-d₆): 1.12 (d, 2H, J = 10.0 Hz); 1.27-1.33 (m, 2H); 1.37-1.51 (m, 6H); 1.55-1.67 (m, 2H); 2.20 (s, 6H); 2.54 (s, 2H); 2.60 (t, 2H, J= 7.4 Hz); 3.39 (s, 1 H); 6.90-6.96 (m, 1H); 7.00-7.06 (m, 2H); 7.09-7.18 (m, 3H); 7.20-7.27 (m, 2H); 7.30 (d, 1H, J = 8.0 Hz); 7.46 (dd, 1H, J = 0.5, 7.8 Hz); 10.69 (s, 1 H).
¹³C-NMR (DMSO-d₆): 23.8; 25.4; 27.2; 31.5; 36.1; 36.7; 44.3; 57.2; 68.4; 111.2; 114.9; 117.9; 118.3; 120.6; 122.0; 125.3; 127.2; 127.6; 130.6; 136.2; 139.3.
LC-MS: m/z: [M+H]⁺ = 391.3, Rₜ = 2.1 min.

### Stufe 4:

### 4'-Benzyl-N,N-dimethyl-2,3,4,9-tetrahydrospiro(carbazol-1,1'-cyclohexan]-4'-amin (Beispiel Nr. 9, unpolares Diastereomer und Beispiel Nr. 10, polares Diastereomer)

Zu einer Lösung von 1-(3-(1 H-Indol-3-yl)propyl)-4-benzyl-4-(dimethylamino)cyclohexanol (700 mg, 1.79 mmol) in wasserfreiem Dichlormethan (30 mL) wurde Trifluormethansulfonsäuretrimethylsilylester (1.30 mL, 1.59 g, 7.16 mmol) gegeben und 2 d bei Raumtemperatur gerührt. Anschließend wurde das Reaktionsgemisch mit Dichlormethan (10 mL) verdünnt, zunächst mit 1 N Natriumcarbonat-Lösung (2 × 10 mL), dann mit Wasser und gesättigter Natriumchloridlösung (je 10 mL) gewaschen, mit Natriumsulfat getrocknet und i. Vak. eingeengt. Der Rückstand wurde in Methanol (30 mL) aufgenommen, über das Wochenende bei Raumtemperatur gerührt und von ausgefallenem Niederschlag (Fraktion 1) abdekantiert. Die Lösung wurde eingeengt, der Rückstand in Methanol (2 x 10 mL) aufgenommen und von ausgefallenem Feststoff jeweils erneut abdekantiert. Die verbleibende Lösung wurde i. Vak. eingeengt, wodurch Fraktion 2 erhalten wurde.
Beispiel Nr. 9: Fraktion 2 (Methanol-lösliche Fraktion, unpolares Diastereomer) wurde durch präparative HPLC gereinigt.
Ausbeute: 510 mg (75 %), braunes Öl
LC-MS: m/z: [M+1]⁺ = 373.3 Rₜ 4.1
Beispiel Nr. 10: Fraktion 1 (Methanol-unlösliche Fraktion, polares Diastereomer) wurde durch präparative HPLC gereinigt.
Ausbeute: 135 mg (20 %), beigefarbener Feststoff
Schmelzpunkt: 174-186 °C
LC-MS: m/z: [M+1]⁺ = 373.3 Rₜ 3.8
¹H-NMR(DMSO-d₆): 1.14-1.41 (m, 6H); 1.55-1.64 (m, 2H); 1.65-1.74 (m, 2H); 2.09-2.22 (m, 2H); 2.36 (s, 6H); 2.63 (s, 2H); 6.83-6.89 (m, 1 H); 6.92-6.98 (m, 1 H); 7.16-7.32 (m, 7H); 10.42 (s, 1 H).
¹³C-NMR (DMSO-d₆): 19.2; 21.0; 27.0; 30.6; 30.7; 33.6; 36.4; 37.0; 56.9; 107.4; 110.9; 117.1; 117.7; 119.9; 125.6; 126.9; 127.7; 130.5; 135.9; 139.2; 142.3.

### Beispiel Nr. 11 und Beispiel Nr. 12

### Stufe 1:

### 4-Dimethylamino-4-(3-fluorphenyl)-1-(5-(triethylsilyl)pent-4-inyl)cyclohexanol

Zu einer Lösung von (7.20 g, 23.4 mmol) in wasserfreiem Diethylether (150 mL) in einer ausgeheizten Apparatur wurde bei -75 °C unter Argon eine 1.7 M Lösung von *tert-*Butyllithium (27.5 mL, 46.8 mmol) in Pentan mittels Kanüle direkt in die Lösung eingespritzt, so dass die Innentemperatur zwischen -70 °C und -75 °C gehalten wurde. Nach 120 min Rühren wurde eine Lösung von 4-Dimethylamino-4-(3-fluorphenyl)cyclohexanon (5.51 g, 23.4 mmol) in wasserfreiem Diethylether (150 mL) langsam zugetropft, 30 min nachgerührt und die Reaktionslösung dann auf Raumtemperatur erwärmt. Anschließend wurde das Reaktionsgemisch mit gesättigter Ammoniumchloridlösung (100 mL) versetzt, die Phasen wurden getrennt und die wässrige Phase mit Diethylether (3 x 50 mL) extrahiert. Die vereinigten organischen Phasen wurden mit Natriumchloridlösung (50 mL) gewaschen, mit Natriumsulfat getrocknet und i. Vak. eingeengt. Das Rohprodukt wurde mittels Flashchromatographie (400 g, 20 x 7.5 cm) mit Cyclohexan / Ethylacetat (3:1) gereinigt.
Ausbeute: 3.46 g (35 %), gelbes Öl
¹H-NMR (DMSO-d₆): 0.54 (q, 6H, J = 7.9, 8.1 Hz); 0.95 (t, 9H, J = 7.9 Hz); 1.25-1.33 (m, 2H); 1.44-1.65 (m, 6H); 1.81 (t, 2H, J = 12.5 Hz); 1.92 (s, 6H); 2.11-2.24 (m, 4H); 3.90 (s, 1 H); 7.02-7.17 (m, 3H); 7.38 (dd, 1H, J = 14.5, 7.9 Hz).
Es handelt sich um ein einheitliches Diastereoisomer.

### Stufe 2:

### 4-Dimethylamino-4-(3-fluorphenyl)-1-(3-(2-(triethylsilyl)-1H-indol-3-yl)propyl]cyclohexanol

Ein Gemisch von 4-Dimethylamino-4-(3-fluol-phenyl)-1-(5-(triethylsilyl)pent-4-inyl)cyclohexanol (970 mg, 2.32 mmol), 2-lodanilin (609 mg, 2.78 mmol), [1,3-Bis-(2,6-düsopropylphenyl)imidazol-2-yliden]-(3-chlorpyridyl)palladium(II)-chlorid (PEPPSI, 156 mg, 0.23 mmol) und Natriumcarbonat (1.23 g, 11.6 mmol) wurde entgast, unter Argon mit sauerstofffreiem N,N-Dimethylformamid (10 mL) versetzt und 7 h bei 100 °C gerührt. Anschließend wurde das Reaktionsgemisch i. Vak. eingeengt, der Rückstand wiederholt in Toluol (10 mL) aufgenommen und jeweils wieder i. Vak. eingeengt. Dieser Rückstand wurde zwischen Ethylacetat (30 mL) und Wasser (30 mL) verteilt, die organische Phase mit Wasser (2 × 15 mL) und Natriumthiosulfat-Lösung (2 × 15 mL) gewaschen, mit Natriumsulfat getrocknet und i. Vak. eingeengt. Das Rohprodukt wurde mittels Flashchromatographie (100 g, 20 × 4.0 cm) mit Cyclohexan / Ethylacetat (4:1) gereinigt.
Ausbeute: 1.11 g (94 %), gelbes Öl
¹H-NMR (DMSO-d₆): 0.83-1.00 (m, 15H); 1.22-1.33 (m, 2H); 1.45-1.82 (m, 8H); 1.89 (s, 6H); 2.10-2.23 (m, 2H); 2.72 (t, 2H, J = 7.7 Hz); 3.84 (s, 1H); 6.94 (ddd, 1H, J = 1.0, 7.0, 7.9 Hz); 7.01-7.16 (m, 4H); 7.33-7.40 (m, 2H); 7.50 (d, 1 H, J = 7.8 Hz); 10.37 (s, 1 H). ¹³C-NMR (DMSO-d₆): 3.3; 7.4; 25.8; 26.3; 26.4; 28.4; 32.4; 37.7; 43.0; 54.8; 58.8; 68.6; 111.2; 112.8 (d, J = 21 Hz); 113.5 (d, J = 21 Hz); 117.8; 118.3; 121.1; 122.8; 125.3; 128.2; 128.8 (d, J = 8 Hz); 129.7; 138.9; 142.5 (d, J = 5 Hz); 161.9 (d, J = 242 Hz).
LC-MS: m/z: [M+H]⁺ = 509.4, Rₜ = 3.2 min.

### Stufe 3:

### 1-(3-(1H-Indol-3-yl)propyl)-4-(dimethylamino)-4-(3-fluorphenyl)cyclohexanol

Eine Lösung von 4-Dimethylamino-4-(3-fluorphenyl)-1-[3-(2-(triethylsilyl)-1*H*-indol-3-yl)propyl]cyclohexanol (1.11 g, 2.18 mmol) in wasserfreiem Tetrahydrofuran (25 mL) wurde mit Tetra-n-butylammoniumfluorid-Trihydrat (2.75 g, 8.72 mmol) versetzt und 3 h unter Rückfluss gekocht. Das Reaktionsgemisch wurde anschließend i. Vak. eingeengt und das Rohprodukt mittels Flashchromatographie (100 g, 20 x 4.0 cm) mit Dichlormethan / Methanol (93:7) gereinigt.
Ausbeute: 550 mg (64 %), weißer Feststoff
Schmelzpunkt: 196-204 °C
¹H-NMR (DMSO-d₆): 1.26-1.36 (m, 2H); 1.42-1.50 (m, 2H); 1.54-1.65 (m, 2H); 1.67-1.83 (m, 4H); 1.90 (s, 6H); 2.12-2.23 (m, 2H); 2.67 (t, 2H, J = 7.3 Hz); 3.84 (s, 1 H); 6.93-6.99 (m, 1 H); 7.02-7.18 (m, 5H); 7.31-7.34 (m, 1 H); 7.38 (d, 1H, J = 6.9 Hz); 7.50 (d, 1H, J =-7.7 Hz); 10.72 (s, 1 H).
¹³C-NMR (DMSO-d₆): 23.8; 25.4; 28.5; 32.5; 37.7; 42.7; 58.8; 68.7; 111.2; 112.8 (d, J = 21 Hz); 113.5 (d, J = 21 Hz); 114.9; 117.9; 118.3; 120.7; 122.0; 122.8; 127.2; 128.8 (d, J = 8 Hz); 136.3; 142.5; 161.9 (d, J = 242 Hz).
LC-MS: m/z: [M+H]⁺ = 395.3, Rₜ = 1.90 min.

### Stufe 4:

### 4'-(3-Fluorphenyl)-N,N-dimethyl-N-2,3,4,9-tetrahydrospiro[carbazol-1,1'-cyclohexan]-4'-amin (Beispiel Nr. 11, polares Diastereomer und Beispiel Nr. 12, unpolares Diastereomer)

Zu einer Lösung von 1-(3-(1*H*-Indol-3-yl)propyl)-4-(dimethylamino)-4-(3-fluorphenyl)cyclohexanol (1.00 g, 2.53 mmol) in wasserfreiem Dichlormethan (30 mL) wurde Trifluormethansulfonsäuretrimethylsilylester (2.25 g, 1.84 mL, 10.1 mmol) gegeben und 8 d bei Raumtemperatur gerührt. Anschließend wurde das Reaktionsgemisch mit Dichlormethan (30 mL) verdünnt, zunächst mit 1 N Natriumcarbonat-Lösung (2 x 30 mL), dann mit Wasser und gesättigter Natriumchloridlösung (je 30 mL) gewaschen, mit Natriumsulfat getrocknet und i. Vak. eingeengt. Der Rückstand wurde in Methanol (15 mL) aufgenommen, über Nacht bei Raumtemperatur gerührt und von ausgefallenem Niederschlag abdekantiert. Die Lösung wurde i. Vak. eingeengt und der Rückstand in Methanol (2 × 10 mL) aufgenommen und von ausgefallenem Feststoff erneut abdekantiert. Der Feststoff ist ein Gemisch aus zwei Diastereomeren im Verhältnis 2:1. Diese (432 mg) werden durch präparative HPLC getrennt.
Beispiel Nr. 11: (polares Diastereomer)
Ausbeute: 66 mg (7 %)
Schmelzpunkt: 170-182 °C
¹H-NMR (DMSO-d₆), charakteristische Signale: 1.92 (s, 2H, NMe₂), 2.06 (s, 4H, NMe₂); 10.17 (s, 0.33H, Indol-NH), 10.55 (s, 0.67H, Indol-NH).
LC-MS : m/z: [M+H]⁺ = 377.3, Rₜ = 3.7 min.
Beispiel Nr. 12: (unpolares Diastereomer)
Ausbeute: 156 mg (16 %)
LC-MS : m/z: [M+H]⁺ = 377.3, Rₜ = 3.9 min.

### Beispiel Nr. 13 und Beispiel Nr. 14

### Stufe 1:

### 4-Dimethylamino-4-(thiophen-2-yl)-1-(5-(triethylsilyl)pent-4-inyl)cyclohexanol

In einer ausgeheizten Apparatur wurde unter Argon in eine Lösung von Triethyl-(5-iodpent-1-inyl)silan (5.64 g, 18.9 mmol) in absolutem Diethylether (50 mL) bei einer Innentemperatur von -70 °C bis -75 °C langsam eine 1.7 M Lösung von tert-Butyllithium in Pentan (22.3 mL, 37.9 mmol) getropft. Nach 120 min wurde bei dieser Temperatur eine Lösung von 4-Dimethylamino-4-(thiophen-2-yl)cyclohexan (4.22 g, 18.9 mmol) in absolutem Diethylether (100 mL) langsam zugetropft und 30 min nachgerührt. Die Reaktionslösung wurde anschließend auf Raumtemperatur erwärmt und mit gesättigter Ammoniumchlorid-Lösung (100 mL) versetzt. Die Phasen wurden getrennt und die wässrige Phase mit Diethylether (3 × 50 mL) extrahiert. Die vereinigten organischen Phasen wurden mit gesättigter Natriumchlorid-Lösung (50 mL) gewaschen, mit Natriumsulfat getrocknet und i. Vak. eingeengt. Der Rückstand (7.02 g) wurde mittels Flashchromatographie (100 g, 20 × 4.0 cm) mit Cyclohexan / Ethylacetat (2:1) gereinigt.
Ausbeute: 3.00 g (39 %), gelbes zähes Öl
¹H-NMR (DMSO-d₆): 0.54 (q, 6H; J = 7.9 Hz); 0.95 (t, 9H, J = 7.8 Hz); 1.23-1.66 (m, 8H); 1.84-1.96 (m, 2H); 1.99 (s, 6H); 2.10-2.11 (m, 2H); 2.22 (t, 2H; J = 6.3 Hz); 3.94 (s, 1 H); 6.91 (d, 1 H; J = 3.0 Hz); 7.04 (dd, 1H, J = 1.2, 4.9 Hz); 7.38 (d, 1H; J = 4.9 Hz).
¹³C-NMR (DMSO-d₆): 4.0; 7.3; 19.7; 22.3; 31.0; 32.4; 37.7; 41.7; 58.4; 68.5; 80.9; 109.4; 123.1; 123.9; 126.1; 144.8.
LC-MS: m/z: [M+1]⁺ = 406.7, Rₜ 3.2 min.

### Stufe 2:

### 4-Dimethylamino-4-(thiophen-2-yl)-1-(3-(2-(triethylsilyl)-1H-indol-3-yl)propyl)cyclohexanol

Eine Mischung von 4-Dimethylamino-4-(thiophen-2-yl)-1-(5-(triethylsilyl)pent-4-inyl)cyclohexanol (3.00 g, 7.4 mmol), 2-lodanilin (1.94 g, 8.9 mmol), [1,3-Bis(2,6-diisopropylphenyl)imidazol-2-yliden]-3-chlorpyridyl-palladium(II)-chlorid (PEPPSI, 503 mg, 0.74 mmol) und Natriumcarbonat (3.92 g, 37 mmol) wurde 30 min evakuiert (Ölpumpe). Anschließend wurde mit Argon gespült und über einen Schlenk-Aufsatz absolutes N,N-Dimethylformamid (10 mL, vorher 1 h mit Argon gespült) per Spritze zugegeben. Das Reaktionsgemisch wurde 18 h bei 100 °C gerührt, wobei sich die Lösung dunkelbraun färbte. Anschließend wurde das Reaktionsgemisch i. Vak. eingeengt, der Rückstand mehrfach mit Toluol versetzt (3 × 30 mL) und jeweils wieder eingeengt. Der Rückstand wurde zwischen Wasser und Ethylacetat (je 100 mL) verteilt, die Phasen wurden getrennt und die wässrige Phase mit Ethylacetat (2 × 50 mL) extrahiert. Die vereinigten organischen Phasen wurden mit 1 M Natriumthiosulfat-Lösung und gesättigter Natriumchlorid-Lösung (je 50 mL) gewaschen, mit Natriumsulfat getrocknet und i. Vak. eingeengt. Der Rückstand (4.40 g) wurde mittels Flashchromatographie (200 g, 20 × 5.7 cm) mit Dichlormethan / Methanol (95:5) gereinigt.
Ausbeute: 2.20 g (62 %), braunes zähes Öl
¹H-NMR (DMSO-d₆): 0.89-0.99 (m, 15H); 1.26-1.71 (m, 8H); 1.81-1.93 (m, 2H); 1.96 (s, 6H); 2.01-2.10 (m, 2H); 2.72 (t, 2H, J = 7.5 Hz); 3.88 (s, 1H); 6.89-6.97 (m, 2H); 7.01-7.07 (m, 2H); 7.35-7.38 (m, 2H); 7.50 (d, 1H; J = 7.8 Hz); 10.37 (s, 1 H).
¹³C-NMR (DMSO-d₆): 3.2; 7.4; 25.3; 25.8; 26.4; 30.9; 32.2; 37.6; 43.4; 58.3; 68.6; 111.2; 117.8; 118.3; 121.0; 123.0; 123.9; 125.2; 126.1; 128.2; 129.8; 138.9; 144.9.
LC-MS: m/z: [M+1]⁺ = 497.8, Rₜ 3.1 min.

### Stufe 3:

### 1-(3-(1H-Indol-3-yl)propyl)-4-(dimethylamino)-4-(thiophen-2-yl)cyclohexanol

Eine Lösung von 4-Dimethylamino-4-(thiophen-2-yl)-1-(3-(2-(triethylsilyl)-1*H*-indol-3-yl)propyl)cyclohexanol (2.30 g, 4.6 mmol) in absolutem Tetrahydrofuran (100 mL) wurde mit Tetra-n-butylammoniumfluorid-Trihydrat (5.68 g, 20 mmol) versetzt und 4 h unter Rückfluss, dann über Nacht bei Raumtemperatur gerührt. Da die Umsetzung noch nicht vollständig war, wurde weitere 6 h unter Rückfluss gerührt. Das Reaktionsgemisch wurde i. Vak. eingeengt und der Rückstand mittels Flashchromatographie (100 g, 20 x 4.0 cm) mit Dichlormethan / Methanol (95:5) gereinigt.
Ausbeute: 1.56 g (89 %) ,heller Feststoff
Schmelzpunkt: 163-165 °C
¹H-NMR (DMSO-d₆): 1.26-1.37 (m, 2H); 1.41-1.47 (m, 2H); 1.54-1.64 (m, 2H); 1.69-1.77 (m, 2H); 1.85-1.94 (m, 2H); 1.98 (s, 6H); 2.02-2.10 (m, 2H); 2.67 (t, 2H, J = 7.4 Hz); 3.88 (s, 1 H); 6.90 (dd, 1 H, J = 1.1, 3.6 Hz); 7.00 (ddd, 1H, J = 1.1; 7.0; 8.0 Hz); 7.02-7.05 (m, 2H); 7.09 (d, 1H, J = 2.2 Hz); 7.32 (td, 1 H, J = 0.9; 8.1 Hz); 7.38 (dd, 1H, J = 1.1; 5.1 Hz); 7.50 (d, 1H, J=7.9Hz); 10.71 (s, 1 H).
¹³C-NMR (DMSO-d₆): 23.7; 25.4; 31.0; 32.4; 37.7; 43.0; 58.3; 68.6; 111.2; 114.9; 117.9; 118.3; 120.6; 122.0; 123.0; 123.9; 126.1; 127.2; 136.2, 145.0.
LC-MS: m/z: [M+1]⁺ = 383.6, Rₜ 1.9 min.

### Stufe 4:

### N, N-Dimethyl-4'-(thiophen2-yl)-2,3,4,9-tetrahydrospiro(carbazol-1,1'-cyclohexan]-4'-amin (Beispiel Nr. 13, polares Diastereomer und Beispiel Nr. 14, unpolares Diastereomer)

Zu einer Aufschlämmung von 1-(3-(1*H*-Indol-3-yl)propyl)-4-(dimethylamino)-4-(thiophen-2-yl)cyclohexanol(800 mg, 2.1 mmol) in absolutem 1,2-Dichlorethan (100 mL) wurde Trifluormethansulfonsäuretrimethylsilylester (1.95 g, 1.59 mL, 8.8 mmol) gegeben, wobei sich eine klare Lösung bildete. Die Lösung wurde 6 h bei 50 °C und anschließend über Nacht bei Raumtemperatur gerührt. Die Reaktionslösung wurde nacheinander mit 1 *N* Natriumcarbonatlösung (2 × 30 mL), Wasser und gesättigter Natriumchlorid-Lösung (je 30 mL) gewaschen, mit Natriumsulfat getrocknet und i. Vak. eingeengt. Das Rohprodukt (720 mg) wurde mit Methanol (100 mL) versetzt und über Nacht bei Raumtemperatur gerührt, wobei sich ein heller Feststoff bildete. Dieser wurde abfiltriert, mit Methanol (10 mL) gewaschen und i. Vak. getrocknet, wodurch ein Diastereomerengemisch im Verhältnis 5:1 erhalten wurde (290 mg (38%)). Dieses wurde durch präparative HPLC getrennt.
Beispiel Nr. 13: (polares Diastereomer)
Ausbeute: 37 mg (5 %).
LC-MS: m/z: [M+1]⁺ = 365.2, Rₜ 3.5
Beispiel Nr. 14: (unpolares Diastereomer)
Ausbeute:174 mg (23%).
LC-MS: m/z: [M+1]⁺ = 365.2, Rₜ 3.8.

### Beispiel Nr. 15

### Stufe 1:

### 2-((S)-3-Brom-2-methylpropoxy)tetrahydropyran

Eine Lösung von (S)-3-Brom-2-methylpropan-1-ol (7.30 g, 5 mL, 47.7 mmol) in wasserfreiem Dichlormethan (100 mL) wurde mit 3,4-Dihydro-2H-pyran (4.63 g, 5.02 mL, 55 mmol) und Pyridiniumtosylat (50 mg) unter Eiskühlung versetzt und danach 1 h bei Raumtemperatur gerührt. Das Reaktionsgemisch wurde anschließend mit 5 % Natriumhydrogencarbonat-Lösung und Wasser (je 3 × 30 mL) gewaschen. Die vereinigten organischen Phasen wurden mit Natriumsulfat getrocknet und i. Vak. eingeengt.
Ausbeute: 11.1 g (98 %), farbloses Öl
¹H-NMR (DMSO-d₆): 0.96 (d, 1.5H, J = 6.7 Hz); 0.97 (d, 1.5H, J = 6.7 Hz); 1.40-1.80 (m, 6H); 1.97-2.13 (m, 1 H); 3.22-3.29 (m, 1 H); 3.39-3.48 (m,1H); 3.51-3.60 (m, 3H); 3.69-3.79 (m, 1 H); 4.53-4.58 (m, 1 H).
Es handelt sich um ein Diastereoisomerengemisch.

### Stufe 2:

### Triethyl-[(R)-4-methyl-5-(tetrahydro-2H-pyran-2-yloxy)pent-1-inyl]silan

Eine Lösung von Triethylsilylacetylen (6.25 g, 8 mL, 44.7 mmol) in wasserfreiem Tetrahydrofuran (150 mL) wurde bei -70 °C unter Argon mit einer 2.5 M-Lösung von n-Butyllithium (19.6 mL, 49.2 mmol) in Hexan versetzt. Nach 15 min Rühren bei dieser Temperatur wurde Hexamethylphosphorsäuretriamid (19.4 g, 18.8 mL, 108 mmol) zugegeben und weitere 15 min gerührt. Zu dieser Lösung wurde eine Lösung von 2-((S)-3-Brom-2-methylpropoxy)tetrahydropyran (10.6 g, 44.7 mmol) in wasserfreiem Tetrahydrofuran (40 mL) getropft und danach langsam auf Raumtemperatur erwärmt. Nach Zugabe von gesättigter Ammmoniumchloridlösung (40 mL) wurde die Mischung i. Vak. eingeengt, der Rückstand in Wasser (150 mL) aufgenommen und mit Cyclohexan / Diethylether (1:1, 3 × 100 mL) extrahiert. Die vereinigten organischen Phasen wurden mit Natriumsulfat getrocknet und i. Vak. eingeengt. Das Rohprodukt (11 g) wurde durch Flashchromatographie (400 g, 20 × 7.5 cm) mit Ethylacetat / Cyclohexan (1:30) gereinigt.
Ausbeute: 4.58 g (35 %), farbloses Öl
¹H-NMR (DMSO-d₆): 0.47-0.58 (m, 6H); 0.90-0.98 (m, 12H); 1.41-1.52 (m, 4H); 1.56-1.74 (m, 1 H); 1.80-1.92 (m, 1 H); 2.17-2.36 (m, 2H); 3.16-3.27 (m, 1 H); 3.36-3.45 (m, 1 H); 3.48-3.58 (m, 1 H); 3.68-3.58 (m, 1 H); 3.68-3.78 (m, 1 H); 4.50-4.57 (m, 1 H).
¹³C-NMR (DMSO-d₆): 4.0; 7.3; 16.0; 16.1; 18.9; 19.0; 23.1; 23.2; 24.98; 25.0; 26.3; 30.1; 30.2; 32.3; 32.4; 60.9; 61.1; 70.0; 70.3; 82.2; 97.6; 98.1; 107.0; 107.1.
Es handelt sich um ein Diastereoisomerengemisch.

### Stufe 3:

### (R)-2-Methyl-5-triethylsilanylpent-4-in-1-ol

Eine Lösung von Triethyl-[(*R*)-4-methyl-5-(tetrahydro-2H-pyran-2-yloxy)pent-1-inyl]silan (3.84 g, 13 mmol) in Tetrahydrofuran (50 mL) wurde mit 2 N Salzsäure (50 mL) versetzt und 6 h bei 50 °C gerührt. Das Reaktionsgemisch wurde anschließend mit 2 N Natronlauge (50 mL) auf pH 7 eingestellt, i. Vak. etwas eingeengt, und die erhaltene wässrige Phase mit Ethylacetat (3 × 30 mL) extrahiert. Die vereinigten organischen Phasen wurden mit Natriumsulfat getrocknet und i. Vak. eingeengt. Das Rohprodukt (3.33 g) wurde durch Flashchromatographie (200 g, 20 × 5.7 cm) mit Ethylacetat / Cyclohexan (1:7) gereinigt.
Ausbeute: 1.79 g (65 %), farbloses Öl
¹H-NMR (DMSO-d₆): 0.53 (q, 6H, J = 8.2 Hz); 0.93 (q, 9H, J = 6.6 Hz); 0.95 (d, 3H, J = 8.0 Hz); 1.67 (dt, 1H, J = 13.3; 6.6 Hz), 2.12 (dd, 1H, J = 16.9, 7.0 Hz); 2.29 (dd, 1 H, J = 16.9, 5.4 Hz); 3.24-3.30 (m, 2H); 4.52 (t, 1 H, J = 5.3 Hz).

### Stufe 4:

### Triethyl-((R)-5-iod-4-methylpent-1-inyl)silan

Zu einer Lösung von Triphenylphosphindiiodid (10.6 g, 20.6 mmol) und Imidazol (4.2 g, 61.8 mmol) in wasserfreiem Acetonitril (100 mL) wurde unter Argon eine Lösung von (R)-2-Methyl-5-triethylsilanylpent-4-in-1-ol (2.18 g, 10.3 mmol) in wasserfreiem Acetonitril (50 mL) getropft und 24 h bei Raumtemperatur gerührt. Anschließend wurde das Lösungsmittel i. Vak. eingeengt und der Rückstand durch Flashchromatographie (100 g, 20 x 4.5 cm) mit Ethylacetat / Cyclohexan (1:9) gereinigt.
Ausbeute: 2.60 g (78 %), farbloses Öl
¹H-NMR (DMSO-d₆): 0.44-0.59 (m, 6H); 0.87-1.03 (m, 12H); 1.63-1.75 (m, 1 H); 2.28-2.32 (m, 2H); 3.29-3.35 (m, 2H, überlagert vom HDO-Signal).
Drehwert: [α]_{D}²⁴ = -2.48° (c1.0, MeOH).

### Stufe 5:

### (R)-4-Dimethylamino-1-(2-methyl-5-(triethylsilyl)pent4-inyl)-4-phenylcyclohexanol

Zu einer Lösung von Triethy)-((R)-5-iod-4-methylpent-1-inyl)silan (2.15 g, 6.6 mmol) in wasserfreiem Diethylether (100 mL) wurde bei -85 °C unter Argon eine 1.7 M Lösung von tert-Butyllithium (7.8 mL, 13.3 mmol) in Pentan getropft, wobei die Innentemperatur bei -85 °C gehalten wurde. Nach 2 h Rühren bei -85 °C wurde eine Lösung von 4-Dimethylamino-4-phenylhexanon (1.43 g, 6.6 mmol) in wasserfreiem Diethylether (30 mL) bei dieser Temperatur zugetropft und weitere 30 min gerührt. Zur Lösung wurde danach eine Lösung von Trimethylchlorsilan (1.44 g, 1.69 mL, 13.3 mmol) in Diethylether (12 mL) getropft und langsam auf Raumtemperatur erwärmt. Das Reaktionsgemisch wurde anschließend mit gesättigter Ammoniumchloridlösung (20 mL) versetzt und der ausgefallene Feststoff (2.00 g) abfiltriert. Die Phasen wurden getrennt und die wässrige mit Diethylether (3 × 10 mL) extrahiert. Die vereinigten Diethylether-Phasen wurden mit Natriumchloridlösung (20 mL) gewaschen, mit Natriumsulfat getrocknet, filtriert und i. Vak. eingeengt. Aus dieser Phase konnte kein Zielprodukt isoliert werden. Der ausgefallene Feststoff (2.00 g) wurde daraufhin mit gesättigter Natriumhydrogencarbonat-Lösung (50 mL) versetzt und die resultierende Suspension mit Ethylacetat (3 x 30 mL) extrahiert. Die vereinigten Ethylacetat-Phasen wurden mit Natriumsulfat getrocknet und i. Vak. eingeengt. Das Rohprodukt (351 mg) wurde durch Flashchromatographie (18 g, 20 × 2.0 cm) mit Ethylacetat / Cyclohexan (1:2) gereinigt.
Ausbeute: 237 mg (9 %), farbloses Öl
¹H-NMR (DMSO-d₆): 0.54 (q, 6H, J = 8.4 Hz); 0.96 (t, 9H, J = 7.8 Hz); 1.03 (d, 3H, J = 6.7 Hz); 1.20-1.40 (m, 3H); 1.52-1.70 (m, 3H); 1.74-1.89 (m, 3H); 1.91 (s, 6H); 2.10-2.34 (m, 4H); 3.85 (s, 1H); 7.19-7.39 (m, 5H).
Es handelt sich um ein einheitliches Diastereoisomer.

### Stufe 6:

### (R)-4-Dimethylamino-1-[2-methyl-3-(2-(triethylsilyl)-1H-indol-3-yl)propyl]-4-phenylcyclohexanol

Eine Lösung von (R)-4-Dimethylamino-1-(2-methyl-5-(triethylsilyl)pent-4-inyl)-4-phenylcyclohexanol (106 mg, 0.25 mmol), [1,3-Bis-(2,6-diisopropylphenyl)imidazol-2-yliden]-(3-chlorpyridyl)palladium(II)-chlorid (PEPPSI, 34 mg, 0.05 mmol), 2-lodanilin (68 mg, 0.31 mmol) und Natriumcarbonat (136 mg, 1.28 mmol) in sauerstoff-und wasserfreiem N,N-Dimethylformamid (5 mL) wurde 18 h bei 100°C gerührt. Das Reaktionsgemisch wurde i. Vak. eingeengt, der Rückstand mehrmals mit Toluol versetzt und das Gemisch jeweils wieder eingeengt. Dieser Rückstand wurde zwischen Wasser und Ethylacetat (je 10 mL) verteilt, die Phasen wurden getrennt und die wässrige Phase mit Ethylacetat (3 × 10 mL) extrahiert. Die vereinigten organischen Phasen wurden mit 1 M Natriumthiosulfatlösung und Wasser (je 10 mL) gewaschen, mit Natriumsulfat getrocknet, filtriert und i. Vak. eingeengt. Das Rohprodukt wurde durch Flashchromatographie (10 g, 20 x 1.5 cm) mit Ethylacetat / Cyclohexan (1:2) gereinigt.
Ausbeute: 89 mg (71 %), gelbliches Öl
¹H-NMR (CDCl₃): 0.87-1.13 (m, 18H); 1.48-1.57 (m, 4H); 1.73-1.95 (m, 4H); 2.00 (s, 6H); 2.03-2.10 (m, 2H); 2.11-2.40 (m, 2H); 2.69 (dd, 1H, J = 14.0, 8.6 Hz); 2.92 (dd, 1 H, J = 14.0, 6.8 Hz); 7.06-7.40 (m, 8H); 7.70 (d, 1H, J = 7.8 Hz); 7.90 (s, 1 H).
¹³C-NMR (CDCl₃): 3.9; 7.4; 22.4; 29.0; 29.1; 29.8; 33.5; 334.1; 35.9; 38.0; 48.7; 59.6; 71.8; 110.7; 118.8; 119.6; 122.0; 125.2; 126.4; 127.1; 127.4; 129.0; 131.4; 138.7.
LC-MS (Methode 1): m/z: [M+H]⁺ = 505.4, Rₜ 4.2 min.

### Stufe 7:

### (R)-1-(3-(1H-Indol-3-yl)-2-methylpropyl)-4-(dimethylamino)-4-phenylcyclohexanol

Eine Lösung von (R)-4-Dimethytamino-1-[2-methyl-3-(2-(triethylsilyl)-1*H*-indol-3-yl)propyl]-4-phenylcyclohexanol (175 mg, 0.34 mmol) in wasserfreiem Tetrahydrofuran (30 mL) wurde mit Tetra-n-butylammoniumfluorid-Trihydrat (109 mg, 0.34 mmol) versetzt und 4 h unter Rückfluss gerührt. Anschließend wurde das Reaktionsgemisch i. Vak. eingeengt und-der Rückstand durch Flashchromatographie (10 g, 20 × 1.5 cm) mit Ethylacetat / Methanol (95:5) gereinigt.
Ausbeute: 115 mg (86 %), farbloser Schaum
Schmelzpunkt: 56-59 °C
¹H-NMR (CDCl₃): 1.06 (d, 3H, J = 6.6 Hz); 1.30-1.41 (m, 2H); 1.49 (dd, 2H, J = 14.5, 7.0 Hz); 1.72-1.98 (m, 4H); 2.02 (s, 6H); 2.04 (s, 1 H); 2.08-2.30 (m, 3H); 2.61 (dd, 1H, J = 14.3, 7.7 Hz); 2.84 (dd, 1H, J = 14.2, 6.4 Hz); 7.02 (d, 1H, J = 2.2 Hz); 7.08-7.40 (m, 8H); 7.65-7.67 (m, 1 H); 8.09 (s, 1 H).
¹³C-NMR (CDCl₃): 22.7; 27.2; 29.2; 29.3; 30.1; 33.6; 34.1; 34.7; 38.1; 48.1; 60.4; 71.8; 111.0; 115.6; 119.1; 119.2; 121.7; 122.3; 126.6; 127.2; 127.6; 127.9; 136.4; 137.9.
¹H-NMR (DMSO-d₆): 0.92 (d, 3H, J = 6.6 Hz); 1.23-1.40 (m, 4H); 1.45-1.86 (m, 6H); 1.84 (s, 6H); 1.91 (s, 2H); 2.00-2.31 (m, 3H); 2.43 (dd, 1H, J = 14.1, 8.6 Hz); 2.81 (dd, 1H, J = 14.0, 5.5 Hz); 6.92-7.10 (m, 2H); 7.17-7.27 (m, 1 H); 7.29-7.38 (m, 6H); 7.53 (d, 1H, J = 7.7 Hz); 10.73 (s, 1 H).
LC-MS (Methode 1): m/z: [M+H]⁺ = 391.4, Rₜ 3.3 min.

### Stufe 8:

### (S)-N,N,3-Trimethyl-4'-phenyl-2,3,4,9-tetrahydrospiro[carbazol-1,1'-cyclohexan]-4'-amin (Beispiel Nr. 15, ein Diastereomer)

Eine Lösung von (R)-1-(3-(1H-Indol-3-yl)-2-methylpropyl)-4-(dimethylamino)-4-phenylcyclohexanol (100 mg, 0.25 mmol) in wasserfreiem 1,2-Dichlorethan (10 mL) wurde mit Trifluormethansulfonsäuretrimethylsilylester (222 mg, 181 µL, 1 mmol) versetzt und 4 h bei 50 °C gerührt. Nach Zugabe von Dichlormethan (10 mL) wurde die Lösung mit 1 M Kaliumcarbonatlösung (2 x 10 mL), Wasser (10 mL) und gesättigter Natriumchloridlösung (10 mL) gewaschen, mit Natriumsulfat getrocknet und i. Vak. eingeengt. Der Rückstand (90 mg) wurde in Methanol (10 mL) aufgenommen und 16 h bei Raumtemperatur gerührt. Der ausgefallene Feststoff wurde durch Dekantieren von der Lösung getrennt, der zurückgebliebene Feststoff nochmals mit Methanol versetzt, gerührt und dekantiert, wodurch Beispiel Nr. 15 erhalten wurde.
Beispiel Nr. 15: Ausbeute: 38 mg (41 %), weißer Feststoff
Schmelzpunkt: 208-211 °C
¹H-NMR (DMSO-d₆): 1.07 (d, 3H, J = 6.5 Hz); 1.26 (br d, 2H, J = 12.0 Hz); 1.52-1.71 (m, 3H); 1.80-2.00 (m, 2H); 2.04 (s, 6H); 2.22 (d, 1H, J = 13.0 Hz); 2.53-2.70 (m, 3H); 2.76 (dd, 1H, J = 14.9, 4.5 Hz); 3.17 (d, 1H, J = 5.2 Hz); 6.85-7.01 (m, 2H); 7.22-7.45 (m, 7H); 10.56 (s, 1 H).
LC-MS: m/z: [M+H]⁺ = 373.3, 3.8 min.

### Untersuchungen zur Wirksamkeit der erfindungsgemäßen Verbindungen

### Messung der ORL1-Bindung

Die Verbindungen wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschließende Messung im Szintillationscounter Trilux (Wallac, Finnland), bestimmt. Die Affinität wird in Tabelle 1 als nanomolarer Kᵢ-Wert in oder% Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden Verbindung mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten. In einigen Fällen wurde auf die Bestimmung des Ki-Wertes verzichtet und nur die Hemmung bei einer Testkonzentration von 1 µM bestimmt.

### Messung der kappa-Bindung

Die Bestimmung erfolgte in einem homogenen Ansatz in Mikrotiterplatten. Hierzu wurden Verdünnungsreihen der jeweils zu prüfenden Substanzen mit einer Rezeptormembranpräparation (7 µg Protein pro 250 µl Inkubationsansatz) von CHO-K1-Zellen, welche den humanen κ-Opiatrezeptor exprimieren in Gegenwart von 1 nmol/l des radioaktiven Liganden [³H]-Cl-977 sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 100 µmol/l Naloxon zugegeben. Nach Beendigung der neunzigminütigen Inkubationszeit wurden die Mikrotiterplatten für 20 Minuten bei 500 Umdrehungen/Minute abzentrifugiert und die Radioaktivität in einem ß-Counter (Microbeta-Trilux 1450, Firma PerkinElmer Wallac, Freiburg, Deutschland) vermessen. Es wurde die prozentuale Verdrängung des radioaktiven Liganden aus seiner Bindung zum humanen κ-Opiatrezeptor bei einer Konzentration der Prüfsubstanzen von 1 µmol/l bestimmt und als prozentuale Hemmung (%Hemmung) der spezifischen Bindung angegeben. Ausgehend von der prozentualen Verdrängung durch unterschiedliche Konzentrationen der zu prüfenden Verbindungen können IC₅₀ Hemmkonzentrationen berechnet werden, die eine 50-prozentige Verdrängung des radioaktiven Liganden bewirken. Durch Umrechnung mittels der Cheng-Prusoff-Beziehung können daraus die Kᵢ-Werte für die Prüfsubstanzen berechnet werden.

### Analgesieprüfung im Tail-Flick-Test an der Ratte

Die analgetische Wirksamkeit der Testverbindungen wurde im Brennstrahl (Tail-flick)-Test an der Ratte nach der Methode von D'Amour and Smith (J. Pharm. Exp. Ther. 72, 74 79 (1941)) untersucht. Dazu wurden weibliche Sprague Dawley Ratten mit einem Gewicht zwischen 130 und 190 g verwendet. Die Tiere wurden einzeln in spezielle Testkäfige gesetzt und die Schwanzbasis einem focussierten Wärmestrahl einer Lampe (Tail-flick Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Tieren 2,5-5 Sekunden betrug. Vor Gabe einer Testverbindung wurden die Tiere innerhalb von 30 Minuten zweimal vorgetestet und der Mittelwert dieser Messungen als Vortestmittetwert berechnet. Die Schmerzmessung wurde 20, 40 und 60 min nach intravenöser Gabe durchgeführt. Die analgetische Wirkung wurde als Zunahme der Schmerzlatenz (% MPE) bestimmt nach folgender Formel: [(T₁ - T₀)/(T₂ - T₀)] x 100. Dabei ist die To die Latenzzeit vor und T₁ die Latenzzeit nach Substanzapplikation, T₂ ist die maximale Expositionszeit (12 sec).Zur Bestimmung der Dosisabhängigkeit wurde die jeweilige Testverbindung in 3 - 5 logarithmisch ansteigenden Dosen, die jeweils die Schwellen- und die maximale Wirkdosis einschlossen, appliziert und die ED₅₀-Werte mit Hilfe der Regressionsanalyse bestimmt. Die ED₅₀-Berechnung erfolgte im Wirkmaximum, 20 Minuten nach intravenöser Substanzgabe.

### Chung-Modell: Mononeuropathieschmerz nach spinaler Nervenligatur

Tiere: Männliche Sprague Dawley Ratten (140-160g), von einem kommerziellen Züchter (Janvier, Genest St. Isle, Frankreich), wurden unter einem 12:12h Licht-Dunkel Rhythmus gehalten. Die Tiere wurden mit Futter und Leitungswasser ad libitum gehalten. Zwischen der Lieferung der Tiere und der Operation wurde eine Pause von einer Woche eingehalten. Die Tiere wurden nach Operation über einen Zeitraum von 4-5 Wochen mehrfach getestet, wobei eine Auswaschzeit von mindestens einer Woche eingehalten wurde.

Modellbeschreibung: Unter Pentobarbital Narkose (Narcoren^{®}, 60 mg/kg i.p., Merial GmbH, Hallbergmoos, Deutschland), wurden die linken L5, L6 Spinalnerven exponiert, indem ein Stück des paravertebralen Muskels und ein Teil des linken spinalen Prozesses des L5 lumbalen Wirbelkörpers entfernt wurden. Die Spinalnerven L5 und L6 wurden vorsichtig isoliert und mit einer festen Ligatur abgebunden (NC-silk black,USP 5/0, metric 1, Braun Melsungen AG, Melsungen, Deutschland) (Kim and Chung 1992). Nach Ligatur wurden Muskel und benachbarte Gewebe zugenäht und die Wunde mittels Metallklammern geschlossen.

Nach einer Woche Erholungszeit wurden die Tiere zur Messung der mechanischen Allodynie in Käfige mit einem Drahtboden gesetzt. An ipsi- und/oder kontralateraler Hinterpfote wurde die Wegziehschwelle mittels eines elektronischen von Frey Filamentes (Somedic AB, Malmö, Schweden) bestimmt. Der Median von fünf Stimulierungen ergab einen Datenpunkt. Die Tiere wurden 30 min vor und zu verschiedenen Zeiten nach Applikation von Testsubstanz-oder Vehikellösung getestet. Die Daten wurden als % maximal mögliche Wirkung (%MPE) aus den Vortesten der Einzeltiere (=0%MPE) und den Testwerten einer unabhängigen Sham-Kontrollgruppe (=100%MPE) bestimmt. Alternativ wurden die Wegziehschwellen in Gramm dargestellt.

Statistische Auswertung: ED₅₀ Werte und 95% Vertrauensbereiche wurden über semilogarithmische Regressionsanalyse zum Zeitpunkt der maximalen Wirkung bestimmt. Die Daten wurden über eine Varianzanalyse mit wiederholten Messungen sowie einer post hoc Analyse nach Bonferroni analysiert. Die Gruppengröße betrug üblicherweise n=10.

Referenzen: Kim, S.H. and Chung, J.M., An experimental model for peripheral neuropathy produced by segmental spinal nerve ligation in the rat, Pain, 50 (1992) 355-363.

### hERK-K+-Kanal-Bindungsassay

Im hERK-Bindungsassay wird die Verdrängung von [³H]-Dofetilide durch Prüfsubstanzen an Zellmembranen (isoliert aus hERK-transfizierten humanen embryonalen Nierenzellen, HEK293) getestet. Unspezifische Bindungen werden in Gegenwart von Dofetilide bestimmt. Die Inkubationszeit beträgt 60 min. bei 37°C. Nach Ablauf der Inkubationszeit wird die Testplatte über einer Filterplatte abgesaugt. Im Filter verbliebene und von [³H]-Dofetilide gebundene Rezeptormoleküle können nun entsprechend über die Messung der Radioaktivität quantifiziert werden, woraus wiederum Aussagen über die Verdrängung von [³H]-Dofetilide durch Prüfsubstanzen resultieren. Weitere Details können im Methodenteil von Finlayson et al. (2001) nachgelesen werden.

Literatur: [3H]dofetilide binding in SHSY5Y and HEK293 cells expressing a HERG-like K+ channel? Finlayson K, Pennington AJ, Kelly JS. Eur J Pharmacol. 2001 Feb 2;412(3):203-12.

### Ergebnisse

| Nr. | % Hemmung (ORL1) [1 µM] | Ki (ORL1) Mittel [µM] | % Hemmung (µ) [1 µM] | Ki (µ) Mittel [µM] | Tail flick Ratte, i.v. | SNL Ratte, i.v. |
|---|---|---|---|---|---|---|
| Bsp.-1 | 33 | 0,240 | 62 | 0,460 | nd | nd |
| Bsp.-2 | 99 | 0,001 | 98 | 0,002 | 100% MPE bei 100 µg/kg | 19% MPE bei 5 µg/kg |
| Bsp.-3 | nd | 0,53 | nd | 0,27 | nd | nd |
| Bsp.-4 | 82 | 0,016 | 100 | 0,011 | nd | nd |
| Bsp.-5 | 40 | nd | 67 | nd | nd | nd |
| Bsp.-6 | 96 | 0,011 | 96 | 0,007 | nd | nd |
| Bsp.-7 | 18 | 0,63 | 57 | 0,28 | nd | nd |
| Bsp.-8 | 100 | 0,002 | 97 | 0,002 | nd | nd |
| Bsp.-11 | 74 | nd | 91 | nd | nd | nd |
| Bsp.-12 | 96 | nd | 98 | nd | nd | nd |
| Bsp.-15 | 96 | nd | 97 | nd | nd | nd |

| | | | | | | |
|---|---|---|---|---|---|---|
| nd=not determined | | | | | | |

In der nachfolgenden Tabelle sind die Eigenschaften der erfindungsgemäßen Verbindungen gemäß Beispiel 1 und 2 und die Eigenschaften der korrespondierenden Verbindungen, welche den gleichen Grundkörper aufwiesen und sich nur im Rest -CR₁₈R₁₉- (= X) unterscheiden, gegenübergestellt:

| Nr. | X | Diastereomer | Ki (kappa)/ Ki (ORL1) | Ki (ORL1) Mittel [µM] | Ki (kappa) Mittel [µM) | Ki (hERG) Mittel [µM] |
|---|---|---|---|---|---|---|
| Bsp.-1 | -CH₂- | polares | 4 | 0,240 | 1,02 | 13%(1 µM) |
| V-1 | -NH- | polares | 1 | 0,006 | 0,007 | 19%(1 µM) |
| Bsp.-2 | -CH₂- | unpolares | 11 | 0,001 | 0,011 | n.e. |
| V-2 | -NH- | unpolares | 2.5 | 0,0002 | 0,0005 | 0,49 |

Wie der vorstehende Vergleich belegt, weisen die erfindungsgemäßen Verbindungen im Vergleich zu den strukturell ähnlichen Spiroaminen (X= -NH-) eine höhere Selektivität gegenüber dem kappa-Opioid-Rezeptor (definiert als 1/[K_{i(ORL1)}/Kᵢ₍ₖₐₚₚₐ₎]) auf.

Es kann daher davon ausgegangen werden, dass bei Verabreichung der erfindungsgemäßen Verbindungen solche Nebenwirkungen, welcher üblicherweise mit einer Bindung an den kappa-Opioid-Rezeptor in Zusammenhang stehen (z.B. Dysphorie, Sedation und Diurese), wenn überhaupt in nur geringem Umfang auftreten.

Darüber hinaus belegt der vorstehende Vergleich, dass die erfindungsgemäßen Verbindungen, insbesondere das jeweils unpolarere Diastereomer, im Vergleich zu strukturell ähnlichen Spiroaminen (X = -NH-) eine geringere Affinität zum hERG-Ionenkanal aufweisen.

Es kann daher ferner davon ausgegangen werden, dass bei Verabreichung der erfindungsgemäßen Verbindungen solche Nebenwirkungen, welcher üblicherweise mit einer Bindung an den hERG-Ionenkanal in Zusammenhang stehen (z.B. kardiovaskuläre Nebenwirkungen), wenn überhaupt in nur geringem Umfang auftreten.

## Patentansprüche

1. Verbindung der allgemeinen Formel (1), 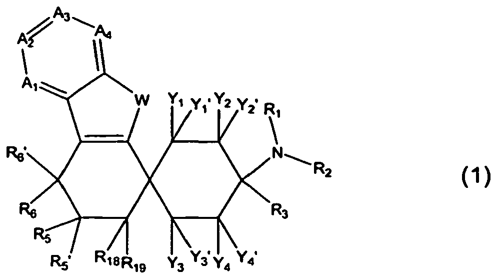 worin
A₁ für -N= oder -CR₇= steht,
A₂ für -N= oder -CR₈= steht,
A₃ für -N= oder -CR₉= steht,
A₄ für -N= oder -CR₁₀= steht;
mit der Maßgabe, dass höchstens zwei der Reste A₁, A₂, A₃ und A₄ für -N= stehen;
Y₁, Y₁', Y₂, Y₂', Y₃, Y₃', Y₄ und Y₄' jeweils unabhängig voneinander ausgewählt sind aus der Gruppe bestehend aus -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ und -NHC(=O)N(R₀)₂; oder Y₁ und Y₁', oder Y₂ und Y₂', oder Y₃ und Y₃', oder Y₄ und Y₄' gemeinsam für =O stehen;
W für -NR₄-, -O- oder -S- steht;
R₀ jeweils unabhängig für -C₁₋₈-Aliphat, -C₃₋₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ und R₂, unabhängig voneinander für -H oder -R₀ stehen; oder R₁ und R₂ zusammen für -CH₂CH₂OCH₂CH₂-, -CH₂CH₂NR₁₁CH₂CH₂- oder -(CH₂)₃₋₆- stehen;
R₃ für -R₀ steht;
R₄ für -H, -R₀, -COR₁₂ oder -S(=O)₂R₁₂ steht;
R₅, R₅', R₆, R₆', R₇, R₈, R₉, R₁₀, R₁₈ und R₁₉ jeweils unabhängig voneinander für -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, =O oder -R₀ stehen; oder R₅ und R₆ gemeinsam für -(CH₂)₂₋₆- stehen, wobei einzelne Wasserstoffatome auch durch -F, -Cl, -Br, -I, -NO₂, -CF₃, -OR₁₃, -CN oder -C₁₋₆-Aliphat ersetzt sein können;
R₁₁ jeweils unabhängig für -H, -R₀ oder -C(=O)R₀ steht;
R₁₂ jeweils unabhängig für -H, -R₀, -OR₁₃, oder -NR₁₄R₁₅ steht;
R₁₃ jeweils unabhängig für -H oder -R₀ steht;
R₁₄ und **R₁₅** unabhängig voneinander für -H oder -R₀ stehen; oder R₁₄ und R₁₅ zusammen für -CH₂CH₂OCH₂CH₂-, -CH₂CH₂NR₁₆CH₂CH₂- oder -(CH₂)₃₋₆- stehen;
R₁₆ für -H oder -C₁₋₆-Aliphat steht;
wobei
"Aliphat" jeweils ein verzweigter oder unverzweigter, gesättigter oder ein ein- oder mehrfach ungesättigter, unsubstituierter oder ein- oder mehrfach substituierter, aliphatischer Kohlenwasserstoffrest ist;
"Cycloaliphat" jeweils ein gesättigter oder ein ein- oder mehrfach ungesättigter, unsubstituierter oder ein- oder mehrfach substituierter, alicyclischer, mono- oder multicyclischer Kohlenwasserstoffrest ist;
wobei in Bezug auf "Aliphat" und "Cycloaliphat" unter "ein- oder mehrfach substituiert" die ein- oder mehrfache Substitution eines oder mehrerer Wasserstoffatome durch -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=OFN(R₀)₂, -Si(R₀)₃ oder-PO(OR₀)₂ verstanden wird;
"Aryl" jeweils unabhängig für ein carbocyclisches Ringsystem mit mindestens einem aromatischen Ring, aber ohne Heteroatome in diesem Ring steht, wobei die Aryl-Reste ggf. mit weiteren gesättigten, (partiell) ungesättigten oder aromatischen Ringsystemen kondensiert sein können und jeder Aryl-Rest unsubstituiert oder ein- oder mehrfach substituiert vorliegen kann, wobei die Aryl-Substituenten gleich oder verschieden und in jeder beliebigen und möglichen Position des Aryls sein können;
"Heteroaryl" für einen 5-, 6- oder 7-gliedrigen cyclischen aromatischen Rest steht, der 1, 2, 3, 4 oder 5 Heteroatome enthält, wobei die Heteroatome gleich oder verschieden Stickstoff, Sauerstoff oder Schwefel sind, und der Heterocyclus unsubstituiert oder ein-oder mehrfach substituiert sein kann; wobei im Falle der Substitution am Heterocyclus die Substituenten gleich oder verschieden und in jeder beliebigen und möglichen Position des Heteroaryls sein können; und wobei der Heterocyclus auch Teil eines bi- oder polycyclischen Systems sein kann;
wobei in Bezug auf "Aryl" und "Heteroaryl" unter "ein- oder mehrfach substituiert" die ein- oder mehrfache Substitution eines oder mehrerer Wasserstoffatome des Ringsystems durch Substituenten ausgewählt aus der Gruppe bestehend aus -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)O⁻, -NHC(=O)R₀, -NHC(=O)OR₀, -NH-C(=O)NH₂, -NHC(=O)NHR₀, -NHC(=O)-N(R₀)₂, -Si(R₀)₃ und -PO(OR₀)₂ verstanden wird; wobei ggf. vorhandene N-Ringatome jeweils oxidiert sein können;
in Form eines einzelnen Stereoisomers oder deren Mischung, der freien Verbindungen und/oder ihrer physiologisch verträglichen Salze.

2. Verbindung nach Anspruch 1, welche die allgemeine Formel (2), (3), (4) oder (5) 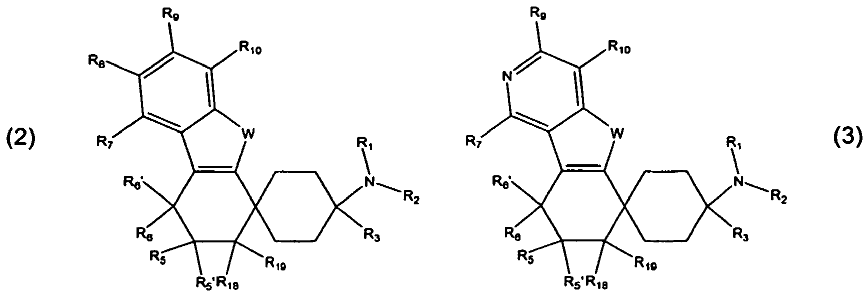 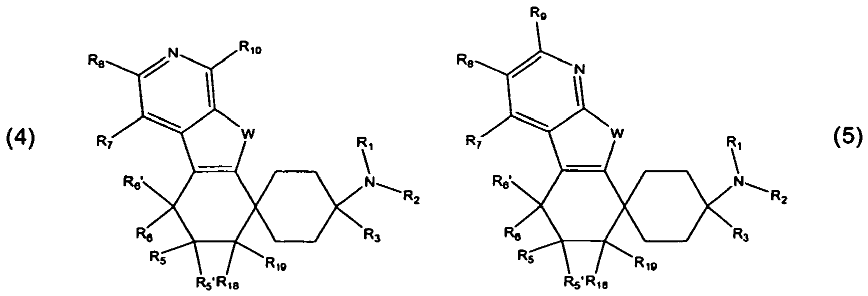 aufweist.

3. Verbindung nach Anspruch 2, welche die allgemeine Formel (2.1), (2.2), (2.3) oder (2.4) 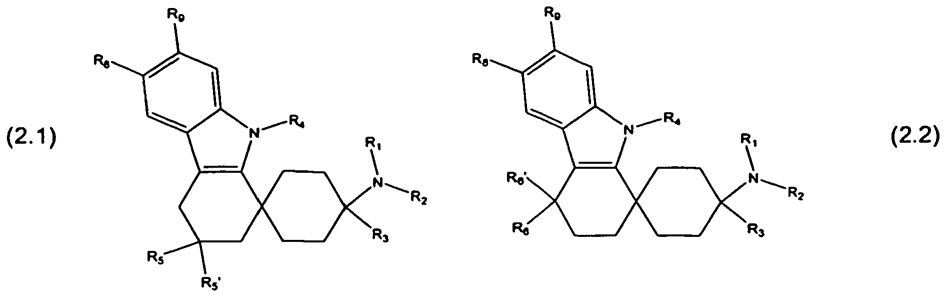 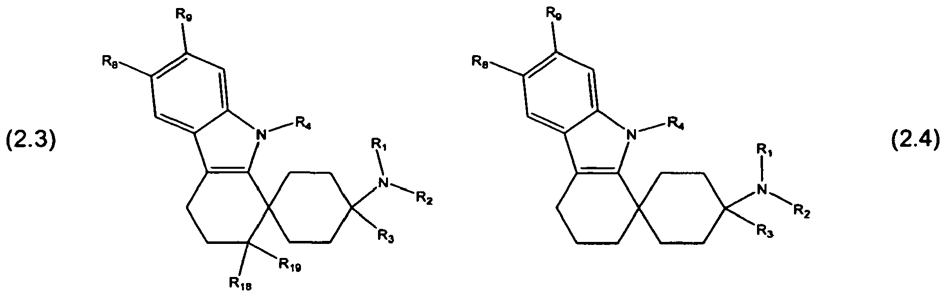 aufweist.

4. Verbindung nach Anspruch 2, welche die allgemeine Formel , (2.5), (2.6), (2.7) oder (2.8) 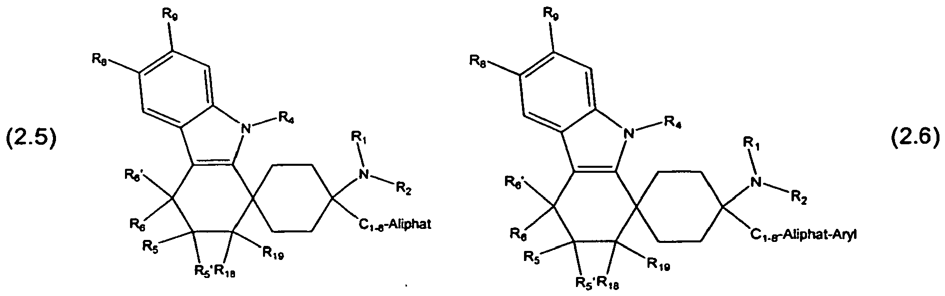 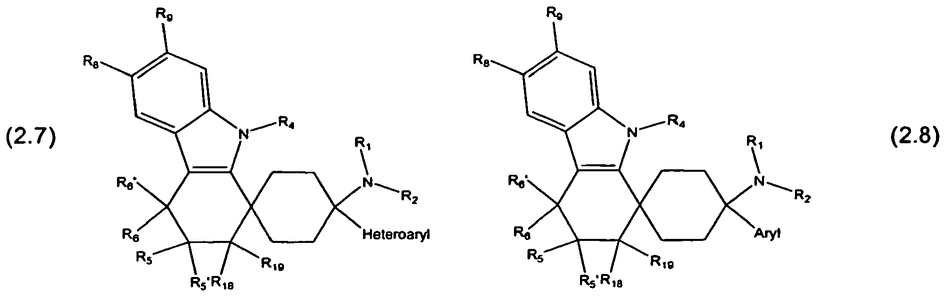 aufweist.

5. Verbindung nach Anspruch 1, welche die allgemeine Formel (6) 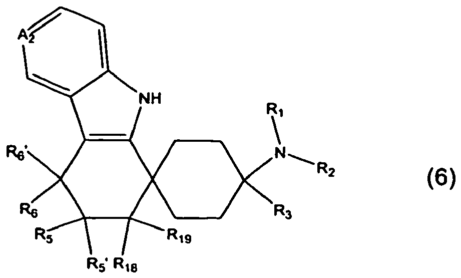 aufweist, worin
A₂ für -N= oder -CR₈= steht,
R₀ jeweils unabhängig für -C₁₋₈-Aliphat, -C₃₋₁₂-Cycloaliphat, -Aryl, -Heteroaryl, -C₁₋₈-Aliphat-C₃₋₁₂-Cycloaliphat, -C₁₋₈-Aliphat-Aryl, -C₁₋₈-Aliphat-Heteroaryl, -C₃₋₈-Cycloaliphat-C₁₋₈-Aliphat, -C₃₋₈-Cycloaliphat-Aryl oder -C₃₋₈-Cycloaliphat-Heteroaryl steht;
R₁ für -CH₃ steht;
R₂ für -H oder -CH₃ steht;
oder R₁ und R₂ zusammen für -(CH₂)₃₋₄- stehen;
R₃ für -C₁₋₆-Aliphat, -Aryl, -Heteroaryl, -C₁₋₆-Aliphat-Aryl oder -C₁₋₆-Aliphat-Heteroaryl;
R₅, R₅', R₆, R₆', R₈, R₁₈ und R₁₉ jeweils unabhängig voneinander für -H, -F, -Cl, -Br, -I, -NO₂, -CF₃, -OR₁₃, -SR₁₃, -S(=O)₂R₁₃, -S(=O)OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)-N(R₀)₂, -NHC(=O)OR₀, -NHS(=O)₁₋₂R₀, =O oder -R₀ stehen;
R₁₃ jeweils unabhängig für -H oder -R₀ steht; und
R₁₄ und R₁₅ unabhängig voneinander für -H oder -R₀ stehen.

6. Verbindung nach einem der vorstehenden Ansprüche ausgewählt aus der Gruppe bestehend aus N,N,3,3-Tetramethyl-4'-phenyl-2,3,4,9-tetrahydrospiro[carbazole-1,1'-cyclohexan]-4'-amin; N,N-Dimethyl-3,4'-diphenyl-2,3,4,9-tetrahydrospiro[carbazol-1,1'-cyclohexan]-4'-amin; 4'-(3-Fluorphenyl)-N,N-dimethyl-3-phenyl-2,3,4,9-tetrahydrospiro[carbazol-1,1'-cyclohexan]-4'-amin; N,N,4,4-Tetramethyl-4'-phenyl-2,3,4,9-tetrahydrospiro[carbazol-1,1'-cyclohexan]-4'-amin; N,N-Dimethyl-4'-phenyl-2-(phenylsulfonyl)-2,3,4,9-tetrahydrospiro[carbazol-1,1'-cyclohexan]-4'-amin; 4'-(Dimethylamino)-N-methoxy-N-methyl-4'-phenyl-2,3,4,9-tetrahydrospiro[carbazol-1, 1'-cyclohexan]-2-carboxamid; N,N-Dimethyl-4'-phenyl-2-(piperidin-1-ylsulfonyl)-2,3,4,9-tetrahydrospiro-[carbazol-1,1'-cyclohexan]-4'-amin; (4'-(Dimethylamino)-4'-phenyl-2,3,4,9-tetrahydrospiro[carbazole-1,1'-cyclohexane]-2-yl)methanol; N4',N4'-dimethyl-4'-(3-fluorphenyl)-2,3,4,9-tetrahydrospiro[carbazol-1,1'-cyclohexan]-3,4'-diamin; N-(4'-(Dimethylamino)-4'-(3-fluorophenyl)-2,3,4,9-tetrahydrospiro[carbazol-1,1'-cyclohexan]-3-yl)cinnamamid; N-(4'-(Dimethylamino)-4'-(3-fluorophenyl)-2,3,4,9-tetrahydrospiro[carbazole-1,1'-cyclohexan]-3-yl)-2-phenylacetamid; N-(4'-(Dimethylamino)-4'-(3-fluorophenyl)-2,3,4,9-tetrahydrospiro[carbazol-1,1'-cyclohexan]-3-yl)benzo[b]thiophen-2-carboxamid; N-(4'-(Dimethylamino)-4'-(3-fluorphenyl)-2,3,4,9-tetrahydrospiro[carbazole-1,1'-cyclohexan]-3-yl)-3-phenylpropanamid; N-(4'-(Dimethylamino)-4'-(3-fluorophenyl)-2,3,4,9-tetrahydrospiro[carbazol-1,1'-cyclohexan]-3-yl)-2-phenylcyclopropancarboxamid; N-(4'-(Dimethylamino)-4'-(3-fluorphenyl)-2,3,4,9-tetrahydrospiro[carbazol-1,1'-cyclohexan]-3-yl)acetamid; 4'-Butyl-N,N-dimethyl-2,3,4,9-tetrahydrospiro[carbazol-1,1'-cyclohexan]-4'-amin; 4'-Benzyl-N,N-dimethyl-2,3,4,9-tetrahydrospiro[carbazol-1,1'-cyclohexan]-4'-amin; N,N-Dimethyl-4'-(thiophen-2-yl)-2,3,4,9-tetrahydrospiro[carbazol-1,1'-cyclohexan]-4'-amin; 4'-(3-Fluorphenyl)-N,N-dimethyl-2,3,4,9-tetrahydrospiro[carbazol-1,1'-cyclohexan]-4'-amin; 4'-(Azetidin-1-yl)-4'-phenyl-2,3,4,9-tetrahydrospiro[carbazol-1,1'-cyclohexan]; 4'-(Azetidin-1-yl)-4'-(3-fluorphenyl)-2,3,4,9-tetrahydrospiro[carbazol-1,1'-cyclohexan], 4'-(Acetidin-1-yl)-4'-phenyl-2,3,4,9-tetrahydrospiro[carbazol-1,1'-cyclohexan] (GRT15126H), N,N-Dimethyl-N-(4-butyl-2',3',4',9'-tetrahydro-1H-spiro[cyclohexan-1,1'-carbazol]-4-yl)amin, 4'-Benzyl-N,N-dimethyl-2,3,4,9-tetrahydrospiro[carbazol-1,1'-cyclohexan]-4'-amin , 4'-(3-Fluorphenyl)-N,N-dimethyl-2,3,4,9-tetrahydrospiro-[carbazol-1,1'-cyclohexan]-4'-amin, N,N-Dimethyl-4'-(thiophen-2-yl)-2,3,4,9-tetrahydrospiro[carbazol-1,1'-cyclohexan]-4'-amin, (S)-N,N,3-Trimethyl-4'-phenyl-2,3,4,9-tetrahydrospiro[carbazol-1,1'-cyclohexan]-4'-amin in Form eines einzelnen Stereoisomers oder deren Mischung, der freien Verbindungen und/oder ihrer physiologisch verträglichen Salze.

7. Arzneimittel enthaltend wenigstens eine Verbindung nach einem der Ansprüche 1 bis 6 in Form eines einzelnen Stereoisomers oder deren Mischung, der freien Verbindungen und/oder ihrer physiologisch verträglichen Salze, sowie ggf. geeignete Zusatz- und/oder Hilfsstoffe und/oder gegebenenfalls weiterer Wirkstoffe.

8. Verwendung einer Verbindung nach einem der Ansprüche 1 bis 6 in Form eines einzelnen Stereoisomers oder deren Mischung, der freien Verbindungen und/oder ihrer physiologisch verträglichen Salze, zur Herstellung eines Arzneimittels zur Behandlung von Schmerz.

9. Verwendung einer Verbindung nach einem der Ansprüche 1 bis 6 in Form eines einzelnen Stereoisomers oder deren Mischung, der freien Verbindungen und/oder ihrer physiologisch verträglichen Salze zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmissbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Compound of the general formula (1), 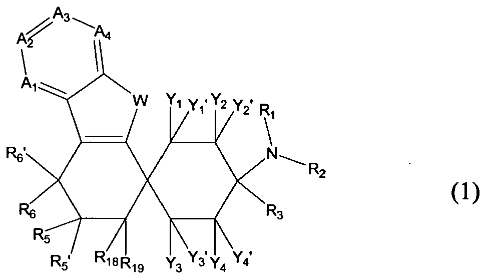 wherein
A₁ stands for -N= or -CR₇=,
A₂ stands for -N= or -CR₈=,
A₃ stands for -N= or -CR₉=,
A₄ stands for -N= or -CR₁₀=;
on condition that at most two of the residues A₁, A₂, A₃ and A₄ stand for -N=;
Y₁, Y₁', Y₂, Y₂', Y₃, Y₃', Y₄ and Y₄' are selected respectively independently of one another from the group comprising -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀ and -NHC(=O)N(R₀)₂; or Y₁ and Y₁', or Y₂ and Y₂', or Y₃ and Y₃', or Y₄ and Y₄' jointly stand for =O;
W stands for -NR₄-, -O- or -S-;
R₀ respectively independently stands for -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
R₁ und R₂, independently of one another, stand for -H or -R₀; or R₁ and R₂ together stand for -CH₂CH₂OCH₂CH₂-, -CH₂CH₂NR₁₁CH₂CH₂- or -(CH₂)₃₋₆-;
R₃ stands for -R₀;
R₄ stands for -H, -R₀, -COR₁₂ or -S(=O)₂R₁₂;
R₅, R₅', R₆, R₆', R₇, R₈, R₉, R₁₀, R₁₈ and R₁₉ respectively independently of one another stand for -H, -F, -Cl, -Br, -I, -NO₂ -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, =O or -R₀; or R₅ and R₆ jointly stand for -(CH₂)₂₋₆-, wherein individual hydrogen atoms can also be replaced by -F, -Cl, -Br, -I, -NO₂, -CF₃, -OR₁₃, -CN or -C₁₋₆-aliphatic;
R₁₁ respectively independently stands for -H, -R₀ or -C(=O)R₀;
R₁₂ respectively independently stands for -H, -R₀, -OR₁₃, or -NR₁₄R₁₅;
R₁₃ respectively independently stands for -H or -R₀;
R₁₄ and R₁₅ independently of one another stand for -H or -R₀; or R₁₄ and R₁₅ together stand for -CH₂CH₂OCH₂CH₂-, -CH₂CH₂NR₁₆CH₂CH₂- or -(CH₂)₃₋₆-;
R₁₆ stands for -H or -C₁₋₆-aliphatic;
wherein
"aliphatic" respectively is a branched or unbranched, saturated or a mono- or polyunsaturated, unsubstituted or mono- or polysubstituted, aliphatic hydrocarbon residue;
"cycloaliphatic" respectively is a saturated or a mono- or polyunsaturated, unsubstituted or mono- or polysubstituted, alicyclic, mono- or multicyclic hydrocarbon residue;
wherein with respect to "aliphatic" and "cycloaliphatic", "mono- or polysubstituted" is understood to mean the mono- or polysubstitution of one or more hydrogen atoms by -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)-OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ or -PO(OR₀)₂;
"aryl", respectively independently, stands for a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl;
"heteroaryl" stands for a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system;
wherein with respect to "aryl" and "heteroaryl", "mono- or polysubstituted" is understood to mean the mono- or polysubstitution of one or more hydrogen atoms of the ring system by substituents selected from the group comprising -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)-N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N+(R₀)₃, -N⁺(R₀)₂O⁻, -NHC(=O)R₀, -NHC(=O)OR₀, -NH-C(=O)NH₂, -NHC(=O)NHR₀, -NHC(=O)-N(R₀)₂, -Si(R₀)₃ and -PO(OR₀)₂; wherein any N-ring atoms present can be respectively oxidised;
in the form of a single stereoisomer or mixture thereof, the free compounds and/or their physiologically compatible salts.

2. Compound according to claim 1, which has the general formula (2), (3), (4) or (5)

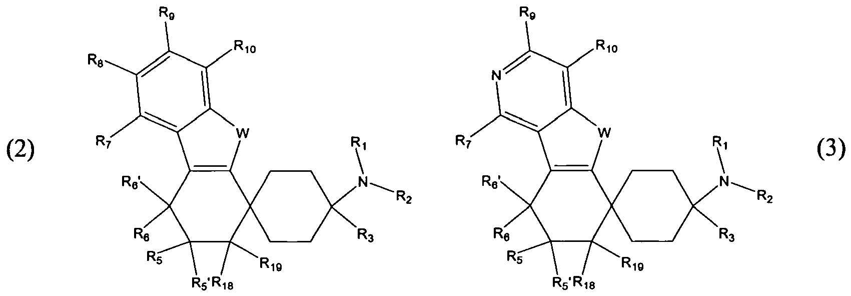

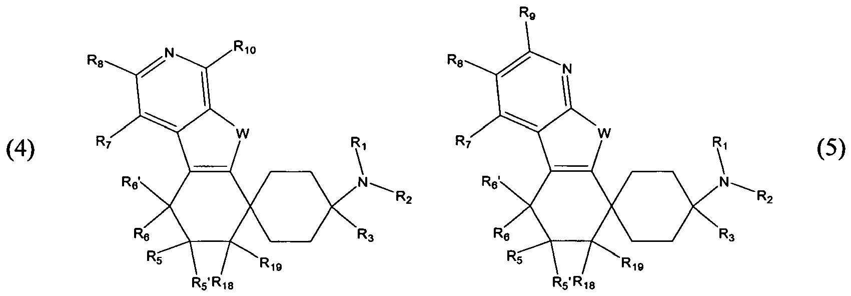

3. Compound according to claim 2, which has the general formula (2.1), (2.2), (2.3) or (2.4)

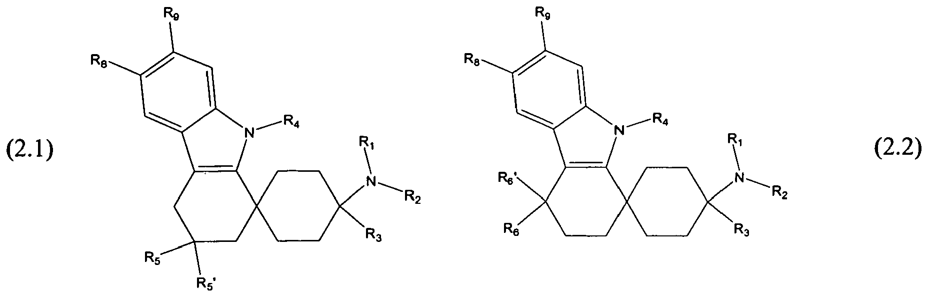

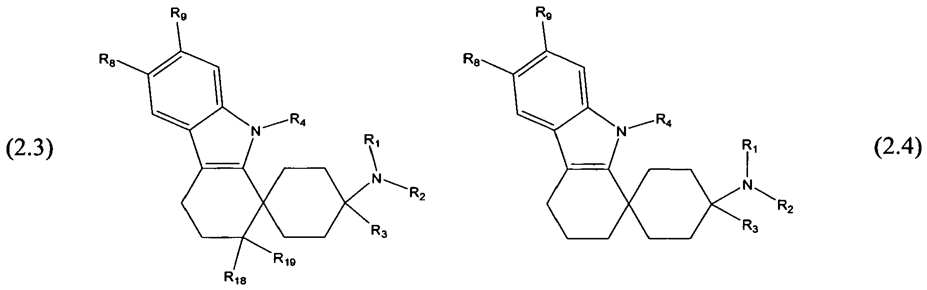

4. Compound according to claim 2, which has the general formula (2.5), (2.6), (2.7) or (2.8)

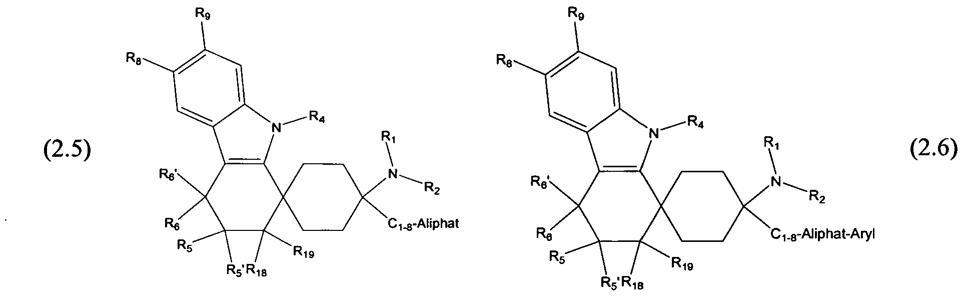

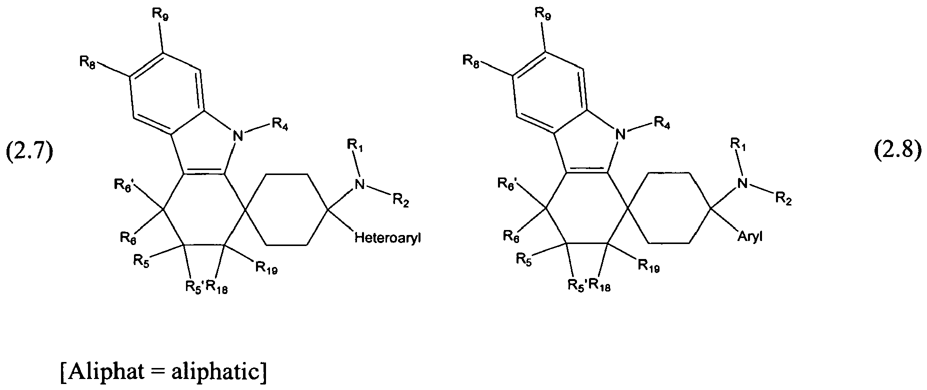

5. Compound according to claim 1, which has the general formula (6) 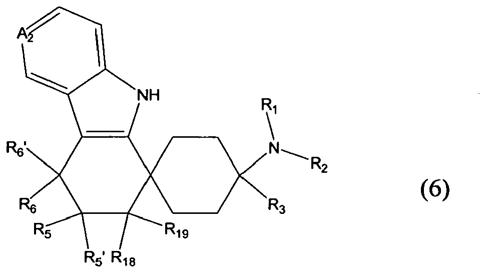 wherein
A₂ stands for -N= or -CR₈=,
R₀ respectively independently stands for -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
R₁ stands for -CH₃;
R₂ stands for -H or -CH₃;
or R₁ and R₂ together stand for -(CH₂)₃₋₄-;
R₃ stands for -C₁₋₆-aliphatic, -aryl, -heteroaryl, -C₁₋₆-aliphatic-aryl or -C₁₋₆-aliphatic-heteroaryl;
R₅, R₅', R₆, R₆', R₈, R₁₈ and R₁₉ respectively independently of one another stand for -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, =O or -R₀;
R₁₃ respectively independently stands for -H or -R₀; and
R₁₄ and R₁₅ independently of one another stand for -H or -R₀.

6. Compound according to one of the preceding claims selected from the group comprising N,N,3,3-tetramethyl-4'-phenyl-2,3,4,9-tetrahydrospiro[carbazole-1,1'-cyclohexane]-4'-amine; N,N-dimethyl-3,4'-diphenyl-2,3,4,9-tetrahydrospiro[carbazole-1,1'-cyclohexane]-4'-amine; 4'-(3-fluorophenyl)-N,N-dimethyl-3-phenyl-2,3,4,9-tetrahydrospiro[carbazole-1,1'-cyclohexane]-4'-amine; N,N,4,4-tetramethyl-4'-phenyl-2,3,4,9-tetrahydrospiro[carbazole-1,1'-cyclohexane]-4'-amine; N,N-dimethyl-4'-phenyl-2-(phenylsulphonyl)-2,3,4,9-tetrahydrospiro[carbazole-1,1'-cyclohexane]-4'-amine; 4'-(dimethylamino)-N-methoxy-N-methyl-4'-phenyl-2,3,4,9-tetrahydrospiro[carbazole-1,1'-cyclohexane]-2-carboxamide; N,N-dimethyl-4'-phenyl-2-(piperidin-1-ylsulphonyl)-2,3,4,9-tetrahydrospiro[carbazole-1,1'-cyclohexane]-4'-amine; (4'-(dimethylamino)-4'-phenyl-2,3,4,9-tetrahydrospiro[carbazole-1,1'-cyclohexane]-2-yl)methanol; N4',N4'-dimethyl-4'-(3-fluorophenyl)-2,3,4,9-tetrahydrospiro[carbazole-1,1'-cyclohexane]-3,4'-diamine; N-(4'-(dimethylamino)-4'-(3-fluorophenyl)-2,3,4,9-tetrahydrospiro[carbazole-1,1'-cyclohexane]-3-yl)cinnamamide; N-(4'-(dimethylamino)-4'-(3-fluorophenyl)-2,3,4,9-tetrahydrospiro[carbazole-1,1'-cyclohexane]-3-yl)-2-phenylacetamide; N-(4'-(dimethylamino)-4'-(3-fluorophenyl)-2,3,4,9-tetrahydrospiro[carbazole-1,1'-cyclohexane]-3-yl)benzo[b]thiophen-2-carboxamide; N-(4'-(dimethylamino)-4'-(3-fluorophenyl)-2,3,4,9-tetrahydrospiro[carbazole-1,1'-cyclohexane]-3-yl)-3-phenylpropanamide; N-(4'-(dimethylamino)-4'-(3-fluorophenyl)-2,3,4,9-tetrahydrospiro[carbazole-1,1'-cyclohexane]-3-yl)-2-phenylcyclopropane carboxamide; N-(4'-(dimethylamino)-4'-(3-fluorophenyl)-2,3,4,9-tetrahydrospiro[carbazole-1,1'-cyclohexane]-3-yl)acetamide; 4'-butyl-N,N-dimethyl-2,3,4,9-tetrahydrospiro[carbazole-1,1'-cyclohexane]-4'-amine; 4'-benzyl-N,N-dimethyl-2,3,4,9-tetrahydrospiro[carbazole-1,1'-cyclohexane]-4'-amine; N,N-dimethyl-4'-(thiophen-2-yl)-2,3,4,9-tetrahydrospiro[carbazole-1,1'-cyclohexane]-4'-amine; 4'-(3-fluorophenyl)-N,N-dimethyl-2,3,4,9-tetrahydrospiro[carbazole-1,1'-cyclohexane]-4'-amine; 4'-(acetidin-1-yl)-4'-phenyl-2,3,4,9-tetrahydrospiro[carbazole-1,1'-cyclohexane]; 4'-(acetidin-1-yl)-4'-(3-fluorophenyl)-2,3,4,9-tetrahydrospiro[carbazole-1,1'-cyclohexane], 4'-(acetidin-1-yl)-4'-phenyl-2,3,4,9-tetrahydrospiro[carbazole-1,1'-cyclohexane] (GRT15126H), N,N-dimethyl-N-(4-butyl-2',3',4',9'-tetrahydro-1H-spiro[cyclohexane-1,1'-carbazole]-4-yl)amine, 4'-benzyl-N,N-dimethyl-2,3,4,9-tetrahydrospiro[carbazole-1,1'-cyclohexane]-4'-amine , 4'-(3-fluorophenyl)-N,N-dimethyl-2,3,4,9-tetrahydrospiro[carbazole-1,1'-cyclohexane]-4'-amine, N,N-dimethyl-4'-(thiophen-2-yl)-2,3,4,9-tetrahydrospiro[carbazole-1,1'-cyclohexane]-4'-amine, (S)-N,N,3-trimethyl-4'-phenyl-2,3,4,9-tetrahydrospiro[carbazole-1,1'-cyclohexane]-4'-amine in the form of a single stereoisomer or mixture thereof, the free compounds and/or their physiologically compatible salts.

7. Medication containing at least one compound according to one of claims 1 to 6 in the form of a single stereoisomer or mixture thereof, the free compounds and/or their physiologically compatible salts, and also possibly suitable additives and/or adjuvants and/or possibly further active substances.

8. Use of a compound according to one of claims 1 to 6 in the form of a single stereoisomer or mixture thereof, the free compounds and/or their physiologically compatible salts for the production of a medication for the treatment of pain.

9. Use of a compound according to one of claims 1 to 6 in the form of a single stereoisomer or mixture thereof, the free compounds and/or their physiologically compatible salts for the production of a medication for the treatment of anxiety conditions, stress and stress-related syndromes, depressive illnesses, epilepsy, Alzheimer's disease, senile dementia, general cognitive dysfunctions, learning and memory disabilities (as nootropic), withdrawal symptoms, alcohol and/or drug and/or medication misuse and/or dependence, sexual dysfunctions, cardiovascular diseases, hypotension, hypertension, tinitus, pruritus, migraine, hearing impairment, deficient intestinal motility, eating disorders, anorexia, bulimia, mobility disorders, diarrhoea, cachexia, urinary incontinence, or as muscle relaxant, anticonvulsive or anaesthetic, or for coadministration in the treatment with an opioid analgesic or with an anaesthetic, for diuresis or anti-natriuresis, anxiolysis, for modulating movement activity, for modulating neurotransmitter release and for treating neuro-degenerative diseases associated therewith, for treating withdrawal symptoms and/or for reducing the addiction potential of opioids.

## Revendications

1. Composé de formule générale (1), 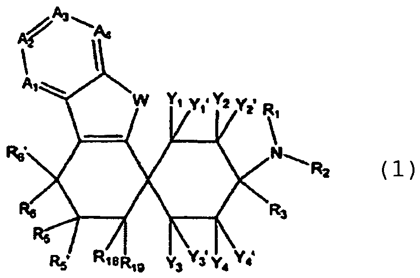 dans laquelle
A₁ représente -N= ou -CR₇=,
A₂ représente -N= ou -CR₈=,
A₃ représente -N= ou -CR₉=,
A₄ représente -N= ou -CR₁₀= ;
étant entendu qu'au maximum deux des radicaux A₁, A₂, A₃ et A₄ représentent -N= ;
Y₁, Y₁', Y₂, Y₂', Y₃, Y₃', Y₄ et Y₄' sont choisis chacun, indépendamment les uns des autres, dans l'ensemble constitué par -H, -F, -Cl, -Br, -I, -CN, -NO₂, -CHO, -R₀, -C(=O)R₀, -C(=O)H, -C(=O)-OH, -C(=O)OR₀, -C(=O)NH₂, -C(=O)NHR₀, -C(=O)N(R₀)₂, -OH, -OR₀, -OC(=O)H, -OC(=O)-R₀, -OC (=O) OR₀, -OC(=O)NHR₀, -OC(=O)N(R₀)₂, -SH, -SR₀, -SO₃H, -S(=O)₁₋₂-R₀, -S (=O)₁₋₂NH₂, -NH₂, -NHR₀, -N(R₀)₂, -N⁺(R₀)₃, -N⁺(R₀)₂O⁻ , -NHC(=O)R₀, -NHC(=O)OR₀, -NHC(=O)NH₂, -NHC(=O)NHR₀ et -NHC(=O)N(R₀)₂ ; ou Y₁ et Y₁' , ou Y₂ et Y₂' , ou Y₃ et Y₃' , ou Y₄ et Y₄' représentent ensemble =O ;
W représente -NR₄-, -O- ou -S- ;
R₀ représente chaque fois indépendamment un groupe -aliphatique en C₁-C₈, -cycloaliphatique en C₃-C₁₂, -aryle, -hétéroaryle, -aliphatique(C₁-C₈)-cycloaliphatique(C₃-C₁₂), -aliphatique(C₁-C₈)-aryle, -aliphatique(C₁-C₈)-hétéroaryle, -cycloaliphatique(C₃-C₈)-aliphatique(C₁-C₈), -cycloaliphatique (C₃-C₈)-aryle ou cycloaliphatique(C₃-C₈)-hétéroaryle ;
R₁ et R₂, indépendamment l'un de l'autre, représentent -H ou -R₀ ; ou R₁ et R₂ représentent ensemble -CH₂CH₂OCH₂CH₂-, -CH₂CH₂NR₁₁CH₂CH₂- ou -(CH₂)₃₋₆- ;
R₃ représente -R₀ ;
R₄ représente -H, -R₀, -COR₁₂ ou -S(=O)₂R₁₂ ;
R₅, R₅' , R₆, R₆' , R₇, R₈, R₉, R₁₀, R₁₈ et R₁₉ représentent chacun, indépendamment les uns des autres -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, -NHC(=O)R₀, -NHC(=O)NHR₀, -NHC(=O)N(R₀)₂, -NHC(=O)OR₀, -NHS(=O)₁₋₂R₀, =O ou -R₀; ou R₅ et R₆ ensemble représentent -(CH₂)₂₋₆-, des atomes d'hydrogène individuels pouvant également être remplacés par -F, -Cl, -Br, -I, -NO₂, -CF₃, -OR₁₃, -CN ou par un groupe aliphatique en C₁-C₆ ;
R₁₁ représente chaque fois indépendamment -H, -R₀ ou -C(=O)R₀ ;
R₁₂ représente chaque fois indépendamment -H, -R₀, -OR₁₃, ou -NR₁₄R₁₅ ;
R₁₃ représente chaque fois indépendamment -H ou -Ro ;
R₁₄ et R₁₅ représentent, indépendamment l'un de l'autre, -H ou R₀ ; ou R₁₄ et R₁₅ représentent ensemble -CH₂CH₂OCH₂CH₂-, -CH₂CH₂NR₁₆CH₂CH₂- ou -(CH₂)₃₋₆- ;
R₁₆ représente -H ou un groupe -aliphatique en C₁-C₆ ;
un groupe « aliphatique » étant chaque fois un radical hydrocarboné aliphatique ramifié ou non ramifié, saturé ou une ou plusieurs fois insaturé, non substitué ou une ou plusieurs fois substitué ;
un groupe « cycloaliphatique » étant chaque fois un radical hydrocarboné alicyclique, mono- ou polycyclique, saturé ou une plusieurs fois insaturé, non substitué ou une ou plusieurs fois substitué ;
en entendant, eu égard à « aliphatique » et « cycloaliphatique », par « une ou plusieurs fois substitué » le remplacement simple ou multiple d'un ou plusieurs atomes d'hydrogène par -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NHC(=O)NH₂, -NHC(=O)NHR₀, -NHC(=O)-N(R₀)₂, -Si(R₀)₃ ou -PO(OR₀)₂;
« aryle » représentant chaque fois indépendamment un système cyclique carbocyclique comportant au moins un cycle aromatique, mais sans hétéroatomes dans ce cycle, les radicaux aryle pouvant éventuellement être condensés avec d'autres systèmes cycliques saturés, (partiellement) insaturés ou aromatiques et chaque radical aryle pouvant être non substitué ou une ou plusieurs fois substitué, les substituants sur le groupe aryle pouvant être identiques ou différents et en toute position quelconque et possible du radical aryle ;
« hétéroaryle" représentant un radical cyclique aromatique à 5, 6 ou 7 chaînons, qui contient 1, 2, 3, 4 ou 5 hétéroatomes, les hétéroatomes étant des atomes d'azote, d'oxygène ou de soufre identiques ou différents, et l'hétérocycle pouvant être non substitué ou une ou plusieurs fois substitué ; dans le cas de la substitution sur l'hétérocycle les substituants pouvant être identiques ou différents, et en position quelconque et possible du radical hétéroaryle ; et l'hétérocycle pouvant également faire partie d'un système bi- ou polycyclique ;
en entendant, eu égard à « aryle » et « hétéroaryle », par « une ou plusieurs fois substitué » le remplacement simple ou multiple d'un ou plusieurs atomes d'hydrogène du système cyclique par des substituants choisis dans l'ensemble constitué par -F, -Cl, -Br, -I, -CN, -NO₂, -CHO, =O, -R₀, -C(=O)R₀, -C(=O)OH, -C(=O)OR₀, -C(=O)NH₂, -C(=O)NHR₀, -C(=O)N(R₀)₂, -OH, -O(CH₂)₁₋₂O-, -OR₀, -OC(=O)H, -OC(=O)R₀, -OC(=O)OR₀, -OC(=O)NHR₀, -OC(=O)N(R₀)₂, -SH, -SR₀, -SO₃H, -S(=O)₁₋₂-R₀, -S(=O)₁₋₂NH₂, -NH₂, -NHR₀, -N(R₀)₂, -N⁺(R₀)₃, -N⁺(R₀)₂O⁻, -NHC(=O)R₀, -NHC(=O)OR₀, -NH-C(=O)NH₂, -NHC(=O)NHR₀, -NHC (=O) -N (R₀)₂, -Si (R₀)₃ ou -PO (OR₀)₂ ; des atomes d'azote éventuellement présents dans le cycle pouvant chacun être oxydés ;
sous forme d'un stéréoisomère individuel ou d'un mélange de tels stéréoisomères, des composés libres et/ou de leurs sels physiologiquement acceptables.

2. Composé selon la revendication 1, qui présente la formule générale (2), (3), (4) ou (5)

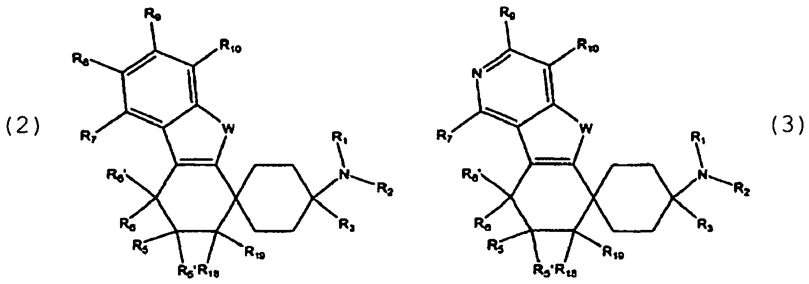

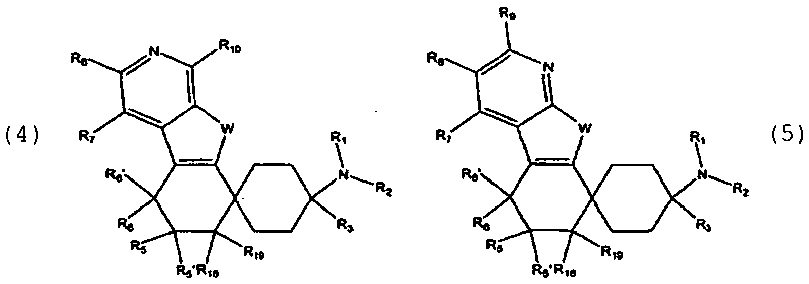

3. Composé selon la revendication 2, qui présente la formule générale (2.1), (2.2), (2.3) ou (2.4)

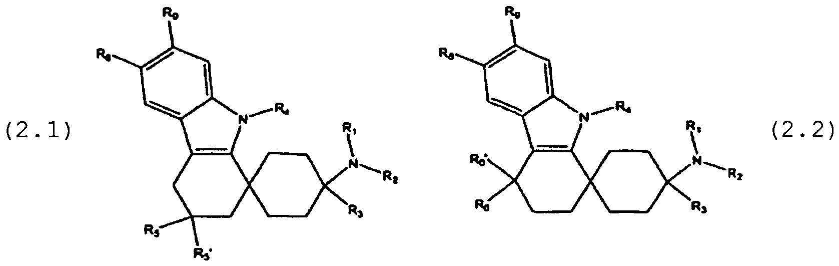

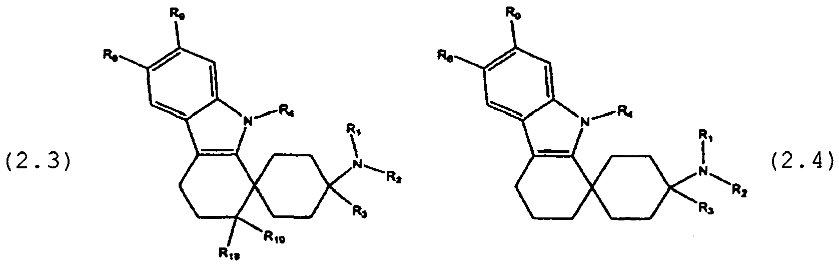

4. Composé selon la revendication 2, qui présente la formule générale (2.5), (2.6), (2.7) ou (2.8)

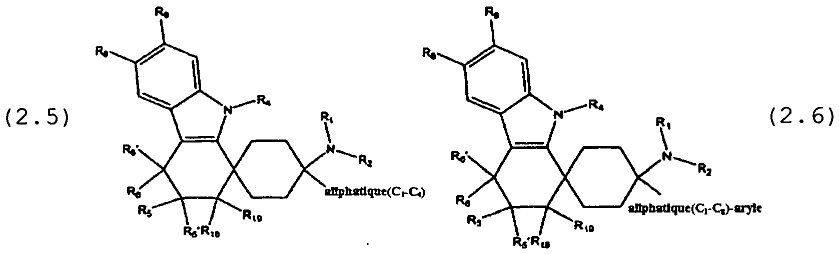

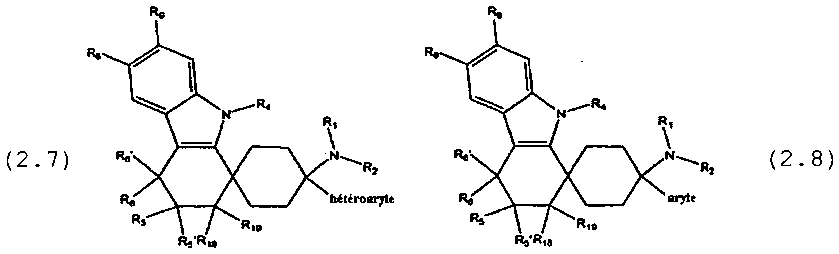

5. Composé selon la revendication 1, qui présente la formule générale (6) 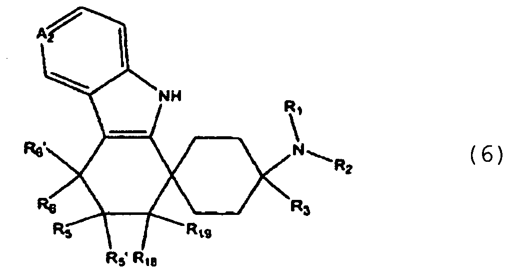 dans laquelle
A₂ représente -N- ou -CR₈= ;
R₀ représente chaque fois indépendamment un groupe -aliphatique en C₁-C₈, -cycloaliphatique en C₃-C₁₂, -aryle, -hétéroaryle, -aliphatique(C₁-C₈)-cycloaliphatique (C₃-C₁₂), -aliphatique (C₁-C₈) -aryle, -aliphatique(C₁-C₈)-hétéroaryle, -cycloaliphatique(C₃-C₈)-aliphatique(C₁-C₈), -cycloaliphatique(C₃-C₈)-aryle ou cycloaliphatique(C₃-C₈)-hétéroaryle ;
R₁ représente -CH₃ ;
R₂ représente -H ou -CH₃ ;
ou R₁ et R₂ représentent ensemble -(CH₂)₃₋₄-;
R₃ représente un groupe -aliphatique en C₁-C₆, -aryle, -hétéroaryle, -aliphatique(C₁-C₆)-aryle ou aliphatique(C₁-C₆)-hétéroaryle ;
R₅, R₅' , R₆, R₆' , R₈, R₁₈ et R₁₉ représentent chacun, indépendamment les uns des autres -H, -F, -Cl, -Br, -I, -NO₂, -CF₃, -OR₁₃, -SR₁₃, -S(=O)₂R₁₃, -S(=O)₂OR₁₃, -S(=O)₂NR₁₄R₁₅, -CN, -C(=O)OR₁₃, -C(=O)NR₁₃, -C(=O)NR₀OR₀, -NR₁₄R₁₅, ₋NHC(=O)R₀, -NHC(=O)NHR₀, -NHC(=O)N(R₀)₂, -NHC(=O)OR₀, -NHS(=O)₁₋₂R₀, =0 ou -R₀;
R₁₃ représente chaque fois indépendamment -H ou -R₀ ; et
R₁₄ et R₁₅ représentent, indépendamment l'un de l'autre, -H ou -R₀.

6. Composé selon l'une quelconque des revendications précédentes, choisi dans le groupe constitué par la N,N,3,3-tétraméthyl-4'-phényl-2,3,4,9-tétrahydrospiro[carbazole-1,1'-cyclohexan]-4'-amine ; la N,N-diméthyl-3,4'-diphényl-2,3,4,9-tétrahydrospiro[carbazole-1,1'-cyclohexan]-4'-amine ; la 4'-(3-fluorophényl)-N,N-diméthyl-3-phényl-2,3,4,9-tétrahydrospiro[carbazole-1,1'-cyclohexan]-4'-amine ; la N,N,4,4-tétraméthyl-4'-phényl-2,3,4,9-tétrahydrospiro[carbazole-1,1'-cyclohexan]-4'-amine ; la N,N-diméthyl-4'-phényl-2-(phénylsulfonyl)-2,3,4,9-tétrahydrospiro[carbazole-1,1'-cyclohexan]-4'-amine ; le 4'-(diméthylamino)-N-méthoxy-N-méthyl-4'-phényl-2,3,4,9-tétrahydrospiro[carbazole-1,1'-cyclohexan]-2-carboxamide ; la N,N-diméthyl-4'-phényl-2-(pipéridin-1-ylsulfonyl)-2,3,4,9-tétrahydrospiro[carbazole-1,1'-cyclohexan]-4'-amine ; le (4'-(diméthylamino)-4'-phényl-2,3,4,9-tétrahydrospiro[carbazole-1,1'-cyclohexane]-2-yl)méthanol ; la N4',N4'-diméthyl-4'-(3-fluorophényl)-2,3,4,9-tétrahydrospiro[carbazole-1,1'-cyclohexane]-3,4'-diamine ; le N-(4'-(diméthylamino)-4'-(3-fluorophényl)-2,3,4,9-tétrahydrospiro[carbazole-1,1'-cyclohexan]-3-yl)cinnamamide ; le N-(4'-(diméthylamino)-4'-(3-fluorophényl)-2,3,4,9-tétrahydrospiro[carbazole-1,1'-cyclohexan]-3-yl)-2-phénylacétamide ; le N-(4'-(diméthylamino)-4'-(3-fluorophényl)-2,3,4,9-tétrahydrospiro[carbazole-1,1'-cyclohexan]-3-yl)benzo[b]thiophène-2-carboxamide ; le N-(4'-(diméthylamino)-4'-(3-fluorophényl)-2,3,4,9-tétrahydrospiro[carbazole-1,1'-cyclohexan]-3-yl)-3-phénylpropionamide ; le N-(4'-(diméthylamino)-4'-(3-fluorophényl)-2,3,4,9-tétrahydrospiro[carbazole-1,1'-cyclohexan]-3-yl)-2-phénylcyclopropanecarboxamide ; le N-(4'-(diméthylamino)-4'-(3-fluorophényl)-2,3,4,9-tétrahydrospiro[carbazole-1,1'-cyclohexan]-3-yl)-acétamide ; la 4'-butyl-N,N-diméthyl-2,3,4,9-tétrahydrospiro[carbazole-1,1'-cyclohexan]-4'-amine ; la 4'-benzyl-N,N-diméthyl-2,3,4,9-tétrahydrospiro-[carbazole-1,1'-cyclohexan]-4'-amine ; la N,N-diméthyl-4'-(thiophén-2-yl)-2,3,4,9-tétrahydrospiro[carbazole-1,1'-cyclohexan]-4'-amine ; la 4'-(3-fluorophényl)-N,N-diméthyl-2,3,4,9-tétrahydrospiro[carbazole-1,1'-cyclohexan]-4'-amine ; le 4'-(azétidin-1-yl)-4'-phényl-2,3,4,9-tétrahydrospiro[carbazole-1,1'-cyclohexane] ; le 4'-(azétidin-1-yl)-4'-(3-fluorophényl)-2,3,4,9-tétrahydrospiro[carbazol-1,1'-cyclohexane, le 4'-(acétidin-1-yl)-4'-phényl-2,3,4,9-tétrahydrospiro-[carbazole-1,1'-cyclohexane] (GRT15126H), la N,N-diméthyl-N-(4-butyl-2',3',4',9'-tétrahydro-1H-spiro-[cyclohexane-1,1'-carbazol]-4-yl)amine, la 4'-benzyl-N,N-diméthyl-2,3,4,9-tétrahydrospiro[carbazole-1,1'-cyclohexan]-4'-amine, la 4'-(3-fluorophényl)-N,N-diméthyl-2,3,4,9-tétrahydrospiro[carbazole-1,1'-cyclohexan]-4'-amine, la N,N-diméthyl-4'-(thiophén-2-yl)-2,3,4,9-tétrahydrospiro[carbazole-1,1'-cyclohexan]-4'-amine, la (S)-N,N,3-triméthyl-4'-phényl-2,3,4,9-tétrahydrospiro[carbazole-1,1'-cyclohexan]-4'-amine sous forme d'un stéréoisomère individuel ou d'un mélange de tels stéréoisomères, des composés libres et/ou de leurs sels physiologiquement acceptables

7. Médicament contenant au moins un composé selon l'une quelconque des revendications 1 à 6, sous forme d'un stéréoisomère individuel ou d'un mélange de tels stéréoisomères, des composés libres et/ou de leurs sels physiologiquement acceptables, ainsi qu'éventuellement des additifs et/ou adjuvants appropriés et/ou éventuellement d'autres substances actives.

8. Utilisation d'un composé selon l'une quelconque des revendications 1 à 6, sous forme d'un stéréoisomère individuel ou d'un mélange de tels stéréoisomères, des composés libres et/ou de leurs sels physiologiquement acceptables, pour la fabrication d'un médicament destiné au traitement de la douleur.

9. Utilisation d'un composé selon l'une quelconque des revendications 1 à 6, sous forme d'un stéréoisomère individuel ou d'un mélange de tels stéréoisomères, des composés libres et/ou de leurs sels physiologiquement acceptables, pour la fabrication d'un médicament destiné au traitement d'états anxieux, du stress et de syndromes liés au stress, de dépressions, de l'épilepsie, de la maladie d'Alzheimer, de la démence sénile, de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de la dépendance à ceux-ci, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration simultanée dans le traitement par un analgésique opiacé ou par un anesthésique, à la diurèse ou à l'antinatriurèse, à l'anxiolyse, à la modulation de l'activité motrice, à la modulation de l'excrétion de neurotransmetteurs et au traitement de maladies neurodégénératives qui y sont liées, au traitement de manifestations inflammatoires et/ou à la réduction du potentiel de dépendance d'opiacés.
